(19) Europäisches Patentamt
European Patent Office
Office européen des brevets

(11) EP 3 458 607 B1

(12) EUROPEAN PATENT SPECIFICATION

(45) Date of publication and mention of the grant of the patent:
**06.10.2021 Bulletin 2021/40**

(21) Application number: **17727137.6**

(22) Date of filing: **22.05.2017**

(51) Int Cl.:
***C12Q 1/6886*** *(2018.01)*

(86) International application number:
**PCT/EP2017/062229**

(87) International publication number:
**WO 2017/198870 (23.11.2017 Gazette 2017/47)**

(54) **METHOD FOR THE PROGNOSIS OF ACUTE PROMYELOCYTIC LEUKEMIA**

VERFAHREN ZUR PROGNOSE VON AKUTER PROMYELOZYTISCHER LEUKÄMIE

MÉTHODE DE PRONOSTIC DE LA LEUCÉMIE PROMYÉLOCYTAIRE AIGUË

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2016 IT UA20163623**

(43) Date of publication of application:
**27.03.2019 Bulletin 2019/13**

(73) Proprietor: **Epi-C S.r.L.**
**80138 Napoli (NA) (IT)**

(72) Inventors:
- **ALTUCCI, Lucia**
  **80138 Napoli (NA) (IT)**
- **MARTENS, Joost, Hendrik, Adriaan**
  **80138 Napoli (NA) (IT)**
- **MAI, Antonello**
  **80138 Napoli (NA) (IT)**

(74) Representative: **Capasso, Olga**
**De Simone & Partners S.r.l.**
**Via Vincenzo Bellini, 20**
**00198 Roma (IT)**

(56) References cited:
**WO-A1-2015/116339**

- **MARIA E. FIGUEROA ET AL: "DNA Methylation Signatures Identify Biologically Distinct Subtypes in Acute Myeloid Leukemia", CANCER CELL, vol. 17, no. 1, 1 January 2010 (2010-01-01), pages 13-27, XP055333895, US ISSN: 1535-6108, DOI: 10.1016/j.ccr.2009.11.020**
- **CHOR S. CHIM ET AL: "Aberrant gene promoter methylation in acute promyelocytic leukaemia: profile and prognostic significance", BRITISH JOURNAL OF HAEMATOLOGY, vol. 122, no. 4, 1 August 2003 (2003-08-01), pages 571-578, XP055334345, GB ISSN: 0007-1048, DOI: 10.1046/j.1365-2141.2003.04462.x**
- **M F ARTEAGA ET AL: "Epigenetics in acute promyelocytic leukaemia pathogenesis and treatment response: A TRAnsition to targeted therapies", BRITISH JOURNAL OF CANCER, vol. 112, no. 3, 3 February 2015 (2015-02-03), pages 413-418, XP055333800, GB ISSN: 0007-0920, DOI: 10.1038/bjc.2014.374**
- **T. SCHOOFS ET AL: "DNA methylation changes are a late event in acute promyelocytic leukemia and coincide with loss of transcription factor binding", BLOOD, vol. 121, no. 1, 3 January 2013 (2013-01-03), pages 178-187, XP055333932, US ISSN: 0006-4971, DOI: 10.1182/blood-2012-08-448860**
- **Yoshitaka Sunami ET AL: "INVOLVEMENT OF HISTONE ACETYLTRANSFERASES IN DEVELOPMENT OF ACUTE PROMYELOCYTIC LEUKEMIA AND RESPONSE TO DIFFERENTIATION-INDUCING COMPOUND", Haematologica, 21 May 2015 (2015-05-21), XP055333452, 20th Congress of the European-Hematology-Association; Vienna, AUSTRIA; June 11 -14, 2015 Retrieved from the Internet: URL:http://learningcenter.ehaweb.org/eha/2015/20th/100298/yoshitaka.sunami.involvement.of.histone.acetyltransferases.in.development.of.html?f=m1 [retrieved on 2017-01-09]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Anonymous: "Data Sheet GeneChip Human Tiling Arrays", , 1 January 2006 (2006-01-01), XP055719760, Retrieved from the Internet: URL:https://www.affymetrix.com/support/tec hnical/datasheets/human_tiling_datasheet.p df [retrieved on 2020-08-03]

SEQUENCE LISTING

<110> EPI-C S.r.L
Altucci, Lucia
Martens, Joost Hendrik Adriaan
Mai, Antonello

<120> Method for the prognosis and/or treatment of acute promyelocytic leukemia

<130> PCT 132779

<150> 102016000051855
<151> 2016-05-20

<160> 16

<170> PatentIn version 3.5

<210> 1
<211> 21
<212> DNA
<213> Artificial Sequence

<220>
<223> Synthetic

<400> 1
gtgttccgcg tgattgaaga c 21

<210> 2
<211> 23
<212> DNA
<213> Artificial Sequence

<220>
<223> synthetic

<400> 2
cagagaaaga agaggagtta taa 23

<210> 3
<211> 18
<212> DNA
<213> Artificial Sequence

<220>
<223> synthetic

<400> 3
cgcagaacag tcgccttg 18

<210> 4
<211> 20
<212> DNA
<213> Artificial Sequence

<220>
<223> synthetic

<400> 4
gtccagagca cgatgtttgg 20

<210> 5
<211> 19
<212> DNA
<213> Artificial Sequence

<220>
<223> synthetic

<400> 5
cgagttggag aggggagtg 19

<210> 6
<211> 20
<212> DNA
<213> Artificial Sequence

<220>
<223> synthetic

<400> 6
aacaaaaccc cagctgtgtg 20

<210> 7
<211> 20
<212> DNA
<213> Artificial Sequence

<220>
<223> synthetic

<400> 7
catcatagct ccagctcccg 20

<210> 8
<211> 20
<212> DNA
<213> Artificial Sequence

<220>
<223> synthetic

<400> 8
catcccggaa agcggttttg 20

<210> 9
<211> 17
<212> DNA
<213> Artificial Sequence

<220>
<223> synthetic

<400> 9
cgatttgcga cagtggg 17

<210> 10
<211> 17
<212> DNA
<213> Artificial Sequence

<220>
<223> synthetic

<400> 10
caggtgcatt tggcgtg 17

<210> 11
<211> 21
<212> DNA
<213> Artificial Sequence

<220>
<223> synthetic

<400> 11
gtcctgcttg tgaaagtttg c 21

<210> 12
<211> 21
<212> DNA
<213> Artificial Sequence

<220>
<223> synthetic

<400> 12
caaatgtctt ttccccatcc t 21

<210> 13
<211> 20
<212> DNA
<213> Artificial Sequence

<220>
<223> synthetic

<400> 13
gaactggggg aggattgtgg 20

<210> 14
<211> 20
<212> DNA
<213> Artificial Sequence

<220>
<223> synthetic

<400> 14
cagcctccgt tatcctggat 20

<210> 15
<211> 21
<212> DNA
<213> Artificial Sequence

<220>
<223> synthetic

<400> 15
tcaacgggaa gcccatcacc a 21

<210> 16
<211> 20
<212> DNA
<213> Artificial Sequence

<220>
<223> synthetic

<400> 16
acggaaggcc atgccagtga 20

## Description

### Technical Field

The present invention relates to a method for the diagnostic of low overall survival acute promyelocytic leukemia and/or of predicting and/or monitoring the response and/or the efficacy of a therapy for acute promyelocytic leukemia or to identify a subject to be treated with an inhibitor of HAT and/or an inhibitor of EZH2 comprising determining the acetylation or methylation status of specific relevant regions and relative kit and microarray. The invention also refers to histone acetyl transferase (HAT) inhibitor for use in the treatment of a solid or hematopoietic tumor.

### Background Art

In recent years, an increasing number of studies have been conducted to gain greater insights into the molecular mechanisms underlying tumorigenesis, and thereby improve the outcome of cancer patients by using new therapeutic approaches (2). Massive parallel sequencing of cancer genomes has identified a myriad of new mutations in genes encoding for enzymes that act as epigenetic modulators of transcription, regulating, amongst others, histone acetylation and histone and/or DNA methylation (1). This scenario suggests that deregulation of the complex genome and epigenome interplay provides a fertile ground for cancer development and progression. Epigenetic deregulation has been reported for 'writers' (3-5), which add acetyl (lysine acetyltransferases, histone acetyl transferases (HATs) or lysine acetyltransferases (KATs) or methyl moieties (lysine methyltransferases, histone methyltransferases (HMT) or lysine methyltransferases (KMTs to histones and other proteins, or a methyl group to DNA (DNMTs, DNA methyltransferases); 'erasers' (5-6), which are able to remove these acetyl (lysine deacetylases, histone deacetylases (HDACs) or lysine deacetylases KDACs, or methyl (lysine demethylases or KDMs) marks, or to participate in the removal of DNA methylation (TET, ten-eleven translocation); and 'readers' (7-12), which functionalize epigenetic marks and modulate downstream signaling, such as methyl-binding proteins (MBD), bromodomain- (BRD), chromodomain-, tudordomain- and plant homeodomain- (PHD) containing proteins (6). Many lines of evidence sustain that aberrant expression of epigenetic enzymes plays a causative role in virtually every step of tumorigenesis, and the reversal of these modifications has therefore emerged as a potential strategy for cancer treatment (13-15). In 2013, The Cancer Genome Atlas (TCGA) research network analyzed the genomes of 200 cases of *de novo* acute myeloid leukemia (AML), demonstrating the presence of at least one non-synonymous mutation in 44% of DNA methylation-related genes and 30% of chromatin-modifying genes (68). This astonishing finding highlights the dual etiology - epigenetic and genetic - of phenomena driving leukemogenesis (17, 18) and likely solid carcinogenesis (19). These statistically-relevant clinical data also indicate the enormous potential of chromatin modulation as treatment against cancer. As a result of these (and other studies) a number of therapeutic compounds targeting epigenetic enzymes have been developed (20-28). Current compounds reaching the clinic are mainly acting to decrease DNA methylation or increase histone acetylation. Azacitidine and decitabine, two DNA hypomethylating agents, are used in myelodysplastic syndrome (MDS) and acute myeloid leukemia (AML) treatment (22). However, in the current settings, they would not be considered curative given the high percentage of relapse cases at variable times after initial response. Also, inhibitors of mutated IDH2/1, both involved in deregulation of DNA methylation levels, entered clinical trials with the orphan drug, fast-track designation from FDA. One HDAC inhibitor has been approved very recently for multiple myeloma (23). Despite their promising anti-cancer activities, usage is currently limited to few AML subtypes (MLLr/11q23 and IDH2/1 AMLs). Finally, tazemetostat and CPI-1205, two inhibitors of Enhancer of zeste homolog 2 (EZH2), which represents the KMT catalytic unit of the polycomb repressive complex 2 (PCR2) that is often overexpressed or mutated in malignancies, entered clinical trials for treatment of B-cell lymphoma (26; 27; 25; 24). Importantly, because cancer relapse due to acquired resistance to treatments remains a major concern, a new generation of 'epidrugs' is urgently needed. Furthermore, some studies have identified methylation profiles that are specific for APL and that in some cases correlate with patients' survival (Maria E. Figueroa ET AL, CANCER CELL, vol. 17, no. 1, 1 January 2010 (2010-01-01), p. 13-27; Chor S. Chim ET AL, British Journal of Haematology, vol. 122, no. 4, 1 August 2003 (2003-08-01), p. 571-578; M F Arteaga ET AL, British Journal of Cancer, vol. 112, no. 3, 3 February 2015 (2015-02-03), p. 413-418; T. Schoofs ET AL, Blood, vol. 121, no. 1, 3 January 2013 (2013-01-03), p. 178-187). However, epigenetic profiles that allow to predict the overall survival specifically for APL patients are not known. Moreover, no profile of hyperacetylated regions has been described before.

### Summary of the invention

The present invention is based on the finding that inhibitors of histone acetyl transferase have anti-cancer therapeutic activity. Further a signature of hyperacetylated regions and hypomethylated regions was identified allowing stratifying low-survival, standard treatment resistant APL patients.

In the present invention, the inventors identify and characterize a novel small molecule displaying inhibitory

actions on both EZH2 and acetyltransferases, displaying this hybrid activity in the low micromolar range. The inventors characterize its anticancer action on a panel of cell lines, *in vivo* tumor models and *ex vivo* patient's blasts. The inventors identify the mechanism of anticancer action as causal activation of mitochondrial apoptotic players. The inventors found that the new compound is able to exert apoptosis in very aggressive models of cancer. Corroborating and strengthening the hypothesis of high anticancer action, the inventors demonstrated its effective action in two very rare cases of acute promyelocytic leukemia patient blasts resistant to standard treatment (APL-R) with a very low survival rate and disease progression to death in days or few weeks. Genomic and epigenomic analyses of these rare APL-R blasts links resistance to a set of loci deregulated in DNA (demethylation) and chromatin hyperacetylation. Thus, the inventors propose application of epigenetic signatures as diagnostic tools for patient stratification and therapeutic direction. In contrast with the current strategies to induce acetylation, acetylation reduction may represent the paradigm shift to reduce tumor recurrence and treatment resistance.

Epigenetic alterations have been associated with both pathogenesis and progression of cancer. Here, the inventors found that the novel epi-drug MC2884 induces *bona fide* cancer-selective cell death in both solid and hematological cancers through the causal mechanism of caspasedependent apoptosis *in vitro, ex vivo* and *in vivo* in xenografts. MC2884 treatment effectively induces massive apoptosis *ex vivo* in All Trans Retinoic Acid (ATRA)/HDACi-resistant primary APL blasts with very poor prognosis as well as in aggressive models of cancer, such as $p53^{-/-}$ or $TET2^{-/-}$ cells suggesting its beneficial effect in late- stage, high-grade and relapsed cancers. Epigenome analysis yielded crucial mechanistic insights into MC2884 action, and histone acetylation and DNA methylation signature profiles for stratification of low survival standard treatment resistant APL patients.

Therefore the invention provides an *in vitro* method for the diagnostic of low overall survival acute promyelocytic leukemia and/or of predicting and/or monitoring the response and/or the efficacy of a therapy for acute promyelocytic leukemia or to identify a subject to be treated with an inhibitor of HAT and/or an inhibitor of EZH2 comprising determining:

a) the acetylation status in all of the following acetylation relevant regions:

| chromosome | start | end |
|---|---|---|
| chr2 | 102761838 | 102765245 |
| chr2 | 138558210 | 138560637 |
| chr3 | 138917149 | 138918464 |
| chr4 | 47722119 | 47725095 |
| chr5 | 177820647 | 177821809 |
| chr5 | 177911126 | 177912881 |
| chr6 | 10080702 | 10085536 |
| chr6 | 100796638 | 100797329 |
| chr7 | 43539997 | 43544821 |
| chr8 | 41107099 | 41109973 |
| chr8 | 41271751 | 41275593 |
| chr8 | 109131198 | 109133248 |
| chr10 | 31145487 | 31151138 |
| chr10 | 31152266 | 31155251 |
| chr10 | 82011240 | 82013721 |
| chr12 | 1741915 | 1746279 |
| chr13 | 110036141 | 110039705 |
| chr13 | 110048447 | 110050505 |
| chr14 | 20955081 | 20960400 |
| chr15 | 97270509 | 97273147 |
| chr17 | 426695 | 431121 |
| chr18 | 61534509 | 61537791 |
| chr18 | 74255741 | 74257346 |
| chr19 | 15742473 | 15746990 |
| chr20 | 48404657 | 48406416 |
| chr21 | 32507312 | 32510923 |
| chr22 | 19230646 | 19232961 |
| chrX | 2850454 | 2853230 |
| chrX | 147574563 | 147578311 |

| chromosome | start | end |
|---|---|---|
| chr1 | 95181929 | 95184074 |
| chr1 | 109626120 | 109627905 |
| chr1 | 249221773 | 249222010 |
| chr11 | 57158202 | 57159391 |
| chr14 | 61416231 | 61417467 |
| chr14 | 105391316 | 105391725 |
| chr17 | 70982083 | 70984392 |
| chr17 | 71061705 | 71070236 |
| chr2 | 27060445 | 27061863 |
| chr2 | 102056668 | 102060845 |
| chr2 | 234072888 | 234075888 |
| chr20 | 2665743 | 2666563 |
| chr22 | 50153595 | 50155770 |
| chr3 | 41011746 | 41014498 |
| chr6 | 10087785 | 10088550 |
| chr6 | 82468660 | 82469114 |
| chr6 | 139948334 | 139949640 |
| chr6 | 140000376 | 140002294 |
| chr7 | 29323864 | 29325989 |
| chr9 | 94907894 | 94910771 |
| chrX | 37605417 | 37607058 |
| chrX | 151152531 | 151154618 |

or

b) the methylation status of all CpG methylation relevant regions of:

| chromosome | start | end |
|---|---|---|
| chr1 | 1052949 | 1052950 |
| chr1 | 12655992 | 12655993 |
| chr1 | 17287501 | 17287502 |
| chr1 | 55266578 | 55266579 |
| chr2 | 237477262 | 237477263 |
| chr3 | 35706114 | 35706115 |
| chr3 | 57198244 | 57198245 |
| chr4 | 3516534 | 3516535 |
| chr4 | 163085348 | 163085349 |
| chr6 | 73972852 | 73972853 |
| chr12 | 57630107 | 57630108 |
| chr12 | 114841708 | 114841709 |
| chr14 | 24779959 | 24779960 |
| chr16 | 1429015 | 1429016 |
| chr16 | 20817501 | 20817502 |
| chr16 | 46919112 | 46919113 |
| chr16 | 67197640 | 67197641 |
| chr17 | 39845949 | 39845950 |
| chr19 | 3275394 | 3275395 |
| chr19 | 35531990 | 35531991 |
| chr19 | 39056084 | 39056085 |
| chr20 | 21687378 | 21687379 |
| chr20 | 62084626 | 62084627 |

Preferably determining the methylation status comprises the steps of:

(a) isolating DNA from a sample obtained from a subject;
(b) subjecting said DNA to methylation-sensitive sequence modification through incubation with chemicals (e.g., sodium bisulfite), or contacting said DNA with a polypeptide capable of binding methylated DNA;
(c) optionally amplifying said DNA modified with sodium bisulfite or bound by said polypeptide; and
(d) determining the methylation status of said DNA.

Preferably determining the acetylation status comprises the steps of comprising the steps of:

(a) isolating DNA from a sample obtained from a subject;
(b) optionally amplifying said DNA; and
(c) determining the acetylation status of said DNA.

The invention also provides a kit for performing the method as defined above, comprising a chemical able to differentially modify DNA sequence depending on methylation status, or a polypeptide or agent capable of binding methylated and/or acetylated DNA, and antibodies, probes, primers and/or aptamers specific for all of the regions as defined above.
The invention also provides the use of the kit as defined above for the diagnostic of resistant acute promyelocytic leukemia and/or of predicting and/or monitoring the response and/or the efficacy of a therapy or to identify a subject to be treated with an inhibitor of HAT and/or an inhibitor of EZH2.
The invention also provides the use of a microarray able to determine the acetylation status in all of the acetylation relevant regions as defined above and the methylation status of all CpG methylation relevant regions as defined above said microarray comprising probes, primers and/or aptamers specific for the regions as defined above for performing the methods as defined above.

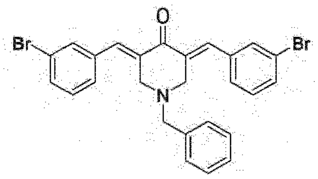

Prognosis is a medical term for predicting the likely outcome of one's current standing.

Acute promyelocytic leukemia (APL) is the M3 subtype of acute myelogenous leukemia (AML). Resistant APL is an APL refractory to standard therapeutic approaches such as 1) all trans retinoic acid (ATRA) and antracyclin-based chemotherapy; 2) the combination treatment of ATRA and arsenic trioxide (ATO).

In the present invention low overall survival acute promyelocytic leukemia are aggressive APLs that, differently from other APLs, bring the patient to death quickly (between few days to few months).

An inhibitor of HAT is an inhibitor of histone acetyl transferase activity and may be any known inhibitor in the art, for instance the ones indicated in 69-70.

An inhibitor of EZH2 is for instance GSK126 or any other known inhibitor, for instance such as indicated in 71-72.

In the present invention any method to determine the acetylation status in at least one acetylation relevant region known to the skilled person is suitable, for instance chromatin immunoprecipitation or MS analyses; ect...including the methods described herein.

In the present invention any method to determine methylation status of at least one cytosine residue in at least one methylation relevant region known to the skilled person is suitable, for instance methylation specific PCR; bisulphite sequencing; array-based technologies, MeDIP analyses; etc... including the methods described herein.

The present invention will be illustrated by means of non-limiting examples in reference to the following figures.

**Figure 1. A.** Overview of the PML-RARA isoforms expressed in patient #7 and #8. **B.** Diagram of the analysis workflow to identify genomic variants in the APL-Rs #7 and #8. **C.** Table showing the top candidate genetic variants identified in #7 and #8. **D.** Venn diagram showing the overlap of PML-RARA binding sites identified in one normal APL and one APL-R. The intersect was used for further analysis. **E.** Heatmap of histone modification densities at promoter and enhancer regions defined in pt#7 and pt#8. **F.** Heatmap of H3K9K14ac densities at promoter and enhancer regions defined in #7 and #8 in two normal APLs and two APL-Rs. **G.** Boxplot showing expression of genes associated with promoter and enhancer regions defined in #7 and #8 in two APL-Rs and one normal APL.

**Figure 2.** Epigenomic analysis of MC2884-responding APL. **A.** Overview of APL ChIP-seq (H3K27ac, H3K4me1, H3K9/14ac) data at the genomic regions of the TGM2, RARB, ASB2 and ID1 genes before and after ATRA treatment. **B.** Boxplot showing changes in H3K9K14ac before and after treatment with ATRA at PML-RARA binding sites in one normal APL and two APL-Rs. * indicates significance at $p < 0.01$ using the Wilcoxon Signed-Rank test. **C.** Boxplot showing H3K27ac levels of normal and APL-Rs, $CD34^+$ cells and neutrophil progenitor populations at enhancer regions defined in APL-Rs. *$p<0.01$ for all pairwise comparisons between APL-Rs and other APLs/progenitors. **D.** Boxplot showing H3K27ac levels of normal APL, high risk APL, APL-R and 27 AMLs with a different genetic status. *$p<0.01$ for all pairwise comparisons between high risk APL/APL-Rs and other APLs/AMLs. E. Left. Heatmap of H3K27me3 levels on differential regions between normal APL and APL-R samples. Right.
Overview of H3K27ac, H3K36me3 and H3K27me3 levels at the ZSCAN12 genomic region in normal APL and APL-R samples.

**Figure 3. A.** Tag density profile (RPKM) of H3K27ac in APL-R/high risk APL (#A, #B, #7 and #8), normal APL (#9) and other AMLs at 22 genomic regions identified as hyperacetylated in APL-Rs. **B.** Event free survival (EFS) and Overall Survival (OS) in 13 APL cases described and analyzed in Ley et al. (2013). **C.** Supervised clustering of DNA methylation patterns using 5 low OS/APL-R and 10 high OS/normal APLs. **D.** Heatmap of histone modification density at the two clusters defined in (C).

**Figure 4.** Low OS APLs represent a defined subgroup of AMLs. (Top) DNA methylation patterns of 181 AMLs over the two APL clusters defined in Figure S2B. (Bottom) DNA methylation patterns using 181 AML and 15 APLs over 23 CpGs hypomethylated in APL-Rs.

**Figure 5. A.** Chemical structure and **B.** half-life in culture medium of MC2884. **C.** MC2884 impairs PRC2 expression (RNA) in NB4 cells at 48 hrs. **D.** MC2884 modulates H3K27ac, H3K27me3 and H3K9/14ac in Kelly neuroblastoma cells. Histone H4 represents the loading control. **E.** MC2884 does not inhibit HDAC1 and SIRT1 action in vitro. SAHA (HDACi; 5 $\mu$M) and EX527 (SIRTi; 5 $\mu$M) have been used as positive controls, respectively. **F.** Cell death induced by MC2884 in the indicated cancer cells and **G.** in normal cells at the indicated times and concentrations. **H.** Anti-proliferative action of MC2884 in colon cancer cells measured in real time. **I.** Migration inhibitory action of MC2884 measured in colon cancer cells as Slope (1/h).

**Figure 6.** Analysis of the epigenetic impact of MC2884. **A.** Simultaneous but not single inhibition of EZH2 and p300

displays anticancer effect in NB4 APL cells, as shown by FACS analysis (top). Western blot analyses for H3K9/14ac, H3K27me3 and EZH2 showing the efficacy of the indicated treatments (bottom). **B.** MC2884 inhibits EZH2 activity *in vitro.* **C.** MC2884 modulates H3K27me3, H3K27ac, EZH2 expression and H3K9/14ac in NB4 APL cells. * indicates that the MC2884 has been added a second time after 10 h. Histone H1 and tubulin represent loading controls. **D**. MC2884 inhibits HAT activity in total cell extracts. **E**. MC2884 inhibits the indicated HATs *in vitro.*

**Figure 7. A.** Cell cycle progression analyses by FACS in several cell lines at the indicated times and concentrations of MC2884. **B.** Anti-proliferative action of the MC2884 in NB4 APL leukemia cells measured in real time. **C.** Differentiation potential of MC2884 in NB4 cells at 30 hrs of treatment. ATRA (1 μM) was used as positive control. **D.** Analysis of the induction of cell death and cell cycle progression at the indicated concentration of MC2884 and time points in Kelly, Hela and U87 cancer cell lines.

**Figure 8.** MC2884 displays anticancer action in both hematological and solid cancer *in vivo.* **A.** *In vivo* experimental design for leukemia xenografts treated with MC2884 (1 mg/Kg). At day 16, bone marrow (BM) cells were isolated. MC2884 antiproliferative action was evaluated by analyzing the % of human CD45+ cells in the isolated population. **B.** Colon cancer (HCT116) xenograft. *In vivo* growth expressed in volume of tumors induced. **C, D.** Ki67 (proliferation index) and TUNEL (apoptotic index) scores were analyzed at the end of treatment. The proliferation index was significantly lower in tumors of treated mice compared to controls (p=0.002). Apoptotic index was significantly higher in tumors of MC2884- treated mice (p=0.002). Immunohistochemical analyses and statistical evaluation for the apo-index, Ki67, H3K9/14ac, H3K27me3 showed significant alterations (increased apo-index and decreased Ki67, H3K9/14ac, H3K27me3) in MC2884-treated versus control (vehicle) cells (p=0.002). **E.** Boxplot of H3K27ac levels at hyperacetylated regions in HCT116 cells before and after treatment for 16h with MC2884. **F.** H3K27ac levels at the BIRC5 locus before and after treatment with MC2884. **G.** Boxplot of H3K27ac levels at hyperacetylated regions in HCT116-DKO cells before and after treatment for 16h with MC2884.

**Figure 9.** In vivo quantification of the anticancer action of MC2884 in xenograft models of NB4 luciferase expressing cells (A) and in HCT116p53-/- colon cancer **(B). C.** Caspase 3/7, 8 and 9 assay upon MC2884 treatment in presence of the indicated inhibitors using NB4 APL cells. SAHA (5 μM) has been used as positive control. **D.** Analysis of DNA fragmentation in NB4 cells upon treatment with MC2884 in presence of Caspase 8 or 9 inhibitors where indicated. **E.** Analysis of DNA fragmentation in NB4 cells in presence of NAC at the indicated concentration. H2O2 has been used as positive control.

**Figure 10.** MC2884 induce anticancer action in cell lines, *ex vivo* AMLs, ALL, aggressive models of cancer and treatment-resistant APLs. **A.** Cleaved BID, BAX, BCL2, BCLX(L), PARP, TRAIL and RIP expression levels in NB4 treated with MC2884 at the indicated time. * indicates that the MC2884 has been added a second time after 10 h. ERK has been used as reference for loading. **B.** BCL2 RNA expression levels in NB4 treated with MC2884 for 48 hours. C. Analysis of cell death induced by MC2884 in *ex vivo* AML blasts (24 h). **D.** Analysis of cell death induced by MC2884 in *ex vivo* ALL blasts (24 h). **E.** Analysis of cell death induced by MC2884, ATRA and the HDACi SAHA or MS275 in *ex vivo* APL blasts (24 h). **F.** Analysis of apoptosis in APL-Tet2wt and APL-Tet2-/- cells upon treatment with various concentration of MC2884. Shown is the proportion of dying cells, normalized to non-treated conditions. Statistical significance is indicated by stars. **G.** Analysis of cell death induced by MC2884 in HCT116 p53-/- colon cancer cells.

**Figure 11. A.** Boxplot of H3K27ac levels at hyperacetylated regions in APL-wt (left) and APL-Tet2-/- (right) cells either not (control) or MC2884 treated. **B.** H3K27ac levels at two genomic loci in APL-wt (top) and APL-Tet2-/- (bottom) cells either not (control) or MC2884 treated. **C.** Heatmap showing H3K27ac changes at promoter and enhancer regions upon ex vivo MC2884 treatment of APL-R (#8) cells. **D.** Heatmap showing H3K27me3 changes at genomic regions upon ex vivo MC2884 treatment of APL-R cells. **E.** Overview of H3K27ac at the AK2 and SEC23A genomic regions in control or MC2884 ex vivo treated APL-R cells. **F.** Table showing functional annotation of genes associated with enhancers that have reduced H3K27ac after ex vivo MC2884 treatment of APL-R (#8).

**Figure 12.** Epigenetic changes upon MC2884 treatment result in reduced BCL2 expression. **A.** Boxplot and heatmap showing H3K27ac changes at enhancer regions that show reduced occupancy levels after *ex vivo* MC2884 treatment of an APL-R sample. * indicates significance at p <0.01. **B.** Overview of H3K27ac, H3K4me1 and H3K4me3 at the BCL2 genomic region in control or MC2884 *ex vivo* treated APL-R cells. C. Overview of H3K27ac at the EZH2 gene in control or MC2884 *ex vivo* treated APL-R cells. **D.** Expression of genes associated with enhancers (distance to gene < 50kb and RPKM sum >100; left) reduced in H3K27ac or of genes associated with promoters with reduced H3K27me3 (RPKM sum > 0.5). * indicates significance at p <0.01. **E.** BCL2 mRNA expression levels in APL-R (#8) and normal APL (#9) blasts. MC2884 has been used at 3 μM, HDACi at 5 μM, ATRA at 1 μM. **F.** Left. ChIP in NB4 cells using H3K27ac antibodies and primers for the BCL2 promoter. Right. ChIP in NB4 cells using H3K27me3 antibodies and primers for the CBS and SLC52A1 promoters. **G.** BCL2 expression by RT-qPCR (top) and western (bottom) after the indicated treatments. **H.** % of ATRA responsive (NB4) and ATRA resistant (NB4-MR4) cells in pre-G1 upon the indicated treatments.

**Figure 13.** Surface Plasmon Resonance. SPR analyses were performed on a Biacore 3000 optical biosensor

corroborating and strengthening p300 direct binding of MC2884.

**Figure 14.** Analysis of histone & non-histone target modulation by MC2884 and analogues in NB4 cells. Western blot analyses (and their quantification by ImageJ) performed on NB4 cells to detect the levels of acetyl-H3K9-14 (as a marker of HAT/anti-HAT activity) and H3K27me3/2 (as marker of PRC2/EZH2 activity) showed a decrease of histone acetylation and methylation after treatment with 3 μM compounds for 48 h, thus confirming the role of dual HAT/EZH2 inhibitors for these derivatives. In addition, the tested compounds also lowered the level of the EZH2 protein.

**Figure 15.** Up. Evaluation of modulation of histone and non-histone targets of MC2884 and its anticancer inactive analogues in NB4 cells. Bottom. qPCR for bcl2 expression at the indicated time points. Compounds inactive in inducing cell death in both cell lines such as MC2910, 2912, 2909, 2886, 2911, 3395 where also unable to modulate histone acetylation and EZH2 deregulation (SAR-Fig. 2). In full agreement, only MC2884 was able to down-regulate BCL2 expression levels at both RNA (histogram bars) and protein levels (western blot) suggesting that this action is crucial for the induction of apoptosis.

**Figure 16.** Both overexpression of p300 and EZH2 improves MC2884 anticancer action; the expression of their catalytic mutants instead, reduces MC2884 induction of cell death. To causally connect the anticancer action with the dual p300 and EZH2 targeting of the drug, we evaluated MCF7 cells viability after overexpression of WT or catalytic mutants of p300 or EZH2, after treatment with MC2884. Interestingly, expression of p300 or EZH2 to higher levels leaded to increased cell death after MC2884, suggesting that both targets are important for the anticancer action of the drug in these settings. When catalytic mutants where used, only the mutant p300 expression fully reversed the MC2884 induction of cell death, whereas the mutant EZH2 gave rise to a partial reversion, potentially suggesting that both activities are needed but p300 inhibitory enzymatic action might be prevalent. Note that MC2884 action on EZH2 expression levels (and thus action that might be additional to the enzymatic regulation) is also potentially playing a role.

**Figure 17.** Mouse terminal IP PK study For ip dosing. After formulation assessment, MC2884 was dissolved at 0.2 mg/mL. This provided a dose of 2 mg free base material/kg when administered ip in a 10 mL/kg dosing volume. Compound MC2884 was given ip at a dose of 2 mg/kg to a group of 24 normally fed male CD1 mice in a 10 mL/kg injection volume. Terminal blood samples (> 230 μL) were taken under CO2 from groups of 3 mice at each of 8 time-points post dose (0.083, 0.25, 0.5, 1, 2, 4, 8 and 24 h) and placed into heparinized tubes. Samples were placed on ice for no longer than 30 min before centrifugation (10,000 rpm x 3 min). Plasma samples (>100 μL) were collected into fresh tubes and frozen on dry ice. All samples were stored at -20 °C. Number of plasma samples = 24.

**Figure 18.** Mouse terminal IV PK study. For iv dosing. Compound was dissolved at 0.4 mg/mL. This provided a dose of 2 mg free base material/kg when administered iv in a 5 mL/kg dosing volume. Compound MC2884 was given iv via the lateral tail vein at a dose of 2 mg/kg to a group of 24 normally fed male CD1 mice in a 5 mL/kg injection volume. Terminal blood samples (> 230 μL) were taken under CO2 from groups of 3 mice at each of 8 time-points post dose (0.083, 0.25, 0.5, 1, 2, 4, 8 and 24 h) and placed into heparinized tubes. Samples were placed on ice for no longer than 30 min before centrifugation (10,000 rpm x 3 min). Plasma samples (>100 μL) were collected into fresh tubes and frozen on dry ice. Number of plasma samples = 24. Consistent IV profiles were obtained, with little variability across the time-points (the average profile is shown in the second spreadsheet). An average half-life of 4.58h was calculated.

## Detailed description of the invention

### Materials and Methods

### Cell culture

U937, NB4, HL60, MCF7, MDA MB-231, HCT116, HeLa, Kelly and U87 tumour cell lines were purchased by DSMZ (NB4) and American Type Culture Collection (ATCC). Cell lines have been tested and authenticated following manufacturer's instruction. All cell lines were maintained in an incubator at a temperature of 37°C and 5% CO2 in a fully humidified atmosphere. The human leukemia cells U937, NB4 and HL60 were grown in RPMI-1640 (Sigma-Aldrich) culture medium; while human breast cancer cells MCF-7, MDA MB-231, HCT116, HeLa and Kelly cells were grown in Dulbecco's Modified Eagle Medium (DMEM) (Sigma) culture medium. Both culture media containing phenol red (GIBCO), were added with 1% L-glutamine (EuroClone), 10% heat-inactivated Fetal Bovine Serum (FBS) (Sigma) and antibiotics (100U/mL of penicillin, 100 mg/mL of streptomycin and 250 mg/mL of amphotericin-B). Endometrial Stromal Cells (ESC) were grown in DMEM-F12 culture medium with 10% FBS, 2mM L-glutamine (Euroclone) and antibiotics (100U/mL of penicillin, 100 mg/mL of streptomycin and 250 mg/mL of amphotericin-B). MePR2B were grown as previously reported (29).

**Chemicals**

SAHA (Merck), MS-275 (Alexis) and MC2884 were dissolved in dimethyl sulfoxide (DMSO) (Sigma); ATRA was obtained from Sigma.

**Chemistry**

The compounds MC2886, MC2911, MC2912, MC2913, MC2914, MC2908, MC2884, MC2909 and MC2910 were prepared through aldol condensation between the cyclic ketones and the properly substituted benzaldehyde in presence of barium hydroxide octahydrate and methanol at room temperature (Scheme 1, 2). By performing the condensation like in scheme 1 and 2 the 3,5-bis-(3-bromobenzylidene)piperidin-4-one scaffold was synthesized (Scheme 3), afterwards it underwent *N*-alkylation with corresponding alkyl chloride by using anhydrous potassium carbonate in acetonitrile at 60 °C for providing MC3269, MC3272 and MC3207 (Scheme 3). Differently, MC3146 and MC3187 were prepared by treating 3,5-bis-(3-bromobenzylidene)piperidin-4-one with appropriate acyl chlorides in presence of triethylamine and dichloromethane at 0 °C. (Scheme 3). Only for derivative MC3395 the aldol condensation was performed by using 4N hydrochloric acid in ethanol refluxing for 2 days (Scheme 4)(53-56).

**Scheme 1**[a]

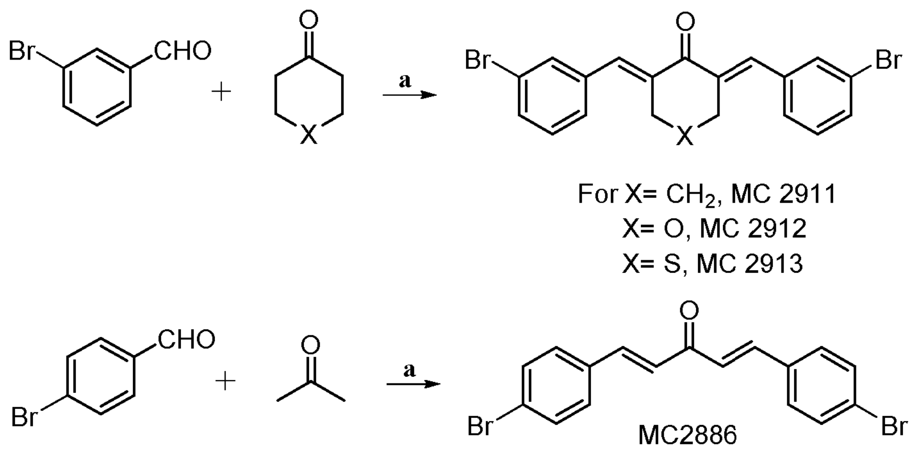


For X= $CH_2$, MC 2911
X= O, MC 2912
X= S, MC 2913

MC2886

**Scheme 2**[a]

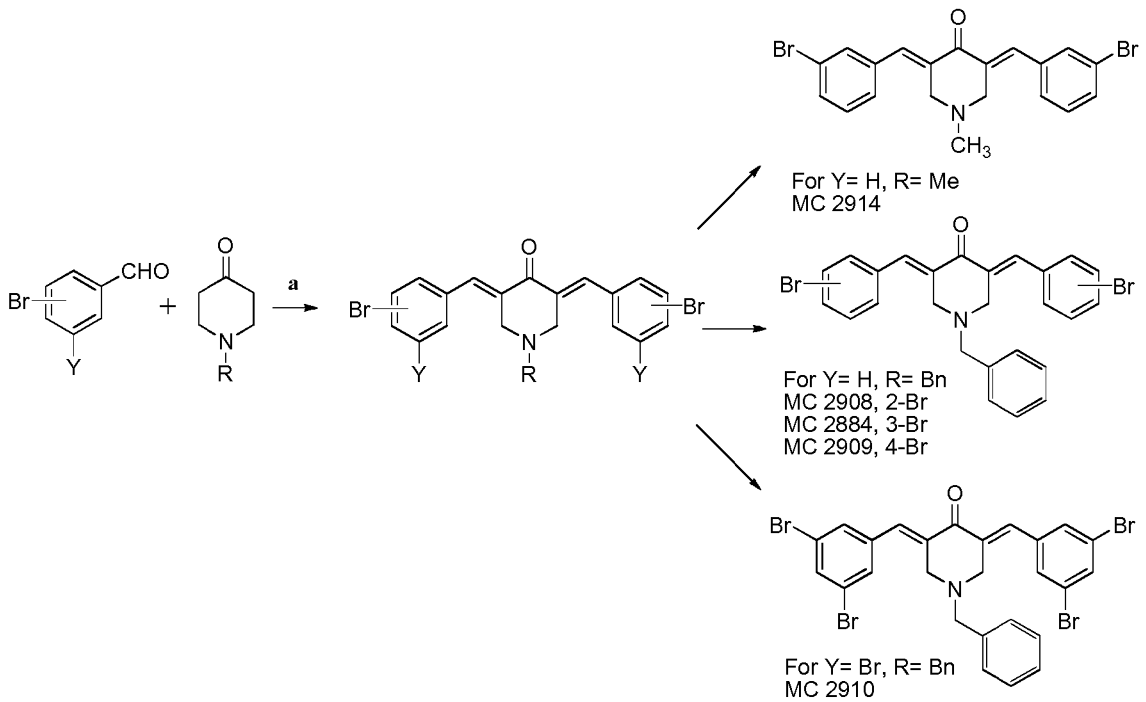


For Y= H, R= Me
MC 2914

For Y= H, R= Bn
MC 2908, 2-Br
MC 2884, 3-Br
MC 2909, 4-Br

For Y= Br, R= Bn
MC 2910

**Scheme 3**[a]

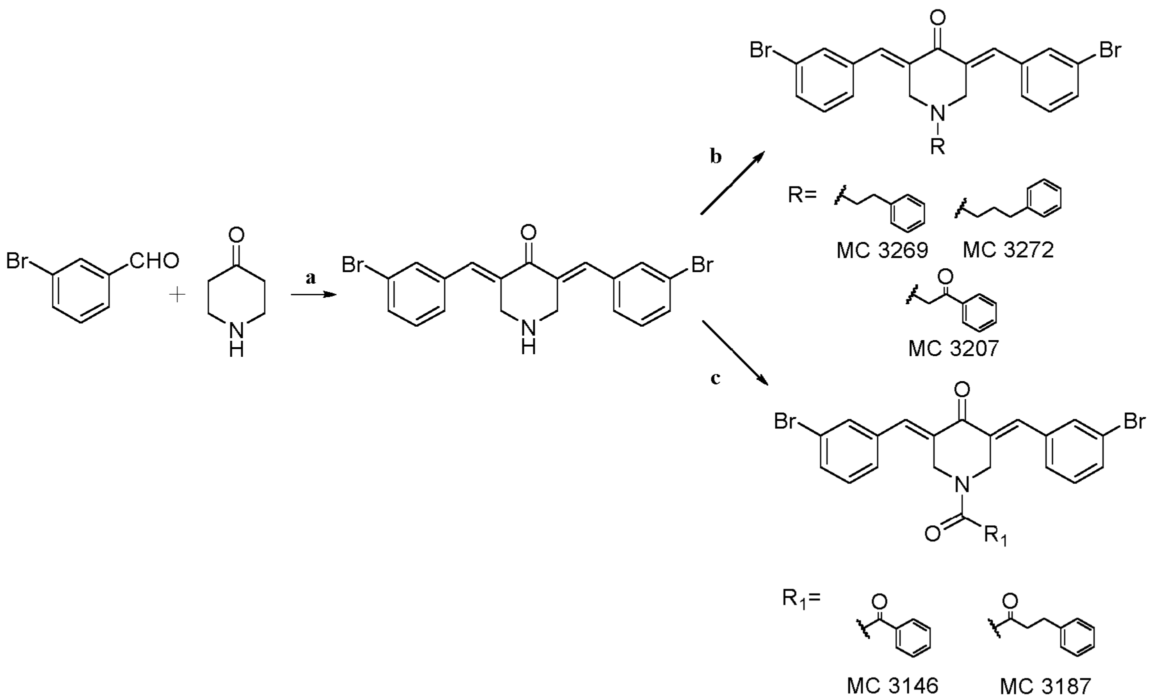


MC 3269 MC 3272

MC 3207

MC 3146 MC 3187

**Scheme 4[a]**

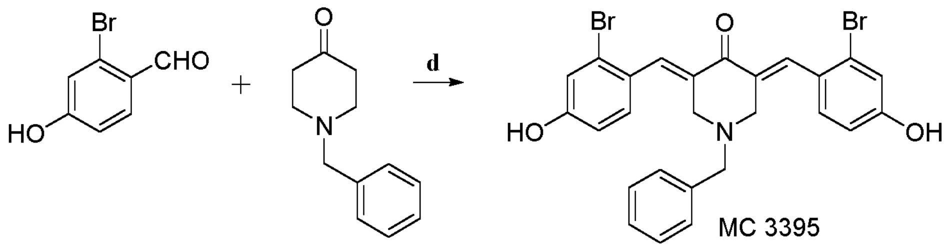


MC 3395

[a]*Reagents and conditions* (a) $Ba(OH)_2 \times 8\ H_2O$, $CH_3OH$, r.t., 2 h; b) alkyl bromide, $K_2CO_3$, $CH_3CN$, 60°C, 2 h; c) $Et_3N$, acyl chloride, DCM, 0°C, 1 h; d) 4N HCl, ethanol, 100°C, 2 days.

Melting points were determined on a Buchi 530 melting point apparatus and are uncorrected. $^1H$ NMR and $^{13}C$ NMR spectra were recorded at 400 and 100 MHz, respectively, on a Bruker AC 400 spectrometer; chemical shifts are reported in $\delta$ (ppm) units relative to the internal reference tetramethylsilane ($Me_4Si$). EIMS spectra were recorded with a Fisons Trio 1000 spectrometer; only molecular ions ($M^+$) and base peaks are given. All compounds were routinely checked by TLC and 1H NMR. TLC was performed on aluminum-backed silica gel plates (Merck DC, Alufolien Kieselgel 60 F254) with spots visualized by UV light. All solvents were reagent grade and, when necessary, were purified and dried by standard methods. Concentration of solutions after reactions and extractions involved the use of a rotary evaporator operating at reduced pressure of ca. 20 Torr. Organic solutions were dried over anhydrous sodium sulfate. Elemental analysis has been used to determine purity of the described compounds, that is, >95%. Analytical results are within $\pm$0.40% of the theoretical values. All chemicals were purchased from Sigma-Aldrich, Milan (Italy), or from Alfa Aesar, Karlsruhe (Germany), and were of the highest purity.

**General Procedure for the Synthesis of MC2908, MC2884, MC2886, MC2909, MC2910, MC 2911, MC2912, MC2013, MC2914.**

**Example: synthesis of 1-benzyl-3,5-bis(3-bromobenzylidene)piperidin-4-one (MC 2884).**

1-benzylpiperidin-4-one (1.06 mmol, 0.2 mL) was added to a suspension of barium hydroxide octahydrate (4.24 mmol, 1.34 g) in methanol (10 mL), and the mixture was stirred for 5 min. Then a solution of 3- bromo-benzaldehyde (2.12 mmol, 0.39 g) in methanol (10 mL) was added, and the resultant mixture was stirred at room temperature. After 2 hours water was added and the resulting suspension was filtered, the precipitate was washed with water (3 x 10 mL), dried and recrystallized to afford the pure product. $^1H$-NMR (DMSO-$d_6$) $\delta_H$/ppm: 3.71 (s, 2H, Ph$CH_2$), 3.81 (s, 4H, N($CH_2$)$_2$, 7.19 (s, 5H, benzene protons), 7.37-7.45 (m, 4H, benzene protons), 7.60-7.65 (m, 6H, benzene protons and PhC*H*=CCO); $^{13}C$-NMR (DMSO-$d_6$) $\delta_C$/ppm: 53.4 (2C), 64.4, 123.0 (2C), 127.2, 127.5 (2C), 128.4 (2C), 128.8 (2C), 129.6 (2C), 130.8 (2C), 132.6, 132.7 (2C), 136.1 (2C), 140.6 (2C), 145.9 (2C), 186.0; MS (EI): *m*/*z* $[M+H]^+$: 523.46.

**(1E,4E)-1,5-bis(4-bromophenyl)penta-1,4-dien-3-one (MC 2886).**

$^1H$-NMR (DMSO-$d_6$) $\delta_H$/ppm: 7.36-7.40 (d, 2H, PhC*H*=CHCO), 7.67-7.80 (m, 10H, benzene protons and Ph-CH=C*H*CO); $^{13}C$-NMR (DMSO-$d_6$) $\delta_C$/ppm: 122.5 (2C), 123.4 (2C), 128.7 (4C), 131.6 (4C), 134.4 (2C), 142.1 (2C), 188.8; MS (EI): *m*/*z* $[M+H]^+$: 389.93.

**1-benzyl-3,5-bis(2-bromobenzylidene)piperidin-4-one (MC 2908).**

$^1H$-NMR (DMSO-$d_6$) $\delta_H$/ppm: 3.60 (s, 2H, Ph$CH_2$), 3.71 (s, 4H, N($CH_2$)$_2$, 7.16 (s, 5H, benzene protons), 7.30-7.42 (m, 7H, benzene protons), 7.73-7.75 (m, 4H, benzene protons and PhC*H*=CCO); $^{13}C$-NMR (DMSO-$d_6$) $\delta_C$/ppm: 53.4 (2C), 64.4, 125.2 (2C), 127.0 (2C), 127.2, 127.5 (2C), 127.6 (2C), 128.4 (2C), 128.8 (2C), 132.6 (3C), 134.3 (2C), 140.6 (2C), 145.9 (2C), 186.0; MS (EI): *m*/*z* $[M+H]^+$: 522.70.

**1-benzyl-3,5-bis(4-bromobenzylidene)piperidin-4-one (MC 2909).**

$^1H$-NMR (DMSO-$d_6$) $\delta_H$/ppm: 3.71 (s, 2H, Ph$CH_2$), 3.80 (s, 4H, N($CH_2$)$_2$, 7.20-7.25 (m, 5H, benzene protons), 7.39 (m, 4H, benzene protons), 7.58-7.64 (m, 6H, benzene protons and PhC*H*=CCO); $^{13}C$-NMR (DMSO-$d_6$) $\delta_C$/ppm: 53.0 (2C), 57.8, 124.0 (2C), 125.3, 126.8 (2C), 128.3 (2C), 130.7 (4C), 133.0 (4C), 134.9, 136.3 (2C), 138.9 (2C), 141.7

(2C), 187.4; MS (EI): *m/z* $[M+H]^+$: 523.78.

**1-benzyl-3,5-bis(3,5-dibromobenzylidene)piperidin-4-one (MC 2910).**

$^1$H-NMR (DMSO-$d_6$) $\delta_H$/ppm: 3.71 (s, 2H, Ph*CH$_2$*), 3.79 (s, 4H, N(C*H$_2$*)$_2$, 7.22 (s, 4H, benzene protons), 7.56 (s, 2H, benzene protons), 7.87 (s, 2H, PhC*H*=CCO); $^{13}$C-NMR (DMSO-$d_6$) $\delta_C$/ppm: 53.4 (2C), 64.4, 123.3 (4C), 127.2, 128.4 (2C), 128.8 (2C), 128.9 (4C), 132.6, 133.9 (2C), 139.6 (2C), 140.6 (2C), 145.9 (2C), 186.0; MS (EI): *m/z* $[M+H]^+$: 680.82.

**2,6-bis((E)-3-bromobenzylidene)cyclohexan-1-one (MC 2911).**

$^1$H-NMR (DMSO-$d_6$) $\delta_H$/ppm: 1.71-1.74 (m, 2H, -*CH$_2$*-cyclohexanone), 2.87-2.90 (t, 4H, -*CH$_2$*-cyclohexanone), 7.40-7.44 (t, 2H, benzene protons), 7.54-7.61 (m, 6H, benzene protons and Ph*CH*-), 7.73 (s, 2H, benzene protons); $^{13}$C-NMR (DMSO-$d_6$) $\delta_C$/ppm: 25.1, 26.3 (2C), 123.1 (2C), 127.6 (2C), 129.5 (2C), 130.9 (2C), 132.4 (2C), 132.7 (2C), 136.3 (2C), 137.2 (2C), 190.6; MS (EI): *m/z* $[M+H]^+$: 429.96.

**3,5-bis(3-bromobenzylidene)dihydro-2H-pyran-4(3H)-one (MC 2912).**

$^1$H-NMR (DMSO-$d_6$) $\delta_H$/ppm: 4.91 (s, 4H, O*(CH$_2$)$_2$*, 7.43-7.45 (m, 4H, benzene protons), 7.65-7.69 (m, 6H, benzene protons and PhC*H*=CCO); $^{13}$C-NMR (DMSO-$d_6$) $\delta_C$/ppm: 67.3 (2C), 123.0 (2C), 127.5 (2C), 129.6 (2C), 130.8 (2C), 132.7 (2C), 136.1 (2C), 143.8 (2C), 146.1 (2C), 186.0; MS (EI): *m/z* $[M+H]^+$: 433.93.

**3,5-bis(3-bromobenzylidene)dihydro-2H-thiopyran-4(3H)-one (MC 2913).**

$^1$H-NMR (DMSO-$d_6$) $\delta_H$/ppm: 3.97 (s, 4H, O*(CH$_2$)$_2$,* 7.41-7.45 (t, 2H, benzene protons), 7.53-7.57 (m, 4H, benzene protons), 7.61-7.63 (d, 2H, benzene protons), 7.73 (s, 2H, PhC*H*=CCO); $^{13}$C-NMR (DMSO-$d_6$) $\delta_C$/ppm: 30.2 (2C), 123.0 (2C), 127.5 (2C), 129.6 (2C), 130.8 (2C), 132.7 (2C), 136.1 (2C), 139.0 (2C), 146.9 (2C), 186.0; MS (EI): *m/z* $[M+H]^+$: 449.91.

**3,5-bis(3-bromobenzylidene)-1-methylpiperidin-4-one (MC2914).**

$^1$H-NMR (DMSO-$d_6$) $\delta_H$/ppm: 2.39 (s, 3H, N*CH$_3$*), 3.73 (s, 4H, (C*H$_2$*)$_2$NCH$_3$), 7.42-7.46 (m, 2H, benzene protons), 7.50-7.52 (d, 2H, benzene protons), 7.57 (s, 2H, PhC*H*=CCO), 7.62-7.64 (d, 2H, benzene protons), 7.71 (s, 2H, benzene protons); $^{13}$C-NMR (DMSO-$d_6$) $\delta_C$/ppm: 45.0, 56.1 (2C), 123.0 (2C), 127.6 (2C), 129.8 (2C), 130.8 (2C), 132.6 (2C), 136.0 (2C), 140.8 (2C), 145.9 (2C), 186.1; MS (EI): *m/z* $[M+H]^+$: 445.97.

General Procedure for the Synthesis of MC3269, MC3272, MC3207.

**Example: synthesis of 3,5-bis(3-bromobenzyliden)-1-(3-phenylpropyl) piperidin-4-one (MC 3272).**

To a suspension of anhydrous $K_2CO_3$ (0.69 mmol, 0.954 g) in acetonitrile (10 mL), the 3,5-bis(3-bromobenzyliden)piperidin-4-one (0.46 mmol, 0.2 g) and the 3-bromo-1-phenylpropane (1.38 mmol, 0.21 mL) were added and the resulting suspension was stirred at 60 °C. After 2 hours the solvent was evaporated, water (50 mL) was added and the aqueous solution was extracted with dichloromethane (3 x30 mL). The collected organic phases were washed with a saturated solution of NaCl (30 mL) and then dried with anhydrous $Na_2SO_4$, filtered and evaporated under vacuum to afford a crude that was purified on silica gel (AcOEt/n-hexane 1:3) to obtain the desired compound. $^1$H-NMR (DMSO-$d_6$) $\delta_H$/ppm: 1.65-1.71 (m, 2H, - NCH$_2$C*H$_2$*CH$_2$Ph), 2.47-2.51 (t, 2H, -NCH$_2$CH$_2$C*H$_2$*Ph), 2.54-2.58 (t, 2H, -NC*H$_2$*CH$_2$CH$_2$Ph), 3.68 (s, 4H, piperidonic protons), 7.05-7.38 (m, 11H, benzene protons), 7.67-7.69 (d, 2H, benzene protons), 7.96 (s, 2H, PhC*H*=CCO); $^{13}$C-NMR (DMSO-$d_6$) δc/ppm:27.5, 31.0, 53.8 (2C), 57.0, 125.2 (2C), 126.0, 127.0 (2C), 127.5 (2C), 127.6 (2C), 128.1 (2C), 128.8 (2C), 132.6 (2C), 134.3 (2C), 140.6 (2C), 142.0, 145.9 (2C), 186.0; MS (EI): m/z $[M+H]^+$: 551.41.

**3,5-bis(3-bromobenzylidene)-1-phenethylpiperidin-4-one (MC3269).**

$^1$H-NMR (DMSO-$d_6$) $\delta_H$/ppm: 2.73-2.77 (t, 2H, NCH$_2$C*H$_2$*Ph), 2.83-2.86 (t, 2H, NC*H$_2$*CH$_2$Ph), 3.87 (s, 4H, (*CH$_2$)*$_2$NCH$_2$CH$_2$Ph), 7.13-7.32 (m, 9H, benzene protons), 7.52-7.53 (m, 4H, benzene protons), 7.75 (s, 2H, PhC*H*=CCO); $^{13}$C-NMR (DMSO-$d_6$) $\delta_C$/ppm: 33.6, 53.9 (2C), 58.9, 123.1 (2C), 125.8, 127.5 (2C), 127.7 (2C), 128.8 (2C), 129.6 (2C), 130.7 (2C), 132.8 (2C), 136.3 (2C), 139.5, 140.6 (2C), 145.8 (2C), 186.2; MS (EI): m/z $[M+H]^+$: 536.01.

**3,5-bis(3-bromobenzylidene)-1-(2-oxo-2-phenylethyl)piperidin-4-one (MC3207).**

$^1$H-NMR (DMSO-$d_6$) $\delta_H$/ppm: 4.09 (s, 4H, (C$H_2$)$_2$NCH$_2$COPh), 4.11 (s, 2H, NC$H_2$COPh), 7.31-7.56 (m, 11H, benzene protons), 7.77 (s, 2H, PhC*H*=CCO), 7.92-7.94 (d, 2H, benzene protons); $^{13}$C-NMR (DMSO-$d_6$) $\delta_C$/ppm: 53.2 (2C), 71.0, 123.2 (2C), 127.5 (2C), 128.7 (2C), 128.8 (2C), 129.8 (2C), 131.0 (2C), 132.7 (2C), 133.2, 135.3, 136.2 (2C), 140.7 (2C), 146.2 (2C), 186.1, 195.5; MS (EI): m/z $[M+H]^+$: 549.99.

**General Procedure for the Synthesis of MC3146, MC3187. Example: synthesis of 3,5-bis(3-bromobenzylidene)-1-(3-phenylpropanoyl) piperidin-4-one (MC3187).**

To a stirring solution of 3,5-bis(3-bromobenzylidene)piperidin-4-one (0.45 mmol, 195 mg) and $Et_3N$ (0.76 mmol, 0.11 mL) in dry dichloromethane (5 mL), hydrocinnamoyl chloride (0.67 mmol, 0.1 mL) was slowly added at 0 °C. The resulting mixture is then allowed to stir at room temperature. After 1 hour the reaction was quenched with water (50 mL) and extracted with dichloromethane (3 × 30 mL). The collected organic layers were washed with HCl 2N (3 × 30 mL) and then with a saturated solution of NaCl (30 mL). The organic phase was dried with anhydrous $Na_2SO_4$, filtered and evaporated under vacuum to afford a crude residue that was then purified on silica gel (AcOEt/n-hexane 1:3) to afford the desired product. $^1$H-NMR (DMSO-$d_6$) $\delta_H$/ppm: 2.45-2.49 (t, 2H, -COC$H_2$), 2.83-2.87 (t, 2H, PhC$H_2$), 4.57 (s, 2H, -NC$H_2$), 4.90 (s, 2H, -NC$H_2$), 6.98-7.00 (d, 2H, benzene protons), 7.18-7.63 (m, 9H, benzene protons), 7.77 (s, 2H, benzene protons); $^{13}$C-NMR (DMSO-$d_6$) $\delta_C$/ppm: 31.4, 33.6, 47.4 (2C), 123.0 (2C), 125.9, 127.5 (2C), 127.7 (2C), 128.6 (2C), 129.6 (2C), 130.8 (2C), 132.7 (2C), 136.1 (2C), 140.6 (2C), 141.3, 145.9 (2C), 172.3, 186.0; MS (EI): *m*/*z* $[M+H]^+$: 565.24.

**1-benzoyl-3,5-bis(3-bromobenzylidene)piperidin-4-one (MC3146).**

$^1$H-NMR (DMSO-$d_6$) $\delta_H$/ppm: 4.68 (s, 2H, -NC$H_2$), 4.95 (s, 2H, -NC$H_2$), 7.15-7.36 (m, 9H, benzene protons), 7.50-7.86 (m, 6H, benzene protons); $^{13}$C-NMR (DMSO-$d_6$) $\delta_C$/ppm: 47.9 (2C), 123.1 (2C), 127.3 (2C), 127.7 (2C), 128.6 (2C), 129.7 (2C), 129.9, 130.9 (2C), 132.7 (2C), 135.4, 136.3 (2C), 140.7 (2C), 145.8 (2C), 169.6, 186.2; MS (EI): m/z $[M+H]^+$: 535.98.

**Synthesis of 1-benzyl-3,5-bis((E)-2-bromo-4-hydroxybenzylidene)piperidin-4-one (MC3395).**

A solution of 2-bromo-4-hydroxy-benzaldehyde (0.5 g, 2.49 mmol) and 1-benzyl-4-piperidone (1.24 mmol, 0.235 g) in a mixture of 4N hydrochloric acid (10 mL) and ethanol (10 mL) was refluxed at 100 °C for 2 days. After this time the reaction was concentrated under vacuum, water (20 mL) was added to the residue and the formed solid was filtered and recrystallized by acetonitrile/ethanol to afford the pure MC3395 as a yellow solid. $^1$H-NMR (DMSO-$d_6$) $\delta_H$/ppm: 3.62 (s, 2H, PhC$H_2$-), 3.70 (s, 4H, -C$H_2$-piperidone), 6.78-6.80 (d, 2H, benzene protons), 7.11-7.73 (m, 9H, benzene protons and PhC*H*-), 7.73 (s, 2H, benzene protons); $^{13}$C-NMR (DMSO-$d_6$) $\delta_C$/ppm: 53.4 (2C), 64.4, 114.9 (2C), 119.1 (2C), 120.4 (2C), 126.9 (2C), 127.2, 128.4 (2C), 128.6 (2C), 130.2 (2C), 132.7, 140.7 (2C), 145.9 (2C), 155.7 (2C), 186.2; MS (EI): *m*/*z* $[M+H]^+$: 552.99.

Table 1: Chemical and physical data of compounds MC2884, MC2908, MC2909, MC2886, MC2910, MC2914, MC3269, MC3272, MC3146, MC3207, MC3187, MC2911, MC2912, MC2913, MC3395.

| Compd | Structure | Melting point (°C) | Recrystallization solvent | Yields (%) |
|---|---|---|---|---|
| MC2884 | 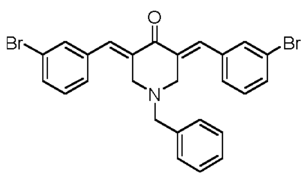 | 238-240 | Acetonitrile/ethanol | 78 |
| MC2908(1) | 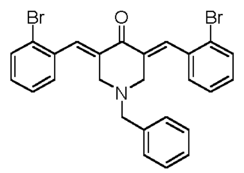 | 144-146 | benzene | 82 |

(continued)

| Compd | Structure | Melting point (°C) | Recrystallization solvent | Yields (%) |
|---|---|---|---|---|
| MC2909(2) | 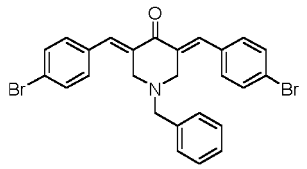 | 163-165 | Benzene/ acetonitrile | 84 |
| MC2886(3) | 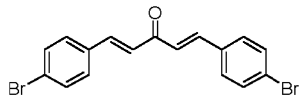 | 198-200 | Acetonitrile/ethanol | 86 |
| MC2910 | 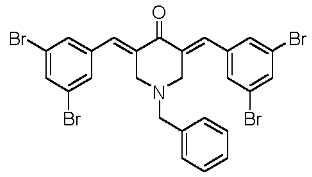 | 178-180 | Benzene/ acetonitrile | 73 |
| MC2914(1) | 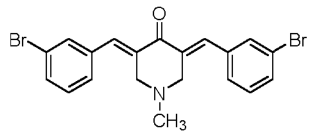 | 130-132 | Cyclohexane/ benzene | 74 |
| MC3269 | 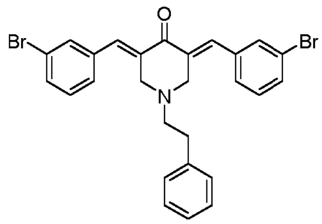 | 120-123 | Cyclohexane/ Benzene | 63 |
| MC3272 | 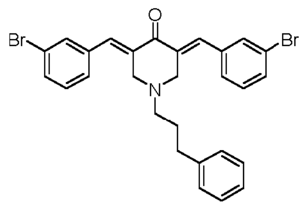 | 121-123 | Cyclohexane/ Benzene | 75 |
| MC3146 | 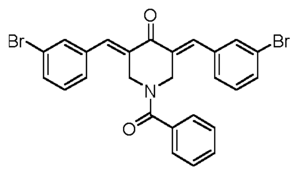 | 135-137 | Cyclohexane/ Benzene | 78 |
| MC3207 | 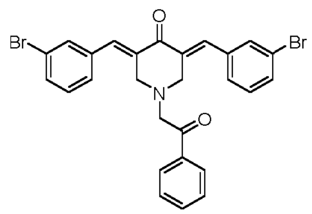 | 121-123 | Cyclohexane/ Benzene | 75 |

(continued)

| Compd | Structure | Melting point (°C) | Recrystallization solvent | Yields (%) |
|---|---|---|---|---|
| MC3187 | 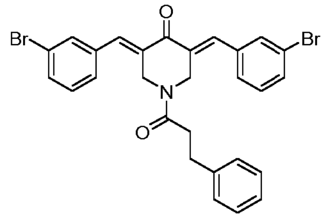 | 113-115 | Cyclohexane | 67 |
| MC2911[(4)] | 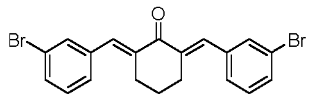 | 175-177 | Cyclohexane/ Benzene | 78 |
| MC2912 | 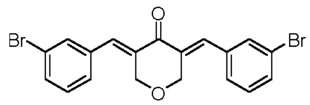 | 170-172 | Benzene/ acetonitrile | 83 |
| MC2913 | 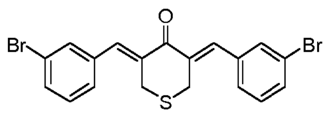 | 148-150 | Benzene | 80 |
| MC3395 | 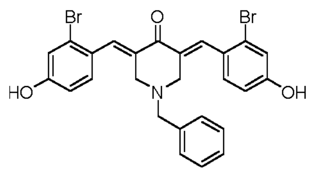 | 205-207 | Acetonitrile/ethanol | 69 |

**Surface Plasmon Resonance**

SPR analyses were performed on a Biacore 3000 optical biosensor equipped with research-grade CM5 sensor chips (Biacore AB). Recombinant p300/KAT3B (Enzo Life Sciences, cat. # BML-SE451; GenBank accession no. NM_001429) HAT domain was immobilized (30 μg/mL in 10 mM sodium acetate, pH 4.5) were immobilized at a flow rate of 10 μL/min by using standard amine-coupling protocols to obtain densities of 15 kRU. Myoglobin was used as negative control and one flow cell was left empty for background subtractions. All compounds, dissolved in DMSO (100%), were diluted in HBS (10 mM HEPES pH 7.4, 0.15 M NaCl) always maintaining a final 0.1% DMSO concentration. Binding experiments were performed at 25 °C, by using a flow rate of 30 μL/min, with 120 s monitoring of association and 300 s monitoring of dissociation. Regeneration of the surfaces was performed, when necessary, by a 10 s injection of 1 mM NaOH.

The simple 1:1 Langmuir binding fit model of the BIA evaluation software was used for determining equilibrium dissociation constants (KD) and kinetic dissociation (kd) and association (ka) constants by using equations 1 and 2:

$$dR/dt=k_a \times C \times (R_{max}-R)-k_d \times R \qquad \text{Equation 1}$$

where R represents the response unit, C is the concentration of the analyte, and

$$K_D=k_d/k_a \qquad \text{Equation 2}$$

As indicated in Figure 13, direct binding of MC2884 to p300 was confirmed.

**Stability of MC2884 in cell culture**

The compound was dissolved in DMEM at 50 μM and its concentration was measured by RP-HPLC using a ONYX 50x2 mm ID C18 column operating at 600 μL/min. The gradient applied was from 1% solvent B to 70% solvent B in 10 minutes. Solvent A was water with added 0.1% trifluoroacetic acid (TFA) and solvent B was acetonitrile with added 0.1% TFA. The eluate was monitored using a diode array detector with wavelengths set between 200-320 nm. The compound was eluted at 11.1 min. Peak integration was carried out on the chromatogram extracted at 326 nm, which is one of the compound absorbance maxima. A calibration curve was built by injecting solutions of the compound in DMSO at concentrations ranging between 1.5 μM and 200 μM. After incubation in DMEM compound concentration was determined at time points ranging between 0 and 72hrs. As shown, half of compound was degraded after 10h. At 72hrs it was fully degraded under these conditions.

**Cell proliferation and cell cycle analysis**

Colorimetric exclusion: cells ($2\times10^5$ cells/mL) were plated in multiwells and in triplicate. After stimulations at different times and concentrations (as indicated in the text), cells were diluted in the ratio 1:1 in Trypan Blue (Sigma) and counted with an optical microscope in order to discriminate dead cells (blue) from living cells, which do not stain.

For cell cycle analyses, the cells were plated ($2\times10^5$ cells/mL) and after stimulation (performed as indicated in the text) were harvested, centrifuged at 1200 rpm for 5 minutes and resuspended in 500 μL of a hypotonic solution containing IX PBS, Sodium Citrate 0.1%, 0.1% NP-40, RNAase A and 50 mg/mL Propidium Iodide (PI) (a dye that intercalates between the DNA bases, marking the DNA content in the cells and emitting a fluorescent signal that enables the analysis). After 30' at room temperature (RT) in the dark, samples were acquired by FACSCalibur (BD Bioscences, San Jose, CA, USA) using CellQuest software (BD Biosciences). The percentage in different phases of the cell cycle was determined by ModFit LT V3 software (Verity). All experiments were performed in triplicate.

**Primary samples**

Blasts cells were purified by the Ficoll-Hypaque gradient separation method (GE Healthcare). RNA was obtained by using TRIzol (Life Technologies) according to the manufacturer's recommendations. Isolation of genomic DNA has complied the FlexiGene DNA protocol (Qiagen).

ChIP extracts preparation has been carried out following IHEC procedures and as reported in (30-33).

**$Tet2^{-/-}$APL experiments**

MSCV backbone was used to express human PML-RARA fusion (Eradication of acute promyelocytic leukemia-initiating cells through PML-RARA degradation. Nasr R, Guillemin MC, Ferhi O, Soilihi H, Peres L, Berthier C, Rousselot P, Robledo-Sarmiento M, Lallemand-Breitenbach V, Gourmel B, Vitoux D, Pandolfi PP, Rochette-Egly C, Zhu J, de Thé H. Nat Med. 2008 Dec;14(12):1333-42. doi: 10.1038/nm.1891. Epub 2008 Nov 23) and used to transduce marrow progenitors from 5FU-treated 5-week-old mice wild type or inactivated for Tet2. After engraftment in irradiated recipient and malignant development, leukemic cells were grown *in vitro,* in standard M3434 methylcellulose conditions. For the experiments, cells were transferred in liquid culture and treated with MC2884. After 16 h of treatment, apoptotic cells were stained with Annexin V/APC and 7-AAD (Beckton Dickinson) and analyzed on a FACS CantoII (Beckton Dickinson). FACS data were analyzed by FlowJo Software (v8.8.7).

**Xenograft *in vivo* experiments**

**AML xenografts.** Xenograft studies using NB4 and U937-AML cells were performed in NOG SCID mice under specific pathogen—free conditions(57, 58). Cells (0.2 $10^6$ in 100 μL) were injected intravenously (retro orbital vein). Injected mice were randomly assigned to receive intra-peritoneal MC2884 (10 mg/kg) or vehicle for two weeks (8 doses). All animal procedures were performed in accordance with protocols approved by the local Committee for Animal Experimentation and with the permission of the Italian Health Ministry (n° 626/2015). At 16 days after injection, mice were sacrificed and the femurs and spleens were collected. Bone marrow (BM) and spleen cells were analyzed by FACS. To avoid non-specific stain, cells were mixed with mouse IgG and incubated with indicated antibodies for 30'. The mixture was depleted of erythrocytes and fixed (BD Pharmingen). NB4 and U937-AML cells were examined with FITC-anti-human CD45 antibody (eBioscence) and APC-conjugated anti-mouse CD45 antibody (Miltenyi). NB4-luc xenografts were performed by using a clone of NB4 luciferase transduced and by injecting $1*10^6$ cells (IP). Injected mice were randomly assigned to receive intra-peritoneal MC2884 (10 mg/kg) or vehicle for 4 weeks (every second day for the first 2 weeks; every third day for the rest). Tumor growth was monitored by weekly bioluminescence imaging (BLI) acquisitions

using an IVIS 3D Imaging System (Caliper Alameda, USA). To quantify bio luminescence, the integrated fluxes of photons (photons per s) within each area of interest were determined using the Living Images Software Package 3.2 (Caliper, Alameda, USA). Emission data were collected and normalized to bioluminescence of the injection day. Efficacy of drug treatment was assessed as inhibition of BLI emission comparing those MC2884 treated mice group and vehicle groups.

**Colon cancer xenografts.** HCT116 cell line was grown in McCoy's medium, supplemented with 10% inactivated FBS, 2 mM glutamine and standard concentration of antibiotics. 7- to 8-week-old male CD1 nude athymic mice were purchased from Charles River and under pathogen-free conditions in accordance with European Directives no. 2010/63 and with Italian D.L. 26/2014. Mice were injected subcutaneously into the right flank with $4X10^6$ of HCT116 cells; tumor volume ($mm^3$) was evaluated three times a week by caliper using the formula $D_{max}\ x\ d_{min}$^2/2, where 'd' and 'D' are the shortest and the longest diameters, respectively. For all experiments, mice were randomly divided (n = 7) and intraperitoneal injected by day five every day with: vehicle sesame oil/DMSO 6%; MC2884 10mg/Kg in sesame oil/DMSO 6%. Mice were sacrificed after 20 days of drug treatment and tumors explanted for immunohistochemical analyses. For HCT116-DKO colon cancer (59), $6X10^6$ cells were injected.

**Histology, Immunohistochemical analyses and TUNEL assay *in vivo* (xenograft)**

The biopsy specimens were fixed in 10% buffered-formalin and paraffin-embedded. Sections of 5 μM were stained with haematoxylin-eosin, and haematoxylin-van Gieson. For immunohistochemistry, specimens were incubated in a microwave oven for 15 min in 10 mM buffered citrate pH 6.0 followed by the immunohistochemical procedure for Ki67 (Santa Cruz Biotechnology Inc., CA, USA), H3K27me3, H3K9/14ac (Diagenode). The conventional avidin-biotin complex procedure was applied according to manufacturer's protocol (Dako Carpinteria, CA, USA) and incubated with secondary antibody. Positive staining was revealed by DAB chromogen, according to supplier's instructions followed by counterstaining with Mayer's hematoxylin. Slides were cover-slipped with a xylene-based mounting medium. Staining was scored as percentage of positive nuclei per high power field 10X40. Negative controls for each tissue section were performed omitting the primary antibody. Positive controls included in each experiment consisted of tissue previously shown to express the antigen of interest. TUNEL reaction was performed using the peroxidase-based Apoptag kit (Oncor, Gaithersburg, MD, US). TUNEL positive cells were detected with DAB and $H_2O_2$ according to the supplier's instructions. The experiments were repeated on different sections for each specimen (two to four). For all immunohistochemical markers, 100 random fields (250X) per section were analysed (12.5 $mm^2$). Mann-Whitney and Wilcoxon tests were used to assess the relationship between ordinal data. Two-tailed P value was considered significant when $\leq 0.05$. SPSS software (version 10.00, SPSS, Chicago, IL, USA) was used for statistical analysis.

**Total protein, histone extraction and western blot analyses**

The procedures were performed as described in (31,34). Primary antibodies used were: H3K27me3 (Abcam); H3K9-14ac (Millipore); H3K27ac, EZH2, PARP, TRAIL, RIP (Abcam); BID, BAX, BCL2 (Cell Signaling); BCL-XL (BD Biosciences); ERKs (Santa Cruz) and α-tubulin (Sigma) were used to normalize the total protein extracts, while H1 (Abcam) to normalize histone extracts.

**Transfections**

MCF7 cells were transfected with lipofectamine (Invitrogen) as previously described (33). The following plasmids were used: 1245 pCMVb p300 was a gift from William Sellers (Addgene plasmid # 10717), MSCV-FlagmuEzh2deltaset-Hygro was a gift from Tobias Neff (Addgene plasmid # 49403), pCMVβ-p300.DY-myc was a gift from Tso-Pang Yao (Addgene plasmid # 30490) and pCMV-VSV-G-Ezh2 wt was a gift from Kristian Helin (University of Copenhagen). After induction with MC2884, cells were diluted 1:1 in Trypan blue (Sigma) and counted.

**Enzymatic assays**

EZH2 assays were performed following BPS Biosciences instructions and as previously described (35).

Cell-based HAT assay. Protein extracts from the indicated cells were obtained in TAP buffer (Tris HC1 pH 7.0 50 mM, NaCl 180 mM, NP-40 0.15%, glycerol 10%, MgCl2 1.5 mM, NaMO4 1 mM, NaF 0.5 mM) with protease inhibitor cocktail (Sigma), DTT 1 mM and PMSF 0.2 mM). Transfection with pCMX-Flag CBP and pP300 were performed in HEK293-FT cells and then proteins extracted. 1000 μg were diluted in TAP buffer and IPed with anti-p300 (Millipore, 2 μg) and anti-CBP (Santa Cruz, 2 μg) following standard procedures. As a negative control, purified IgG rabbit (Santa Cruz, 2 μg) and IgG mouse (Santa Cruz, 2 μg) were also used. All samples were washed in HAT Assay Buffer IX (DTT 1 mM in PBS IX) and the HAT radioactive assay was carried out. According to supplier's instructions (Millipore), 10 μL of HAT Assay Buffer, 3 μL (2 μg) of Core Histones and 5 μL of the diluted [$^3$H]-Acetyl-CoA were added to the beads.

MC2884 was tested at the final concentration of 3 μM. All the components were incubated for 60 minutes at 37 °C in gently rock/shake. Samples were spotted on P81 paper, washed three times with 10% trichloroacetic acid and once with acetone, transferred to a scintillation vial containing 5 mL scintillation cocktail and read in a scintillation counter. Anacardic Acid (AA, ENZO LIFE) was used directly on incubation mix, as positive control. HAT in vitro assay. MC2884 activity was also tested *in vitro* and IC50 was calculated as indicated in the scheme. MC2884 was tested in a 10-dose IC50 mode with 2-fold serial dilution, in singlet, starting at 200 μM. Anacardic Acid (A7236, SIGMA) or C646 (SML0002, SIGMA), were tested in 10-dose IC50 mode with 3-fold serial dilution starting at 100 μM. Reactions were carried out at 3 μM Acetyl-CoA. Empty cells indicate no inhibition or compound activity that could not be fit to an IC50 curve.

**Table 2.** IC50 of MC2884, C646 and Anacardic acid on the indicated enzymatic activities.

| **Acetyltransferase:** Substrate: | Compound IC50(M) | | | | |
|---|---|---|---|---|---|
| | CBP | GCN5 | KAT5 | p300 | pCAF |
| | Histone H3 | Histone H3 | Histone H2A | Histone H3 | Histone H3 |
| MC2884 | **3,27E-06** | | 8,35E-07 | 4,56E-07 | |
| C646 | **7,87E-08** | | **2,04E-05** | 1,54E-07 | |
| **Anacardic Acid** | | 3,86E-05 | | | 3,88E-05 |

The HDAC1 and SIRT1 assays were performed as described previously (60,61).

**Proliferation and migration analysis in real time**

Proliferation and migration analysis were performed by xCELLigence technology (Roche) following standard procedures and as reported in (38).

**Caspase assays**

Caspase activity was detected within living cells using B-BRIDGE Kits with cell-permeable fluorescent substrates following manufacturer's instructions. The fluorescent substrates for caspase-3, 7, -8, and -9 were FAM-DEVD-FMK, FAM-LETD-FMK, and FAM-LEHDFMK, respectively. Cells were washed twice in cold PBS and incubated for 1 h in ice with the corresponding substrates, as recommended by suppliers. Cells were analysed using Cell Quest software applied to a FACScalibur (BD Biosciences). Experiments were performed in triplicate and values expressed in mean +/- SD.

**Reagents**

Z-VAD (R&D), Caspase-8 Inhibitor Z-IETD-FMK (R&D) and Caspase-9 Inhibitor Z-LEHDFMK (R&D) were used at 100 μM respectively. N-Acetyl cysteine (NAC) (Sigma) was used at 20 μM. ABT-737 (BCL2i) (Selleckchem) was used at 1 μM; GSK126 (EZH2i) (Selleckchem) was used at 10 μM; C646 (HATi) (Sigma) was used at 5 μM.

**Next Generation Sequencing** (NGS) **experiments**

Samples for NGS experiments were obtained from different laboratories but have been processed similarly, using standard operating procedures set by BLUEPRINT at the start of the project using extensively validated and documented antibody batches and other reagents. Moreover, all samples underwent strict quality control at different levels (e.g. sequence quality, read depth, visual inspection) before they were included in the downstream analysis pipeline. The exact procedures and quality filters can be found at the BLUEPRINT website (www.blueprint-epigenome.eu)

**Chromatin immunoprecipitation (ChIP)**

ChIP extracts preparation and procedures have been carried out following IHEC procedures using Diagenode antibodies and as reported (62-64). The fold enrichment of H3K27ac and H3K27me3 ChIP'ed DNA was evaluated. Primer sequences were as follows: BCL2 promoter region (at -410 and -282 from +1), forward (5'- GTG TTC CGC GTG ATT GAA GAC-3', SEQ ID No. 1) and reverse (5'- CAG AGA AAG AAG AGG AGT TAT AA-3', SEQ ID No. 2); for chr21:44496353-44496918/CBS region, forward (5'- CGC AGA ACA GTC GCC TTG-3', SEQ ID No. 3) and reverse (5'-GTC CAG AGC ACG ATG TTT GG-3', SEQ ID No. 4); for chr17:4938577-4939058/SLC52A1 region, forward (5'- CGA

GTT GGA GAG GGG AGT G-3', SEQ ID No. 5) and reverse (5'- AAC AAA ACC CCA GCT GTG TG-3', SEQ ID No. 6).

**RNA extraction and RT-PCR**

Total RNA was extracted with Trizol (Invitrogen) and converted into cDNA using VILO (Invitrogen). For amplification the following primers were used: EZH2, forward (5'-CATCATAGCTCCAGCTCCCG-3', SEQ ID No. 7) and reverse (5'-CATCCCGGAAAGCGGTTTTG-3', SEQ ID No. 8); EED, forward (5'-CGATTTGCGACAGTGGG-3', SEQ ID No. 9) and reverse (5'-CAGGTGCATTTGGCGTG-3', SEQ ID No. 10); SUZ12, forward (5'-GTCCTGCTTGTGAAAGTTTGC-3', SEQ ID No. 11) and reverse (5'-CAAATGTCTTTTCCCCATCCT-3' SEQ ID No. 12); BCL2, forward (5'-GAACTGGGGGAGGATTGTGG-3', SEQ ID No. 13) and reverse (5'-CAGCCTCCGTTATCCTGGAT-3', SEQ ID No. 14); GAPDH, forward (5'-TCAACGGGAAGCCCATCACCA-3', SEQ ID No. 15) and reverse (5'-ACGGAAGGCCATGCCAGTGA-3', SEQ ID No. 16).

**RNA-sequencing**

RNA sequencing (RNA-seq) preparation and library creation was performed at the Max Planck Institute for Molecular Genetics (MPIMG). Following purification, cells were lysed in TRIZOL reagent (Life Technologies) at a concentration of approximately 2.5 million cells/ml. RNA was extracted as per manufacturer's instructions, resuspended in ultra-pure water and quantified (Qubit, Invitrogen) prior to library preparation. Sequencing libraries were prepared from 200 ng RNA using an Illumina TruSeq Stranded Total RNA Kit with Ribo-Zero Gold (Illumina). Adapter-ligated libraries were amplified and indexed via PCR. Up to six libraries were multiplexed per lane and sequenced at MPIMG using protocols following manufacturer's instructions (V3 chemistry, HiSeq 2000, Illumina). Furthermore, detailed protocols can be found on the Blueprint website (http://www.blueprint-epigenome.eu/index.cfm?p=7BF8A4B6-F4FE-861A2AD57A08D63D0B58)

**Whole-genome bisulfite sequencing**

Genomic DNA (1-2μg) was spiked with unmethylated λ DNA (5ng of λ DNA per μg of genomic DNA) (Promega).

The DNA was sheared by sonication to 50-500 bp using a Covaris E220 and fragments of size 150-300 bp were selected using AMPure XP beads (Agencourt Bioscience Corp.). Genomic DNA libraries were constructed using the Illumina TruSeq Sample Preparation kit (Illumina Inc.) following the Illumina standard protocol: end repair was performed on the DNA fragments, an adenine was added to the 3' extremities of the fragments and Illumina TruSeq adapters were ligated at each extremity. After adaptor ligation, the DNA was treated with sodium bisulfite using the EpiTexy Bisulfite kit (Qiagen) following the manufacturer's instructions for formalin-fixed and paraffin-embedded (FFPE) tissue samples. Two rounds of bisulfite conversion were performed to assure a high conversion rate. An enrichment for adaptor-ligated DNA was carried out through 7 PCR cycles using the PfuTurboCx Hotstart DNA polymerase (Stratagene). Library quality was monitored using the Agilent 2100 BioAnalyzer (Agilent), and the concentration of viable sequencing fragments (molecules carrying adaptors at both extremities) estimated using quantitative PCR with the library quantification kit from KAPA Biosystem. Paired-end DNA sequencing (2×100 nucleotides) was then performed using the Illumina Hi-Seq 2000.

**ChIP-sequencing**

Purified cells were fixed with 1% formaldehyde (Sigma) at a concentration of approximately 10 million cells/mL. Fixed cell preparations were washed and either stored re-suspended in PBS at 4 °C before shipping to the processing institutes (NCMLS). Sonication was performed using a Diagenode Bioruptor UCD-300 for 3x 10 minutes (30 seconds on; 30 seconds off). Chromatin (67 μL, 1 million cells) was incubated with 229 μL dilution buffer, 3 μL protease inhibitor cocktail and 0.5-1 μg of H3K27ac, H3K4me3, H3K4me1, H3K27me3, H3K9me3 or H3K36me3 antibodies (Diagenode) and incubated overnight at 4 °C with rotation. Protein A/G magnetic beads were washed in dilution buffer with 0.15% SDS and 0.1% BSA, added to the chromatin/antibody mix and rotated for 60 minutes at 4°C. Beads were washed with 400 μL buffer for 5 minutes at 4 °C with five rounds of washes. After washing chromatin was eluted using elution buffer for 20 minutes. Supernatant was collected, 8 μL 5M NaCl, 3 μL proteinase K were added and samples were incubated for 4 hours at 65 °C. Finally samples were purified using a Qiagen Qiaquick MinElute PCR purification Kit and eluted in 20 μL EB. Illumina library preparation was done using the Kapa Hyper Prep Kit. For end repair and A-tailing double stranded DNA was incubated with end repair and A-tailing buffer and enzyme and incubated first for 30 minutes at 20 °C and then for 30 minutes at 65 °C. Subsequently, adapters were ligated by adding 30 μL ligation buffer, 10 Kapa 1 DNA ligase, 5 μL diluted adaptor in a total volume of 110 μL and incubated for 15 minutes at 15°C. Post-ligation cleanup was performed using Agencourt AMPure XP reagent and products were eluted in 20 μL elution buffer. Libraries were

amplified by adding 25 μL 2x KAPA HiFi Hotstart ReadyMix and 5 μL 10x Library Amplification Primer Mix and PCR, 10 cycles. Samples were purified using the QIAquick MinElute PCR purification kit and 300bp fragments selected using E-gel. Correct size selection was confirmed by BioAnalyzer analysis. Sequencing was performed using Illumina HiSeq 2000 machines and generated 43bp single end reads.

Detailed protocols can be found on the Blueprint website (http://www.blueprint-epigenome.eu/index.cfm?p=7BF8A4B6-F4FE-861A-2AD57A08D63D0B58)

**Peak Calling and identification of promoter and enhancer regions**

For peak calling the BAM files were first filtered to remove the reads with mapping quality less than 15, followed by fragment size modeling (http://code.google.com/p/phantompeakqual-tools/). The peak-calling algorithm MACS2 (http://github.com/taoliu/MACS/) was used to detect the binding sites for the three studied histone marks at default q-value (5.00e-02). H3K4me1 peaks were called using the broad setting of MACS2 while peaks for marks H3K27ac, H3K4me3 and transcription factor PML-RARA were called using the default (narrow) setting. H3K27ac tracks in mouse APL cells and human HCT116 cells were analyzed similarly.

In order to identify promoter and enhancer regions, non-overlapping H3K4me3 and H3K4me1 bound regions were taken into account, respectively. The common promoter and enhancer regions across the APL-R samples were computed using the in-house script intersectbed.pl. Further, normalized tag enrichment for H3K27ac and H3K27me3 was used to mark active and inactive promoter and enhancer regions. Also, normalized tag enrichment for H3K27ac and H3K9K14ac in common enhancer regions was used to compute differentially active regions across different samples (pt#7, pt#8 and pt#9) and cell types (CD34+, promyelocytes, metamyelocytes, band neutrophiles and segmented neutrophiles). The intensity graphs were generated using the in house script makeColorprofiles.pl. Discriminating hyperacetylated regions were identified using DESEQ (http://bioconductor.org/packages/release/bioc/html/DESeq.html) and selection for regions with an FDR < 0.01 and p-value < 0.0002 between APL-Rs/high risk APLs and all other samples (APL and AML). Motif analysis was performed as before (16).

**RNA-seq alignment and expression analysis**

RNA-seq reads were aligned using GSNAP (65) using non-default parameters -m 1 - N 1 -n 1 - Q -s Ensembl_splice_68. RNA-seq library data was initially subjected to a quality control step, where, based on read distribution over the annotated genome, libraries that are outliers were identified and discarded from further analysis. For expression analyses reads were aligned to the Ensembl v70 (GRCh37.70) human transcriptome using Bowtie. Quantification of gene expression was performed using MMSEQ (66).

**SNP Calls and identification of potential functional SNPs**

SNP calls were made using the CNAG in house pipeline using WGBS data. The list of variant calls was reduced by applying a number of filters, starting from number of tags (≥20) and Phred score (≥40) on the SNP calls from WGBS. The reduced list was then given as an input to the CHASM web server (http://www.cravat.us/ version 3.1) for functional annotation and only the missense variants present in driver genes (as identified by Vogelstein) (67) at MAF ≤5% (1000G) were selected. Further, only the variants reported in ChIP-seq & RNA-seq tracks by applying computational tools mpileup and GATK along with the variations confirmed through visual inspection of the tracks using IGV were selected.

**Identification of signature methylation regions**

Cytosine methylation level was called for every cytosine site in different sequence contexts such as CHH, CXG and CG (H denotes A, T, or C and X denotes A or T). Due to the fact that symmetric methylation level is the common case for CG methylation, the inventors focused on CG methylation and excluded non-CpG methylation for further analysis. Within a symmetric CG context, CG pairs on both forward and reverse strands were combined and a CpG coverage threshold of >=5 was used. Further, the methylation samples #7 and #8 were grouped together with the 3 APL samples of low OS from TCGA(1) and compared with the normal APL samples. The inventors selected the regions for which CG methylation was determined across all samples and applied the Wilcoxon test to determine statistically significant different CpGs between the two groups. A cut-off of p-value of 0.005 was applied as the selection criteria and regions were plotted. To identify the most discriminating CpGs the inventors applied the same test at a more stringent cut-off p-value of 0.0007. Moreover, only the CpG regions that had beta values in all 181 AML samples were selected.

## Pathway enrichment analysis

The Gene Set Enrichment Analysis (GSEA, http://software.broadinstitute.org/gsea/msigdb/annotate.jsp) was applied to determine the enriched pathways at FDR 0.05.

## Patient characteristics

Patients' characteristics are reported in Table 3.

**Table 3.** Patient characteristics

| Number | Blueprint ID | Leukemia | karyotype |
|---|---|---|---|
| pt#1 | n/a | AML | NK |
| pt#2 | n/a | AML | NK |
| pt#3 | n/a | AML | del(2) |
| pt#4 | n/a | AML | +8 |
| pt#5 | n/a | AML | +8 |
| pt#6 | n/a | AML | inv3 |
| pt#7 | 284 | APL | t(15;17) |
| pt#8 | 289 | APL | t(15;17) |
| pt#9 | 302 | APL | t(15;17) |
| pt#10 | n/a | ALL | |
| pt#11 | UMCG00003 | AML | NK |
| pt#12 | UMCG00012 | AML | NK |
| pt#13 | UMCG00001 | AML | 49 XY, +13, +14, +21 |
| pt#14 | UMCG00017 | AML | NK |
| pt#15 | UMCG00005 | AML | NK |
| pt#16 | UMCG00007 | AML | NK |
| pt#17 | UMCG00008 | AML | NK |

| | | | |
|---|---|---|---|
| pt#18 | UMCG00004 | AML | NK |
| pt#19 | UMCG00011 | AML | +8 |
| pt#20 | UMCG00018 | AML | NK |
| pt#21 | UMCG00006 | AML | NK |
| pt#22 | UMCG00002 | AML | NK |
| pt#23 | UMCG00025 | AML | NK |
| pt#24 | UMCG00024 | AML | +11 |
| pt#25 | UMCG00009 | AML | t(8;21) |
| pt#26 | UMCG00014 | AML | t(3;5) |
| pt#27 | UMCG00021 | AML | t(8;21) |
| pt#28 | UMCG00027 | AML | NK |
| pt#29 | UMCG00015 | AML | inv16 |
| pt#30 | UMCG00029 | AML | NK |
| pt#31 | UMCG00019 | AML | +8 |
| pt#32 | UMCG00013 | AML | NK |
| pt#33 | UMCG00016 | AML | NK |
| pt#34 | UMCG00028 | AML | aberant chr3 and chr 5 |
| pt#35 | UMCG00023 | AML | complex |
| pt#36 | UMCG00026 | AML | t(3;5) |
| pt#37 | UMCG00010 | AML | inv16 |
| pt#38 | n/a | APL | t(15;17) |
| pt#39 | n/a | APL | t(15;17) |

## Results

### A hyperacetylation signature typifies treatment-resistant APLs with low overall survival

To investigate the molecular determinants of aggressiveness and resistance (39), the inventors analyzed two APL blast samples resistant to standard ATRA plus chemo treatment (APL-R). The inventors confirmed expression of the PML-RARA fusion (Fig. 1A) and analyzed the mutational landscape of the two APL patients (Fig. 1B and Material), but this did not reveal a common second genetic mutation (Fig. 1C, Table 4).

**Table 4.** Karyotype and molecular characteristics of APL-Rs. In the columns from left to right: sample number, sample code, sample leukemia type, translocation or aberrations.

| | | | |
|---|---|---|---|
| #7 | APL | t(15;17) | BCR2+, FLT3 ITD+ |
| #8 | APL | t(15;17)(q24;q21)(10) | BCR1+ |
| #A | APL | t(15;17) | |
| #B | APL | t(15;17) | FLT3 ITD+ |

PML-RARA recruits HDACs and establishes a hypoacetylated chromatin signature, which can be alleviated by ATRA induced PML-RARA degradation (30). To analyze whether PML-RARA binding sites in the APL-R had retained this ATRA response, the inventors performed ChIP-seq for PML-RARA as well as for several epigenetic marks (39) (Fig. 2A). Treatment-sensitive APL (APL) and the APL-R PML-RARA overlapping sites (Fig. 1D) displayed ATRA treatment-dependent H3K9/14ac increases (Fig. 2B), indicating that the APL-Rs still respond to ATRA. Interestingly however, the inventors also observed elevated steady-state levels of histone acetylation at PML-RARA binding sites in APL-Rs as compared to APL (Fig. 2B). To analyze whether increased acetylation (H3K9/14ac and H3K27ac) was a genome-wide feature, the inventors identified all APL-R promoter and enhancer regions (Fig. 1E) and compared APL-R H3ac occupancy with levels in treatment-sensitive APL as well as in $CD34^+$ progenitor cells, promyelocytes, metamyelocytes, band neutrophils and segmented neutrophils from healthy individuals. Inventors' analysis revealed that promoter and enhancer regions identified in APL-Rs largely overlap with those found in the other cell types (data not shown), but that they are generally enriched for H3ac as compared to levels in normal cells and treatment-sensitive APLs (Fig. 2C; Fig. IF). In line with the general correlation between H3ac levels and gene expression the inventors observed that associated genes are higher expressed in APL-Rs as compared to APL (Fig. 1G). As the hyperacetylation signature in APL-Rs was most apparent for the enhancers (Fig. 2C) the inventors used these regions for motif analysis, revealing enrichment for RUNX1 and ETS consensus motifs, suggesting factors belonging to these families might assist in epigenetic markings of these regions.

Next, the inventors asked whether the acetylation state at identified enhancers (Fig. 2C) is specific for APL-Rs. The inventors compared the APL-R H3K27ac pattern with levels of 27 AMLs of varying prognosis and cytogenetic background (39). In addition, the inventors included 2 high risk APLs (pz#A and B; number of leukocytes > 1 million/ml). Indeed, acetylation levels at the set of high risk/APL-R enhancers is increased as compared to acetylation at these regions in any other subtype of AML ($p<0.01$ for each pairwise comparison) (Fig. 2D), suggesting that the acetylation signature might be used to identify this particular subclass of APLs. To allow clinical discrimination of this subset using assays that are NGS independent, the inventors defined the most differential acetylated regions. Using an FDR < 0.01 and p-value < 0.0002, the inventors identified 22 regions specifically acetylated in high risk/APL-Rs that can be used for stratification (Table 5 and Fig. 3A).

**Table 5.** Hyperacetylated regions (hg19) in APL-Rs.

| chromosome | start | end |
|---|---|---|
| chr1 | 95181929 | 95184074 |
| chr1 | 109626120 | 109627905 |
| chr1 | 249221773 | 249222010 |
| chr11 | 57158202 | 57159391 |
| chr14 | 61416231 | 61417467 |
| chr14 | 105391316 | 105391725 |
| chr17 | 70982083 | 70984392 |
| chr17 | 71061705 | 71070236 |
| chr2 | 27060445 | 27061863 |
| chr2 | 102056668 | 102060845 |
| chr2 | 234072888 | 234075888 |
| chr20 | 2665743 | 2666563 |
| chr22 | 50153595 | 50155770 |
| chr3 | 41011746 | 41014498 |
| chr6 | 10087785 | 10088550 |
| chr6 | 82468660 | 82469114 |
| chr6 | 139948334 | 139949640 |
| chr6 | 140000376 | 140002294 |
| chr7 | 29323864 | 29325989 |
| chr9 | 94907894 | 94910771 |
| chrX | 37605417 | 37607058 |
| chrX | 151152531 | 151154618 |

Altogether these results reveal that the histone acetylation landscape of APL-Rs is different as compared to APLs and myeloid progenitors and generally represents a hyperacetylated histone signature.

Using an FDR < $10^{-15}$ and >5-fold change (as compared to all other APLs/AMLs) the inventors identified 29 regions specifically acetylated in low OS APLs that can be used as target regions (Table 6).

**Table 6.** Hyperacetylated regions (hg19) in low OS APLs

| chromosome | start | end |
|---|---|---|
| chr2 | 102761838 | 102765245 |
| chr2 | 138558210 | 138560637 |
| chr3 | 138917149 | 138918464 |
| chr4 | 47722119 | 47725095 |
| chr5 | 177820647 | 177821809 |
| chr5 | 177911126 | 177912881 |
| chr6 | 10080702 | 10085536 |
| chr6 | 100796638 | 100797329 |
| chr7 | 43539997 | 43544821 |
| chr8 | 41107099 | 41109973 |
| chr8 | 41271751 | 41275593 |
| chr8 | 109131198 | 109133248 |
| chr10 | 31145487 | 31151138 |
| chr10 | 31152266 | 31155251 |
| chr10 | 82011240 | 82013721 |
| chr12 | 1741915 | 1746279 |
| chr13 | 110036141 | 110039705 |
| chr13 | 110048447 | 110050505 |
| chr14 | 20955081 | 20960400 |
| chr15 | 97270509 | 97273147 |
| chr17 | 426695 | 431121 |
| chr18 | 61534509 | 61537791 |
| chr18 | 74255741 | 74257346 |
| chr19 | 15742473 | 15746990 |
| chr20 | 48404657 | 48406416 |
| chr21 | 32507312 | 32510923 |
| chr22 | 19230646 | 19232961 |
| chrX | 2850454 | 2853230 |
| chrX | 147574563 | 147578311 |

Together these results reveal the histone landscape, in particular the H3ac of low OS APLs, is different as compared to responding (and high OS) APLs and other progenitors of the myeloid compartment and that this chromatin signature can be used to specifically identify this subset of APLs.

**Deregulation in repressive histone methylation impacts on APLs with low overall survival**

APL leukemogenesis has also been linked to H3K27me3 alterations (30,40), suggesting an important role for the PRC2 complex and H3K27me3 occupied genomic regions in APL. The inventors hypothesized that in APL-Rs, the hyperacetylated signature might impact the presence of repressive marks such as H3K27me3. Examining H3K27me3 patterns in APL-R and APLs revealed differential occupancy at a number of loci, for example at the ZSCAN12/GPX6 genomic region, indicating that besides H3 acetylation also other epigenetic features such as H3K27me3 are altered in APL-Rs (Fig. 2E).

**DNA methylation profile can identify low OS APLs**

Next, the inventors assessed whether differential CpG methylation could be used to distinguish APL-Rs from APLs. The inventors included DNA methylation data of a cohort of 15 APL patients (450K, from TCGA (1)), of which 3 had a survival below 1 month, indicating low treatment response (Fig. 3B).

Supervised clustering identified a set of 1817 CpGs that could stratify APLs in a good or resistant prognosis category (Fig. 3C). These included 399 hypomethylated methylated CpG regions (CpG coverage >4, p-value <0.005), mostly putative promoter regions enriched in H3K4me3 and H3K27ac, and 1418 hypermethylated CpG regions (Fig. 3D). To validate this observation, the inventors included all TCGA AML data (1). This revealed that the unique hyper/hypo methylation signature discovered in APL-R is specific (Fig. 4, top). Importantly, this signature could be further filtered to 23 CpGs (CpG coverage >4, p-value <0.0007) (Table 7; Fig. 4, bottom), which distinguish APL-Rs from all APLs/AMLs, potentially enabling PCR-based approaches to identify the APL-R subtype at diagnosis.

**Table 7.** Hypomethylated CpGs (hg19) in APL-Rs.

| chromosome | start | end |
|---|---|---|
| chr1 | 1052949 | 1052950 |
| chr1 | 12655992 | 12655993 |
| chr1 | 17287501 | 17287502 |
| chr1 | 55266578 | 55266579 |
| chr2 | 237477262 | 237477263 |
| chr3 | 35706114 | 35706115 |
| chr3 | 57198244 | 57198245 |
| chr4 | 3516534 | 3516535 |
| chr4 | 163085348 | 163085349 |
| chr6 | 73972852 | 73972853 |
| chr12 | 57630107 | 57630108 |
| chr12 | 114841708 | 114841709 |
| chr14 | 24779959 | 24779960 |
| chr16 | 1429015 | 1429016 |
| chr16 | 20817501 | 20817502 |
| chr16 | 46919112 | 46919113 |
| chr16 | 67197640 | 67197641 |
| chr17 | 39845949 | 39845950 |
| chr19 | 3275394 | 3275395 |
| chr19 | 35531990 | 35531991 |
| chr19 | 39056084 | 39056085 |
| chr20 | 21687378 | 21687379 |
| chr20 | 62084626 | 62084627 |

**MC2884 impairs methylation and acetylation levels**

Since APL-Rs display multiple epigenome deregulations, the inventors wondered whether a multi epi-drug approach targeting HATs and PRC2 would be beneficial. The inventors designed and identified MC2884 (Fig. 5A) as a promising candidate with a high stability in cell culture media (half-life of 10 hours and a time for total degradation of 72 hours) (Fig. 5B). Structural modeling suggested that MC2884 acts as a modulator of EZH2 activity (35). When compared with EZH2-targeting drugs, such as GSK126, MC2884 displayed higher anticancer potential in APL cells (Fig. 6A). Excitingly, the anticancer potential of MC2884 was partially recapitulated combining p300 (C646, a lysine-acetylation inhibitor) and EZH2 (GSK126, a lysine-methylation inhibitor) inhibition resulting in the modulation of H3K9/14ac and H3K27me3 (Fig 6A, bottom). *In vitro* assays showed that MC2884 is a genuine dose-dependent inhibitor of the EZH2 enzyme (Fig. 6B; see also SAR study below). In NB4 cells, MC2884 caused loss of H3K27me3 in a time and dose dependent fashion (Fig. 6C). Importantly, EZH2 showed a dose-dependent decreased expression, suggesting that MC2884 also acts on EZH2 expression, potentially destabilizing the enzyme and/or the PRC2 complex components (Fig. 6A, 6C, 5C, SAR results below). In addition, both H3K9/14ac and H3K27ac signals displayed a drastic dose-dependent reduction, demonstrating a strong inhibitory effect on H3 acetylation levels (Fig. 6C, Fig. 5D). HAT assays in NB4 and HCT116 (Fig. 6D), showed decreased HAT activity in living cells after MC2884 treatment and reduced HAT activity of immunoprecipitated p300 and CBP (Fig. 6E, see also SAR studies). MC2884 did not display inhibitory or activating actions on HDACs or sirtuins (Supplementary Fig. 5E). Extensive SAR studies confirmed the correlation between the effects on EZH2, HATs and the anticancer potential suggesting that MC2884 modulates the epigenome through direct inhibition of EZH2 and HAT (see below SAR studies).

**SAR studies**

When tested in leukemia NB4 cells at 3 μM for 30 h, MC2884 induced 52.1% cell death. Some MC2884 analogues have been tested to obtain SAR data (Table 8).

**Table 8.** Percentage of cell death induction by MC2884 and its analogues in NB4 and SHSY5Y cells.

| Cmpd | Structure | % Cell death | |
|---|---|---|---|
| | | NB4 cells, 3 μM, 30 h | SHSY5Y cells, 3 μM, 48 h |
| Vehicle | | 2.5 | 1.3 |
| MC2884 | 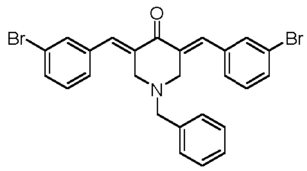 | 52.1 | 19.1 |
| MC2908 | 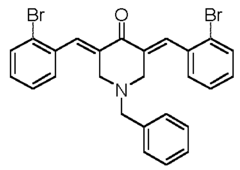 | 30.3 | 9.2 |
| MC2909 | 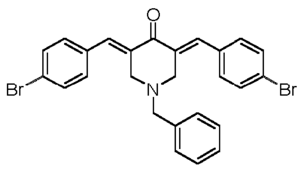 | 8.8 | 4.7 |
| MC2886 | 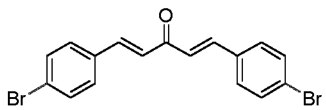 | 0,56 | 1,2 |
| MC2910 | 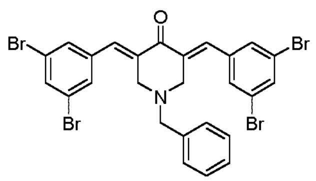 | 4.2 | 2.4 |
| MC2914 | 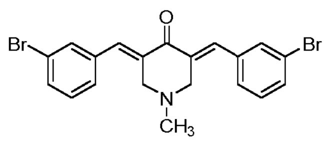 | 36.0 | 9.0 |

(continued)

| Cmpd | Structure | % Cell death | |
|---|---|---|---|
| | | NB4 cells, 3 μM, 30 h | SHSY5Y cells, 3 μM, 48 h |
| MC3269 | 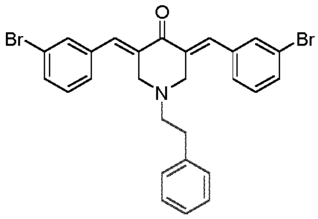 | 62.5 | 19.2 |
| MC3272 | 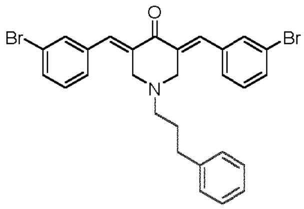 | 29.1 | 7.0 |
| MC3146 | 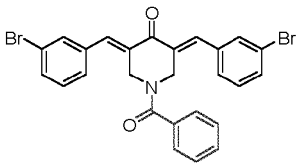 | 13.2 | 5.6 |
| MC3207 | 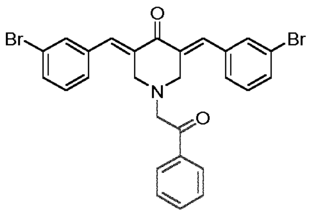 | 38.0 | 7.0 |
| MC3187 | 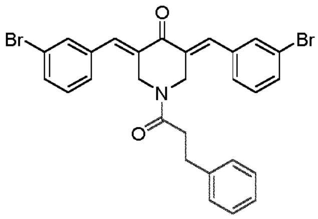 | 28.9 | 19.9 |
| MC2911 | 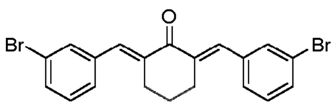 | 0,76 | 0,8 |
| MC2912 | 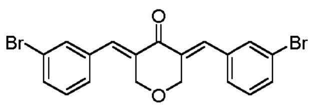 | 4.1 | 1.0 |
| MC2913 | 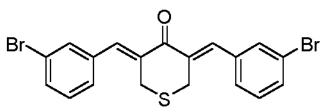 | 52.7 | 23.9 |

(continued)

| Cmpd | Structure | % Cell death | |
|---|---|---|---|
| | | NB4 cells, 3 μM, 30 h | SHSY5Y cells, 3 μM, 48 h |
| MC3395 | 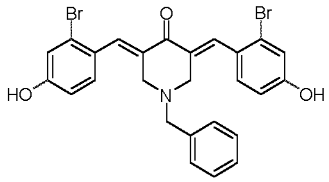 | 0,53 | 1,0 |

In particular, the shift of bromine atoms from *meta* to *ortho* (MC2908) and mainly to *para* (MC2909) position at the benzene rings reduced cell death to 30.3% and to 8.8%, respectively. Also the introduction of further bromine atoms at the *meta* positions of the two phenyl rings (MC2910) abated cell death induction (4.2%) of the derivative. At the N1 position, replacement of the benzyl with a methyl group (MC2914) led to a decrease of cell death induction (36%), whereas introduction of the longer phenylethyl moiety (MC3269) gave an increase of the effect (62.5% cell death). Further stretching of the N1 substituent to the phenylpropyl one (MC3272) lowered cell death induction (29.2%). Changing the N1-benzyl group of MC2884 with the N1-benzoyl group (MC3146) produced a severe loss of activity (13.2% cell death), but it was partially restored by introducing at N1 a 1-phenyl-2-ethan-1-one (MC3207, 38% cell death) or 3-phenyl-1-propan-1-one (MC3187, 28.9% cell death) unit, thus giving more flexibility to the N1 substituent. Replacement of the N1-benzyl-4-piperidone moiety with the tetrahydro-4*H*-pyran-4-one (MC2912) totally abated the cellular effect of the derivative (4.1% cell death), whereas the use of the isosteric tetrahydro-4H-thiopyran-4-one (MC2913) strongly restored the cell death, MC2913 showing the same potency of MC2884 (52.7% cell death).

Western blot analyses (and their quantification by ImageJ) performed on NB4 cells to detect the levels of acetyl-H3K9-14 (as a marker of HAT/anti-HAT activity) and H3K27me3/2 (as marker of PRC2/EZH2 activity) showed a decrease of histone acetylation and methylation after treatment with 3 μM compounds for 48 h, thus confirming the role of dual HAT/EZH2 inhibitors for these derivatives. In addition, the tested compounds also lowered the level of the EZH2 protein (Fig. 14).

When tested in neuroblastoma SHSY5Y cells at 3 μM for 48 h, MC2884 and its analogues furnished similar trend of death induction as in NB4 cells (Table 7). In general, the SHSY5Y cell line is less sensitive to the tested molecules than NB4 cells. Anyway, also in this cell line the bis 2-bromophenyl (MC2908), bis 4-bromophenyl (MC2909), and bis 3,5-dibromophenyl (MC2910) were less potent or not active as cell death inducers. As in NB4 cells, substitution at N1 with methyl (MC2914) or 3-phenylpropyl (MC3272) groups reduced the potency of the corresponding derivatives whereas introduction of a phenethyl group (MC3269) gave a compound with the same potency as MC2884. The presence of a carbonyl function within the N1 substituent abated the activity of the compounds, with the exception of that showing a 3-phenyl-1-propan-1-one chain at N1 (MC3187), which in this case was slightly more potent than MC2884. The tetrahydro-4*H*-pyran-4-one MC2912 failed in inducing cell death in SHSY5Y cells, whereas its thio-analogue MC2913 displayed the best death induction activity, similarly to what observed in NB4 cells.

Interestingly, compounds inactive in inducing cell death in both cell lines such as MC2910, 2912, 2909, 2886, 2911, 3395 were also unable to modulate histone acetylation and EZH2 deregulation (Fig. 15). In full agreement, only MC2884 was able to down-regulate BCL2 expression levels at both RNA (histogram bars) and protein levels (western blot) suggesting that this action is crucial for the induction of apoptosis.

Finally, to causally connect the anticancer action with the dual p300 and EZH2 targeting of the drug, the inventors evaluated MCF7 cells viability after overexpression of WT or catalytic mutants of p300 or EZH2, after treatment with MC2884 (Fig. 16). Interestingly, expression of p300 or EZH2 to higher levels leaded to increased cell death after MC2884, suggesting that both targets are important for the anticancer action of the drug in these settings. When catalytic mutants where used, only the mutant p300 expression fully reversed the MC2884 induction of cell death, whereas the mutant EZH2 gave rise to a partial reversion, potentially suggesting that both activities are needed but p300 inhibitory enzymatic action might be prevalent. Note that MC2884 action on EZH2 expression levels (and thus action that might be additional to the enzymatic regulation) is also potentially playing a role.

**MC2884 induces cancer-specific cell death**

Next, the inventors investigated the effects of MC2884 on cell cycle progression and cell death induction in several leukemias or solid tumor cell lines, such as colon, breast, cervix and brain (Fig. 5F, 7A and D). Dose-dependent apoptosis was observed for all. Especially leukemic cells appear responsive, showing early induction of apoptosis and

no increase in differentiation (Fig. 7A-C, see also SAR studies). The effect of MC2884 on two different immortalized non-cancer cell systems (29,41,42) (Fig. 5G) revealed low cytotoxicity at the dose effective on cancer cells. These data indicate that inhibitory action of MC2884 is potentially tumor cell selective, in line with *in vivo* pharmacokinetic and in *vitro* microsomal toxicity studies (see Pharmacology study below).

To assess effects on proliferation and invasiveness of cancer, the inventors performed proliferation and migration analyses using HCT116 and NB4 cells in real-time mode, revealing that inhibitory effects start at early time points (7-9 h) (Fig. 5H, 7B). Slope analysis (38) showed a strong decrease of migration rate (Fig. 5I), suggesting a strong anti-invasion effect of the drug. These data suggest wide-ranging anti-proliferative, anti-invasion and apoptotic effects of MC2884.

**Pharmacology study**

**Pharmacokinetic Analysis (Mice — IV, IP PK Study)**

Compound was administered both intravenously and intraperitoneally to mice. Blood samples were collected at 8 time points over 24hr and plasma analysed by LC-MS/MS to determine the concentration of compound.

The plasma time concentration profile was calculated with the main PK parameters (Co, AUClast, t.,VD, and CL).

**Formulation**

For ip dosing. After formulation assessment, MC2884 was dissolved at 0.2 mg/mL. This provided a dose of 2 mg free base material/kg when administered ip in a 10 mL/kg dosing volume.

For iv dosing. Compound was dissolved at 0.4 mg/mL. This provided a dose of 2 mg free base material/kg when administered iv in a 5 mL/kg dosing volume.

**Mouse terminal IP PK study**

Compound MC2884 was given ip at a dose of 2 mg/kg to a group of 24 normally fed male CD1 mice in a 10 mL/kg injection volume. Terminal blood samples (> 230 μL) were taken under CO2 from groups of 3 mice at each of 8 time-points post dose (0.083, 0.25, 0.5, 1, 2, 4, 8 and 24 h) and placed into heparinized tubes. Samples were placed on ice for no longer than 30 min before centrifugation (10,000 rpm × 3 min). Plasma samples (>100 μL) were collected into fresh tubes and frozen on dry ice. All samples were stored at -20 °C. Number of plasma samples = 24. Figure 17-18 display results for IV-IP administration of MC2884 in normal mice

**Bioavailability**

**Table 9.** MC2884 bioavailability

**AUC (IV)**

| Compound | Average (µg-hr/mL) |
|---|---|
| MC2884 | 13,322 |

**Dose (IV)**

| Compound | Average (mg) |
|---|---|
| MC2884 | 0,051 |

**AUC (IP)**

| Compound | Average (µg-hr/mL) |
|---|---|
| MC2884 | 18,413 |

**Dose (IP)**

| Compound | Average (mg) |
|---|---|
| MC2884 | 0,050 |

**% Bioavailability**

| Compound | Dose Route | Time frame (hr) | % Bioavailability |
|---|---|---|---|
| MC2884 | IP | 0-inf | 140,1% |

Studies of MC2884 bioavailability indicated the potential use of such compound *in vivo.*

**Mouse terminal IV PK study**

Compound MC2884 was given iv via the lateral tail vein at a dose of 2 mg/kg to a group of 24 normally fed male CD1 mice in a 5 mL/kg injection volume. Terminal blood samples (> 230 µL) were taken under CO2 from groups of 3 mice at each of 8 time-points post dose (0.083, 0.25, 0.5, 1, 2, 4, 8 and 24 h) and placed into heparinized tubes. Samples were placed on ice for no longer than 30 min before centrifugation (10,000 rpm × 3 min). Plasma samples (>100 µL) were collected into fresh tubes and frozen on dry ice. Number of plasma samples = 24. Consistent IV profiles were obtained, with little variability across the time-points (the average profile is shown in the second spreadsheet). An average half-life of 4.58h was calculated. Figure 17-18 display results for IV-IP administration of MC2884 in normal mice.

**Cytochrome P450 Inhibition (5 Isoform IC50 Determination)**

ADME-Tox Cytochrome P450 inhibition of MC2884 using human liver microsomes as an estimation *of in vitro* metabolism.

MC2884 (0.1 µM — 25 µM) was incubated with human liver microsomes and NADPH in the presence of a cytochrome P450 isoform-specific probe substrate. For the CYP2C9, CYP2C19, CYP2D6 and CYP3A4 specific reactions, the metabolites were monitored by mass spectrometry. CYP1A activity was monitored by measuring the formation of a fluorescent metabolite. A decrease in the formation of the metabolite compared to the vehicle control was used to calculate an IC50 value (test compound concentration which produces 50 % inhibition).

**CYP1A Inhibition**

Six test compound concentrations (0.1, 0.25, 1, 2.5, 10, 25 µM in DMSO; final DMSO concentration = 0.3 %) were incubated with human liver microsomes (0.25 mg/mL) and NADPH (1 mM) in the presence of the probe substrate ethoxyresorufin (0.5 µM) for 5 min at 37 °C. The selective CYP1A inhibitor, alpha-naphthoflavone, was screened alongside the test compounds as a positive control.

**CYP2C9 Inhibition**

Six test compound concentrations (0.1, 0.25, 1, 2.5, 10, 25 µM in DMSO; final DMSO concentration = 0.25 %) were incubated with human liver microsomes (1 mg/mL) and NADPH (1 mM) in the presence of the probe substrate tolbutamide (120 µM) for 60 min at 37 °C. The selective CYP2C9 inhibitor, sulphaphenazole, was screened alongside the test compounds as a positive control.

### CYP2C19 Inhibition

Six test compound samples at concentrations of 0.1, 0.25, 1, 2.5, 10, 25 μM in DMSO (final DMSO concentration = 0.25 %) were incubated with human liver microsomes (0.5 mg/mL) and NADPH (1 mM) in the presence of the probe substrate mephenytoin (25 μM) for 60 min at 37 °C. The selective CYP2C19 inhibitor, tranylcypromine, was screened alongside the test compounds as a positive control.

### CYP2D6 Inhibition

Six test compound samples at concentrations of 0.1, 0.25, 1, 2.5, 10, 25 μM in DMSO (final DMSO concentration = 0.25 %) were incubated with human liver microsomes (0.5 mg/mL) and NADPH (1 mM) in the presence of the probe substrate dextromethorphan (5 μM) for 5 min at 37 °C. The selective CYP2D6 inhibitor, quinidine, was screened alongside the test compounds as a positive control.

### CYP3A4 Inhibition

Six test compound concentrations (0.1, 0.25, 1, 2.5, 10, 25 μM in DMSO; final DMSO concentration 0.26 %) were incubated with human liver microsomes (0.1 mg/mL) and NADPH (1 mM) in the presence of the probe substrate midazolam (2.5 μM) for 5 min at 37 °C. The selective CYP3A4 inhibitor, ketoconazole, was screened alongside the test compounds as a positive control.

For the CYP1A incubations, the reactions were terminated by methanol, and the formation of the metabolite, resorufin, was monitored by fluorescence (excitation wavelength = 535 nm, emission 5 wavelength = 595 nm). For the CYP2C9, CYP2C19, CYP2D6 and CYP3A4 incubations, the reactions were terminated by methanol. The samples were then centrifuged, and the supernatants were combined, for the simultaneous analysis of 4-hydroxytolbutamide, 4-hydroxymephenytoin, dextrorphan, and 1-hydroxymidazolam plus internal standard by LC-MS/MS. Formic acid in deionized water containing internal standard (final formic acid concentration = 0.1 %) was added to the final sample prior to analysis. A decrease in the formation of the metabolites compared to vehicle control was used to calculate an IC50 value (test compound concentration which produces 50 % inhibition).

a) CYP1A no inhibition, IC50 (μM) >25
b) CYP2C9 no inhibition, IC50 (μM) >25
c) CYP2C19 no inhibition, IC50 (μM) >25
d) CYP2D6 no inhibition, IC50 (μM) >25
d) CYP3A4 45% inhibition, observed only at 25 μM

The results demonstrate that microsomial enzymes are not affected by MC2884, supporying that the compound possesses substantially no cytotoxic effects. It should be noted that the 45% of CYP3A4 inhibition was observed at a 25 μM dose. Such dose is very high compared to the dose use in the clinic.

### MC2884 displays anticancer action in both hematological and solid cancer *in vivo models*

To assess the anti-leukemic activity of MC2884 *in vivo,* immune-compromised mice were retro-orbitally injected with U937 or NB4 cells, and treated with 1 mg/kg MC2884 (Fig. 8A). The mice were sacrificed 16 days post-xenograft and the bone marrow examined by flow cytometry. In NB4 and U937 xenografts, MC2884-treatment caused a reduction of human $CD45^+$ cells to 17% and 24%, respectively (Fig. 8A), suggesting a strong *in vivo* anti-leukemic action. These results could be further strengthened using NB4 cells expressing luciferase (Figure 9A). MC2884 also displayed a strong anticancer effect in a xenograft model of HCT116 colon carcinoma (Fig. 8B). Tumor-immunohistochemical analyses showed that MC2884 was able to induce strong apoptosis and a block of cell proliferation (Fig. 8C). In addition, MC2884 displayed a strong down-regulation of both H3K9/14ac and H3K27me3 (Fig. 8C-D). Similar effects were observed in a $HCT116^{p53-/-}$ xenograft model (Fig. 9D). The inventors examined genome-wide H3K27ac in HCT116 cells and observed a decrease after treatment (Fig. 8E), for example at the anti-apoptotic gene BIRC5 (Fig. 8F). In addition, when using DNMT1/DNMT3B double knock out HCT116 cells (HCT116 DKO), which are characterized by increased acetylation (43), the inventors again observed decreases in H3K27ac after MC2884 treatment specifically at those regions hyperacetylated in HCT116 DKO as compared to normal HCT116 (Fig. 8G). Taken together these results strengthen MC2884 anticancer action in both hematological and solid cancer *in vivo* models and its connection to epigenome modulation.

**MC2884 induces caspase- and mitochondrial-dependent cancer-selective apoptosis**

To gain mechanistic insight into the cell death induced by MC2884, caspase activities were analyzed. MC2884 treatment induced caspase-3/7 activity as well as the activation of both caspase-8 and -9 (Fig. 9C-D). This activation was blocked by the pan-caspase inhibitor Z-VAD, but also by IETD and LEHD, showing a key role of caspases in MC2884-mediated apoptosis. MC2884-induced apoptosis is causally associated with ROS production since, when the inventors treated NB4 cells with MC2884 and the antioxidant N-acetylcysteine (NAC) this blocked apoptosis induced by MC2884 (Fig. 9E).

To further characterize the molecular pathways affected by MC2884, protein levels of crucial players of the apoptosis pathway were investigated (Fig. 10A). MC2884 induced activation of the mitochondrial pathway by up-regulation of cleaved-BID, and strong down-regulation of BCL2 and BCLX(L). In accordance with caspase-3/7 activation, MC2884 induced activation of PARP. Interestingly, BCL2 reduction occurred also at the RNA level (Fig. 10B) suggesting its potential regulation at the chromatin level. As TRAIL regulation was weak and RIP up-regulation relatively late, the intrinsic pathways associated with caspase-9 and apoptosome activation seemed to play an essential role.

**MC2884-induced apoptosis in aggressive cancers, primary AML blasts and APL-Rs**

To further strengthen our findings in primary human cells, apoptosis was evaluated in 6 AML patient blasts (Fig. 10C). In all cases, 24-hour treatment with MC2884 correlated with apoptosis. In addition, MC2884 treatment led to apoptosis in *ex vivo* primary blasts derived from one acute lymphoblastic leukemia (ALL) (Fig. 10D). Importantly, the APL-Rs (Fig. 2 and 4) that were not responding to ATRA *in vivo* (pt#7 and 8), also did not undergo efficient apoptosis *ex vivo* in response to HDACi or ATRA when compared to ATRA sensitive blasts (pt#9), whereas *ex vivo* MC2884 treatment was accompanied by apoptosis induction (Fig. 10E). Accordingly, MC2884 was able to induce an apoptotic response in Tet2$^{-/-}$ APL murine cells, a mouse model for aggressive human APL (Fig. 10F), and in HCT116-$^{p53-/-}$ colon cancer cells (Fig. 10G), which also displays a very aggressive behavior. In full agreement with our findings (Fig. 2 and 4), H3K27ac ChIP-seq analysis of MC2884 treated APL-Tet2$^{-/-}$ and APL-wt murine cells showed a strong H3K27ac reduction in both (Fig. 11A-B).

***Ex vivo* MC2884-treatment remodels the epigenome of APL-Rs**

As the APL-Rs harbor an aberrant H3K9/14ac and H3K27me3 signature the inventors wondered whether MC2884 would reset acetylation and H3K27me3 patterns. The inventors therefore treated pt#8 blasts with MC2884 *ex vivo* for 4 and 24 h and examined H3K27ac and H3K27me3 levels. The inventors identified 3636 H3K27ac (Fc > 3x stdev, % gain, % loss) and 799 H3K27me3 genomic regions for which occupancy was changed (Fc > 3x st dev, % gain, % loss) (Fig. 12A; Fig. 11C-D). Functional annotation of these regions revealed enrichment for genes associated with AML, PML-RARA binding, JAK/STAT signaling, Toll-like receptor signaling, and the RAS pathway, for example the SEC32A/GEMIN2, AK2 and BCL2 genomic regions (Fig. 12B; Fig. 11E-F).

Interestingly, reduced H3K27ac was also observed at a putative enhancer region of EZH2 (Fig. 12C), strengthening that MC2884-chromatin action also affects EZH2 transcription (Fig. 5C and 9B). Indeed expression of EZH2 was reduced, similar as for other genes associated with reduced enhancer acetylation (Fig. 12D, left). In contrast, genes associated with reduction in H3K27me3 increased in expression (Fig. 12D, right). In agreement with these and earlier results (Fig. 10A-B), decreased BCL2 RNA expression was observed upon MC2884 treatment in primary blasts derived from pt#8 (Fig. 12E). Inversely, ATRA induced an up-regulation of BCL2. Notably, in a sensitive APL (pt#9), ATRA induced a down-regulation of BCL2, suggesting a key role for BCL-2 reduction in allowing good responsiveness.

Importantly, MC2884-structurally related compounds inactive in inducing both anticancer effects and epigenome modulation were not able to down-regulate BCL2 (SAR studies). As expected, MC2884 reduced BCL2 promoter acetylation (Fig. 12F, left) and decreased BCL2 expression more than a p300 and EZH2 inhibitor combination (Fig. 12G). Furthermore, the inventors found loss of H3K27me3 and reactivation of different genes associated with the folic acid metabolic pathway, which is commonly deregulated in AML (44,45) (Fig. 12F, right panel).

To assess the contribution of BCL2 to the antitumor action of MC2884, the effects of an inhibitor of BCL2 (ABT-737) was analyzed (Fig. 12H). The anti-leukemic action of ABT-737 was strongly potentiated, reaching up to 60% of cell death, by addition of MC2884 at concentrations that alone induced little apoptosis. This synergistic antitumor effect was also reproduced in the resistant NB4-MR4 (46), suggesting that MC2884 has potential for combination therapy in a wide range of leukemias.

**Discussion**

Progressive genome alterations and epigenome deregulation, and their cross-talk, underlie cancer etiology

and progression. Currently approved treatments focusing on deregulation of epigenetic components are often based on the use of HDACi (47,48) or DNA demethylating agents, aiming at chromatin opening by increasing acetylation levels or lowering DNA methylation, respectively. However, conceptually many pathways that increase stemness and cancer cell survival should rather be switched off. We here describe such an opposite approach and as such a 'paradigm shift', where hyperacetylation of chromatin areas together with selective H3K27 methylation (and consequently transcriptome and methylome remodeling) are targets for cancer therapy.

MC2884, selected from a collection of newly synthesized chromatin-modulating drugs for its strong anticancer potential, seems highly effective in the treatment of solid and hematopoietic cancers, including relapsed cancers and those that are resistant to standard treatments. It unbalances histone acetylation acting as a *bona fide* HAT inhibitor in the low micromolar range. Displaying also an inhibitory action towards EZH2, it potentially targets and counteracts both pathologically opened and activated loci by inhibiting the acetyltransferases, and regions closed by increased H3K27me3 by the histone methyltransferase EZH2. MC2884 results in down-regulation of anti-apoptotic drivers such as BCL2 and BCLX(L), mitochondrial modulation and in activation of related death pathways that kill cancer cells in a selective manner. *In vivo,* AML, APL and colon cancer xenograft models suggest a very robust anticancer potential most likely related to chromatin modulation. Excitingly, both $p53^{-/-}$ colon cancer and $Tet2^{-/-}$ APL cells, which show a highly aggressive behavior (49-51), displayed high sensitivity to MC2884, suggesting that as a consequence of the MC2884-mediated induction of hypoacetylation, patients with aggressive cancer phenotypes (that may be in an overall hyperacetylated state) might benefit from this type of approach. Strengthening this view, *ex vivo* treatment of APL-R blasts displayed improved and selective sensitivity to MC2884, suggesting an enhanced benefit of this therapeutic strategy.

The mechanism of apoptosis of MC2884 treatment pointed to BCL2 mediated regulation; acetylation at BCL2 chromatin regions is reduced in MC2884-treated APL-R and in accordance BCL2 expression is decreased. Furthermore, BCL2 is oppositely regulated by ATRA in 'normal' APLs and APL-Rs being down- and up-regulated, respectively, suggesting a link to ATRA (and HDACi) resistance. Strikingly, the use of the BCL2 protein inhibitor (ABT-737) together with the chromatin modulation exerted by MC2884 on BCL2 is a successful combination that synergistically kills both ATRA-sensitive and -resistant APL cells. This suggests that APL-R patients might benefit from a personalized, therapeutic strategy. Since the BCL2i ABT-737 is in clinical trial for different types of cancers (52), this approach should be feasible in not too long a time.

**References**

1. Genomic and epigenomic landscapes of adult de novo acute myeloid leukemia. N Engl J Med 2013;368(22):2059-74 doi 10.1056/NEJMoa1301689.
2. Weinstein JN, et al., Nat Genet 2013;45(10):1113-20 doi 10.1038/ng.2764.
3. Sun XJ, et al., Front Oncol 2015;5:108 doi 10.3389/fonc.2015.00108.
4. Avvakumov N, Cote J. Oncogene 2007;26(37):5395-407 doi 10.1038/sj.onc.1210608.
5. Greer EL, Shi Y. Nat Rev Genet 2012;13(5):343-57 doi 10.1038/nrg3173.
6. Falkenberg KJ, Johnstone RW. Nat Rev Drug Discov 2014;13(9):673-91
7. Parry L, Clarke AR. Genes Cancer 2011;2(6):618-30 doi 10.1177/1947601911418499.
8. Muller S, Filippakopoulos P, Knapp S. Expert Rev Mol Med 2011;13:e29
9. Filippakopoulos P, et al. Nature 2010;468(7327):1067-73 doi 10.1038/nature09504.
10. Kolla V, et al., Cancer Res 2014;74(3):652-8 doi 10.1158/0008-5472.CAN-13-3056.
11. Jariwala N, et al., . Int J Oncol 2015;46(2):465-73 doi 10.3892/ijo.2014.2766.
12. Musselman CA, et al., Mol Interv 2009;9(6):314-23 doi 10.1124/mi.9.6.7.
13. Conte M, et al., Clin Cancer Res 2012;18(20):5526-34
14. You JS, et al., Cancer cell 2012;22(1):9-20 doi 10.1016/j.ccr.2012.06.008.
15. Shen H, Laird PW. Cell 2013;153(1):38-55 doi 10.1016/j.cell.2013.03.008.
16. Mandoli A, et al., Cell reports 2016;17(8):2087-100 doi 10.1016/j.celrep.2016.08.082.
17. Papaemmanuil E, et al., N Engl J Med 2016;374(23):2209-21
18. Queiros AC, et al., Cancer cell 2016;30(5):806-21 doi 10.1016/j.ccell.2016.09.014.
19. Nebbioso A, et al., Expert Opin Ther Targets 2015;19(9):1187-202
20. Nebbioso A, et al., Mol Oncol 2012;6(6):657-82 doi 10.1016/j.molonc.2012.09.004.
21. Nebbioso A, et al., Clin Cancer Res 2016 doi 10.1158/1078-0432.CCR-15-2388.
22. Baylin SB, et al., Nat Rev Cancer 2011;11(10):726-34 doi 10.1038/nrc3130.
23. Laubach JP, et al., Clin Cancer Res 2015;21(21):4767-73
24. Wu H, et al., PLoS One 2013;8(12):e83737 doi 10.1371/journal.pone.0083737.
25. Wagener N, et al., BMC Cancer 2010;10:524 doi 10.1186/1471-2407-10-524.

26. Antonysamy S, et al., PLoS One 2013;8(12):e84147 doi 10.1371/journal.pone.0084147.
27. Dillon SC, et al., Genome Biol 2005;6(8):227 doi 10.1186/gb-2005-6-8-227.
28. Franci G, et al., Epigenetics 2016:0 doi 10.1080/15592294.2016.1249089.
29. Miceli M, et al., Stem Cells Dev 2013;22(17):2368-83 doi 10.1089/scd.2012.0498.
30. Martens et al., FEBS Lett 2010;584(12):2662-9 doi 10.1016/j.febslet.2010.04.002.
31. Conte M, et al., Oncotarget 2015;6(2):886-901 doi 10.18632/oncotarget.2816.
32. Saeed S, et al., Blood 2012;120(15):3058-68 doi 10.1182/blood-2011-10-386086.
33. De Bellis F, et al., Cancer Res 2014;74(8):2328-39
34. Nebbioso A, et al., Nat Med 2005;11(1):77-84 doi 10.1038/nm1161.
35. Valente S, et al., Biochimie 2012;94(11):2308-13 doi 10.1016/j.biochi.2012.06.003.
36. Nebbioso A, et al., EMBO Rep 2009;10(7):776-82 doi 10.1038/embor.2009.88.
37. Valente S, et al., J Med Chem 2016 doi 10.1021/acs.jmedchem.5b01117.
38. Rotili D, et al., J Med Chem 2014;57(1):42-55 doi 10.1021/jm4012802.
39. Adams D, et al., Nat Biotechnol 2012;30(3):224-6 doi 10.1038/nbt.2153.
40. Villa R, et al., Cancer cell 2007;11(6):513-25 doi 10.1016/j.ccr.2007.04.009.
41. Boccellino M, et al., J Cell Biochem 2012;113(4):1292-301 doi 10.1002/jcb.24000.
42. Miceli M, A et al., Proteomics 2015 doi 10.1002/pmic.201500223.
43. Maurano MT, et al., Cell reports 2015;12(7):1184-95 doi 10.1016/j.celrep.2015.07.024.
44. de Jonge R, et al., Blood 2009;113(10):2284-9 doi 10.1182/blood-2008-07-165928.
45. Thaler R, et al., J Biol Chem 2011;286(7):5578-88 doi 10.1074/jbc.M110.166181.
46. Rosenauer A, et al., Blood 1996;88(7):2671-82.
47. Benedetti R, et al., Antioxid Redox Signal 2015;23(1):99-126
48. Dell'Aversana C, et al., Leukemia 2017 doi 10.1038/leu.2017.64.
49. Delhommeau F, et al., N Engl J Med 2009;360(22):2289-301
50. Quivoron C, et al., Cancer cell 2011;20(1):25-38 doi 10.1016/j.ccr.2011.06.003.
51. Malinge S, et al., Blood 2008;112(10):4220-6 doi 10.1182/blood-2008-01-136366.
52. Rooswinkel RW, et al., Cell Death Dis 2012;3:e366 doi 10.1038/cddis.2012.109.
53. Rajesh SM, Bala BD, Tetrahedron Letters 2012;53(40):5367-71.
54. Han Z. TS, et al., Synthesis 2009:8 doi 10.1055/s-0028-1088052.
55. Wei X, Du ZY, et al., Eur J Med Chem 2012;53:235-45
56. Wan YW, et al., Synthetic Communications 2010;40(15):9.
57. Chaib H, et al., Leukemia 2012;26(4):662-74 doi 10.1038/leu.2011.271.
58. Weisberg E, et al., Mol Cancer Ther 2015;14(10):2249-59
59. Rhee I, et al., Nature 2002;416(6880):552-6 doi 10.1038/416552a.
60. Nebbioso A, et al., EMBO Rep 2009;10(7):776-82 doi 10.1038/embor.2009.88.
61. Valente S, et al., J Med Chem 2016 doi 10.1021/acs.jmedchem.5b01117.
62. Martens JH, et al., Biochim Biophys Acta 2011;1812(8):818-23
63. Conte M, et al., Oncotarget 2015;6(2):886-901 doi 10.18632/oncotarget.2816.
64. Saeed S, et al., Blood 2012;120(15):3058-68 doi 10.1182/blood-2011-10-386086.
65. Wu TD, et al., Bioinformatics 2010;26(7):873-81 doi 10.1093/bioinformatics/btq057.
66. Kanitz A, et al., Genome Biol 2015;16:150 doi 10.1186/s13059-015-0702-5.
67. Vogelstein B, et al., Science 2013;339(6127):1546-58 doi 10.1126/science.1235122.
68. Ley TJ et al. (2013). N Engl J Med. 2013 May 30;368(22):2059-74.
69. Di Cerbo V, et al., Brief Funct Genomics. 2013 May;12(3):231-43.
70. Manzo F, et al., Expert Opin Ther Pat. 2009 Jun;19(6):761-74.
71. Kim KH, et al., Nat Med. 2016 Feb 4;22(2):128-34.
72. Tanaka M, et al., Pharm Pat Anal. 2015;4(4):261-84.

## Claims

**1.** An *in vitro* method for the diagnostic of low overall survival acute promyelocytic leukemia and/or of predicting and/or monitoring the response and/or the efficacy of a therapy for acute promyelocytic leukemia or to identify a subject to be treated with an inhibitor of HAT and/or an inhibitor of EZH2 comprising determining:

a) the acetylation status in all of the following acetylation relevant regions:

| chromosome | start | end |
|---|---|---|
| chr2 | 102761838 | 102765245 |
| chr2 | 138558210 | 138560637 |
| chr3 | 138917149 | 138918464 |
| chr4 | 47722119 | 47725095 |
| chr5 | 177820647 | 177821809 |
| chr5 | 177911126 | 177912881 |
| chr6 | 10080702 | 10085536 |
| chr6 | 100796638 | 100797329 |
| chr7 | 43539997 | 43544821 |
| chr8 | 41107099 | 41109973 |
| chr8 | 41271751 | 41275593 |
| chr8 | 109131198 | 109133248 |
| chr10 | 31145487 | 31151138 |
| chr10 | 31152266 | 31155251 |
| chr10 | 82011240 | 82013721 |
| chr12 | 1741915 | 1746279 |
| chr13 | 110036141 | 110039705 |
| chr13 | 110048447 | 110050505 |
| chr14 | 20955081 | 20960400 |
| chr15 | 97270509 | 97273147 |
| chr17 | 426695 | 431121 |
| chr18 | 61534509 | 61537791 |
| chr18 | 74255741 | 74257346 |
| chr19 | 15742473 | 15746990 |
| chr20 | 48404657 | 48406416 |
| chr21 | 32507312 | 32510923 |
| chr22 | 19230646 | 19232961 |
| chrX | 2850454 | 2853230 |
| chrX | 147574563 | 147578311 |

| chromosome | start | end |
|---|---|---|
| chr1 | 95181929 | 95184074 |
| chr1 | 109626120 | 109627905 |
| chr1 | 249221773 | 249222010 |
| chr11 | 57158202 | 57159391 |
| chr14 | 61416231 | 61417467 |
| chr14 | 105391316 | 105391725 |
| chr17 | 70982083 | 70984392 |
| chr17 | 71061705 | 71070236 |
| chr2 | 27060445 | 27061863 |
| chr2 | 102056668 | 102060845 |
| chr2 | 234072888 | 234075888 |
| chr20 | 2665743 | 2666563 |
| chr22 | 50153595 | 50155770 |
| chr3 | 41011746 | 41014498 |
| chr6 | 10087785 | 10088550 |
| chr6 | 82468660 | 82469114 |
| chr6 | 139948334 | 139949640 |
| chr6 | 140000376 | 140002294 |
| chr7 | 29323864 | 29325989 |
| chr9 | 94907894 | 94910771 |
| chrX | 37605417 | 37607058 |
| chrX | 151152531 | 151154618 |

or

b) the methylation status of all CpG methylation relevant regions of:

| chromosome | start | end |
|---|---|---|
| chr1 | 1052949 | 1052950 |
| chr1 | 12655992 | 12655993 |
| chr1 | 17287501 | 17287502 |
| chr1 | 55266578 | 55266579 |
| chr2 | 237477262 | 237477263 |
| chr3 | 35706114 | 35706115 |
| chr3 | 57198244 | 57198245 |
| chr4 | 3516534 | 3516535 |
| chr4 | 163085348 | 163085349 |
| chr6 | 73972852 | 73972853 |
| chr12 | 57630107 | 57630108 |
| chr12 | 114841708 | 114841709 |
| chr14 | 24779959 | 24779960 |
| chr16 | 1429015 | 1429016 |
| chr16 | 20817501 | 20817502 |
| chr16 | 46919112 | 46919113 |
| chr16 | 67197640 | 67197641 |
| chr17 | 39845949 | 39845950 |
| chr19 | 3275394 | 3275395 |
| chr19 | 35531990 | 35531991 |
| chr19 | 39056084 | 39056085 |
| chr20 | 21687378 | 21687379 |
| chr20 | 62084626 | 62084627 |

in a biological sample obtained from the subject.

**2.** The in vitro method of claim 1 wherein determining the methylation status comprises the steps of:

(a) isolating DNA from a sample obtained from a subject;
(b) subjecting said DNA to methylation-sensitive sequence modification through incubation with chemicals (e.g., sodium bisulfite), or contacting said DNA with a polypeptide capable of binding methylated DNA;
(c) optionally amplifying said DNA modified with sodium bisulfite or bound by said polypeptide; and
(d) determining the methylation status of said DNA.

**3.** The in vitro method of any one of claims 1 or 2 wherein determining the acetylation status comprises the steps of comprising the steps of:

(a) isolating DNA from a sample obtained from a subject;
(b) optionally amplifying said DNA; and
(c) determining the acetylation status of said DNA.

**4.** A kit for performing the method of any one of claims 1 to 3, comprising a chemical able to differentially modify DNA sequence depending on methylation status, or a polypeptide or agent capable of binding methylated and/or acetylated DNA, and antibodies, probes, primers and/or aptamers specific for the regions as defined in claim 1.

**5.** Use of the kit of claim 4 for the diagnostic of resistant acute promyelocytic leukemia and/or of predicting and/or monitoring the response and/or the efficacy of a therapy or to identify a subject to be treated with an inhibitor of HAT and/or an inhibitor of EZH2.

**6.** Use of a microarray able to determine the acetylation status in all acetylation relevant regions as defined in claim

1 and the methylation status of all CpG methylation relevant regions as defined in claim 1 said microarray comprising probes, primers and/or aptamers specific for the regions as defined in claim 1 for performing the method of any one of claims 1 to 3.

## Patentansprüche

**1.** In Vitro-Verfahren zum Diagnostizieren von akuter Promyelozytenleukämie mit geringem Gesamtüberleben und/oder Voraussagen und/oder Verfolgen der Reaktion und/oder der Wirksamkeit einer Therapie für akute Promyelozytenleukämie oder zum Identifizieren eines Subjekts, das mit einem Inhibitor von HAT und/oder einem Inhibitor von EZH2 behandelt werden soll, umfassend Bestimmen:

a) des Acetylierungszustands in allen den folgenden, für die Acetylierung relevanten Regionen

| Chromosom | Anfang | Ende |
|---|---|---|
| chr2 | 102761838 | 102765245 |
| chr2 | 138558210 | 138560637 |
| chr3 | 138917149 | 138918464 |
| chr4 | 47722119 | 47725095 |
| chr5 | 177820647 | 177821809 |
| chr5 | 177911126 | 177912881 |
| chr6 | 10080702 | 10085536 |
| chr6 | 100796638 | 100797329 |
| chr7 | 43539997 | 43544821 |
| chr8 | 41107099 | 41109973 |
| chr8 | 41271751 | 41275593 |
| chr8 | 109131198 | 109133248 |
| chr10 | 31145487 | 31151138 |
| chr10 | 31152266 | 31155251 |
| chr10 | 82011240 | 82013721 |
| chr12 | 1741915 | 1746279 |
| chr13 | 110036141 | 110039705 |
| chr13 | 110048447 | 110050505 |
| chr14 | 20955081 | 20960400 |
| chr15 | 97270509 | 97273147 |
| chr17 | 426695 | 431121 |
| chrl8 | 61534509 | 61537791 |
| chrl8 | 74255741 | 74257346 |
| chrl9 | 15742473 | 15746990 |
| chr20 | 48404657 | 48406416 |
| chr21 | 32507312 | 32510923 |
| chr22 | 19230646 | 19232961 |
| chrX | 2850454 | 2853230 |
| chrX | 147574563 | 147578311 |

(fortgesetzt)

| Chromosom | Anfang | Ende |
|---|---|---|
| chr1 | 95181929 | 95184074 |
| chr1 | 109626120 | 109627905 |
| chr1 | 249221773 | 249222010 |
| chr11 | 57158202 | 57159391 |
| chr14 | 61416231 | 61417467 |
| chr14 | 105391316 | 105391725 |
| chr17 | 70982083 | 70984392 |
| chr17 | 71061705 | 71070236 |
| chr2 | 27060445 | 27061863 |
| chr2 | 102056668 | 102060845 |
| chr2 | 234072888 | 234075888 |
| chr20 | 2665743 | 2666563 |
| chr22 | 50153595 | 50155770 |
| chr3 | 41011746 | 41014498 |
| chr6 | 10087785 | 10088550 |
| chr6 | 82468660 | 82469114 |
| chr6 | 139948334 | 139949640 |
| chr6 | 140000376 | 140002294 |
| chr7 | 29323864 | 29325989 |
| chr9 | 94907894 | 94910771 |
| chrX | 37605417 | 37607058 |
| chrX | 151152531 | 151154618 |

b) des Methylierungszustands aller für CpG-Methylierung relevanten Regionen von

| Chromosom | Anfang | Ende |
|---|---|---|
| chr1 | 1052949 | 1052950 |
| chr1 | 12655992 | 12655993 |
| chr1 | 17287501 | 17287502 |
| chr1 | 55266578 | 55266579 |
| chr2 | 237477262 | 237477263 |
| chr3 | 35706114 | 35706115 |
| chr3 | 57198244 | 57198245 |
| chr4 | 3516534 | 3516535 |
| chr4 | 163085348 | 163085349 |
| chr6 | 73972852 | 73972853 |
| chr12 | 57630107 | 57630108 |
| chr12 | 114841708 | 114841709 |

(fortgesetzt)

| Chromosom | Anfang | Ende |
|---|---|---|
| chr14 | 24779959 | 24779960 |
| chrl6 | 1429015 | 1429016 |
| chrl6 | 20817501 | 20817502 |
| chrl6 | 46919112 | 46919113 |
| chrl6 | 67197640 | 67197641 |
| chr17 | 39845949 | 39845950 |
| chr19 | 3275394 | 3275395 |
| chr19 | 35531990 | 35531991 |
| chr19 | 39056084 | 39056085 |
| chr20 | 21687378 | 21687379 |
| chr20 | 62084626 | 62084627 |

in einer biologischen Probe, die von dem Subjekt erhalten worden ist.

**2.** In-Vitro-Verfahren nach Anspruch 1, wobei das Bestimmen des Methylierungszustands die Schritte umfasst des:

(a) Isolierens von DNA aus einer Probe, die von einem Subjekt abgenommen worden ist;
(b) Unterwerfens der DNA einer methylierungsempfindlichen Sequenzmodifikation durch Inkubieren mit Chemikalien (z.B. Natriumbisulfit) oder Kontaktieren der DNA mit einem Polypeptid, das in der Lage ist, methylierte DNA zu binden;
(c) wahlweise Amplifizierens der mit Natriumbisulfit modifizierten oder durch das Polypeptid gebundenen DNA; und
(d) Bestimmens des Methylierungszustands der DNA.

**3.** In-Vitro-Verfahren nach einem der Ansprüche 1 oder 2, wobei das Bestimmen des Acylierungszustands die Schritte umfasst des:

(a) Isolierens von DNA von einer Probe, die von einem Subjekt abgenommen worden ist;
(b) wahlweise Amplifizierens der DNA; und
(c) Bestimmens des Acetylierungszustands der DNA.

**4.** Kit zum Ausführen des Verfahrens nach einem der Ansprüche 1 bis 3, umfassend eine Chemikalie, die in der Lage ist, eine DNA-Sequenz je nach dem Methylierungszustand unterschiedlich zu modifizieren, oder ein Polypeptid oder Mittel, das in der Lage ist, methylierte und/oder acetylierte DNA und Antikörper, Sonden, Primer und/oder Aptamere, die für die Regionen, wie in Anspruch 1 definiert, spezifisch sind, zu binden.

**5.** Verwendung des Kits nach Anspruch 4 zum Diagnostizieren von resistenter akuter Promyelozytenleukämie und/oder Voraussagen und/oder Verfolgen der Reaktion und/oder der Wirksamkeit einer Therapie oder zum Identifizieren eines mit einem Inhibitor von HAT und/oder einem Inhibitor von EZH2 zu behandelnden Subjekts.

**6.** Verwendung eines Microarrays, das in der Lage ist, den Acetylierungszustand in allen acetylierungsrelevanten Regionen, wie in Anspruch 1 definiert, und den Methylierungszustand aller CpG-methylierungsrelevanten Regionen, wie in Anspruch 1 definiert, zu bestimmen, wobei das Microarray Sonden, Primer und/oder Aptamere, die für die Regionen, wie in Anspruch 1 definiert, spezifisch sind, umfasst, zum Ausführen des Verfahrens nach einem der Ansprüche 1 bis 3.

## Revendications

**1.** Procédé *in vitro* de diagnostic d'une leucémie promyélocytaire aiguë à faible survie globale et/ou de prédiction et/ou de surveillance de la réponse et/ou de l'efficacité d'un traitement contre la leucémie promyélocytaire aiguë ou d'identification d'un sujet devant être traité à l'aide d'un inhibiteur de la HAT et/ou d'un inhibiteur d'EZH2, comprenant la détermination :

a) de l'état d'acétylation dans toutes les régions pertinentes d'acétylation suivantes :

| chromosome | début | fin |
|---|---|---|
| chr2 | 102761838 | 102765245 |
| chr2 | 138558210 | 138560637 |
| chr3 | 138917149 | 138918464 |
| chr4 | 47722119 | 47725095 |
| chr5 | 177820647 | 177821809 |
| chr5 | 177911126 | 177912881 |
| chr6 | 10080702 | 10085536 |
| chr6 | 100796638 | 100797329 |
| chr7 | 43539997 | 43544821 |
| chr8 | 41107099 | 41109973 |
| chr8 | 41271751 | 41275593 |
| chr8 | 109131198 | 109133248 |
| chr10 | 31145487 | 31151138 |
| chr10 | 31152266 | 31155251 |
| chr10 | 82011240 | 82013721 |
| chr12 | 1741915 | 1746279 |
| chr13 | 110036141 | 110039705 |
| chr13 | 110048447 | 110050505 |
| chr14 | 20955081 | 20960400 |
| chr15 | 97270509 | 97273147 |
| chr17 | 426695 | 431121 |
| chr18 | 61534509 | 61537791 |
| chrl8 | 74255741 | 74257346 |
| chrl9 | 15742473 | 15746990 |
| chr20 | 48404657 | 48406416 |
| chr21 | 32507312 | 32510923 |
| chr22 | 19230646 | 19232961 |
| chrX | 2850454 | 2853230 |
| chrX | 147574563 | 147578311 |
| chr1 | 95181929 | 95184074 |
| chr1 | 109626120 | 109627905 |
| chr1 | 249221773 | 249222010 |
| chr11 | 57158202 | 57159391 |

(suite)

| chromosome | début | fin |
|---|---|---|
| chr14 | 61416231 | 61417467 |
| chr14 | 105391316 | 105391725 |
| chr17 | 70982083 | 70984392 |
| chr17 | 71061705 | 71070236 |
| chr2 | 27060445 | 27061863 |
| chr2 | 102056668 | 102060845 |
| chr2 | 234072888 | 234075888 |
| chr20 | 2665743 | 2666563 |
| chr22 | 50153595 | 50155770 |
| chr3 | 41011746 | 41014498 |
| chr6 | 10087785 | 10088550 |
| chr6 | 82468660 | 82469114 |
| chr6 | 139948334 | 139949640 |
| chr6 | 140000376 | 140002294 |
| chr7 | 29323864 | 29325989 |
| chr9 | 94907894 | 94910771 |
| chrX | 37605417 | 37607058 |
| chrX | 151152531 | 151154618 |

b) de l'état de méthylation de toutes les régions pertinentes de méthylation CpG

| chromosome | début | fin |
|---|---|---|
| chr1 | 1052949 | 1052950 |
| chr1 | 12655992 | 12655993 |
| chr1 | 17287501 | 17287502 |
| chr1 | 55266578 | 55266579 |
| chr2 | 237477262 | 237477263 |
| chr3 | 35706114 | 35706115 |
| chr3 | 57198244 | 57198245 |
| chr4 | 3516534 | 3516535 |
| chr4 | 163085348 | 163085349 |
| chr6 | 73972852 | 73972853 |
| chr12 | 57630107 | 57630108 |
| chr12 | 114841708 | 114841709 |
| chr14 | 24779959 | 24779960 |
| chr16 | 1429015 | 1429016 |
| chr16 | 20817501 | 20817502 |
| chr16 | 46919112 | 46919113 |

(suite)

| chromosome | début | fin |
|---|---|---|
| chr16 | 67197640 | 67197641 |
| chr17 | 39845949 | 39845950 |
| chr19 | 3275394 | 3275395 |
| chr19 | 35531990 | 35531991 |
| chr19 | 39056084 | 39056085 |
| chr20 | 21687378 | 21687379 |
| chr20 | 62084626 | 62084627 |

dans un échantillon biologique obtenu du sujet.

**2.** Procédé *in vitro* selon la revendication 1 dans lequel la détermination de l'état de méthylation comprend les étapes suivantes :

(a) isolement d'ADN d'un échantillon obtenu du sujet ;
(b) soumission dudit ADN à une modification de séquence sensible à la méthylation par incubation avec des produits chimiques (par exemple, bisulfite de sodium), ou mise en contact dudit ADN avec un polypeptide capable de se lier à l'ADN méthylé ;
(c) facultativement amplification dudit ADN modifié avec du bisulfite de sodium ou lié par ledit polypeptide ; et
(d) détermination de l'état de méthylation dudit ADN.

**3.** Procédé *in vitro* selon l'une quelconque des revendications 1 ou 2 dans lequel la détermination de l'état d'acétylation comprend les étape suivantes :

(a) isolement d'ADN d'un échantillon obtenu du sujet ;
(b) facultativement amplification dudit ADN ; et
(c) détermination de l'état d'acétylation dudit ADN.

**4.** Kit de réalisation du procédé selon l'une quelconque des revendications 1 à 3, comprenant un produit chimique capable de modifier de manière différentielle une séquence d'ADN en fonction d'un état de méthylation, ou un polypeptide ou un agent capable de se lier à l'ADN méthylé et/ou acétylé, et des anticorps, des sondes, des amorces et/ou des aptamères spécifiques des régions telles que définies dans la revendication 1.

**5.** Utilisation du kit selon la revendication 4 pour le diagnostic d'une leucémie promyélocytaire aiguë résistante et/ou la prédiction et/ou la surveillance de la réponse et/ou de l'efficacité d'une thérapie ou d'identification d'un sujet à traiter avec un inhibiteur de la HAT et/ou un inhibiteur d'EZH2.

**6.** Utilisation d'une micropuce capable de déterminer l'état d'acétylation dans toutes les régions pertinentes d'acétylation telles que définies dans la revendication 1 et l'état de méthylation de toutes les régions pertinentes de méthylation CpG telles que définies dans la revendication 1 ladite micropuce comprenant des sondes, des amorces et/ou des aptamères spécifiques des régions telles que définies dans la revendication 1 pour la réalisation du procédé selon l'une quelconque des revendications 1 à 3.

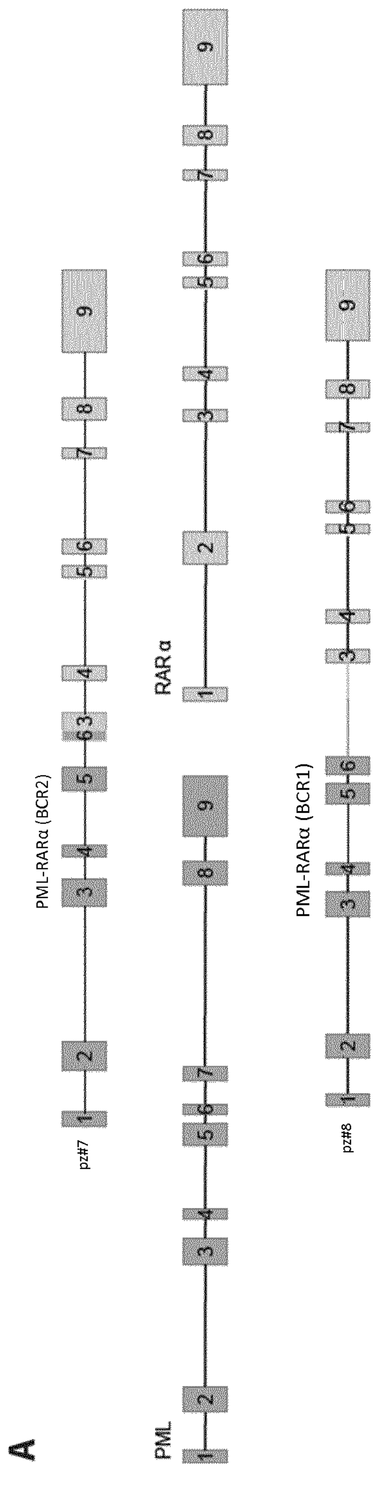


Fig. 1 (1/3)

B

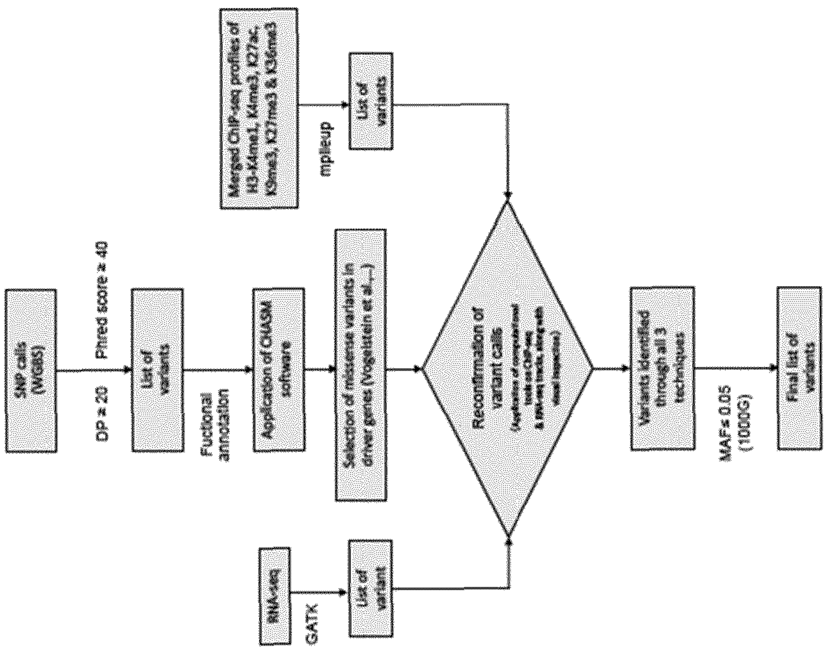


C

| Gene | Sample | MAF (1000G) | Zygosity |
|---|---|---|---|
| TET2 | #7 | 0 | Heterozygous |
| BRCA2 | #7 | 0 | Heterozygous |
| AXIN1 | #7 | 0 | Heterozygous |
| TRAF7 | #8 | 0 | Heterozygous |
| ARID1B | #8 | 0 | Heterozygous |

D

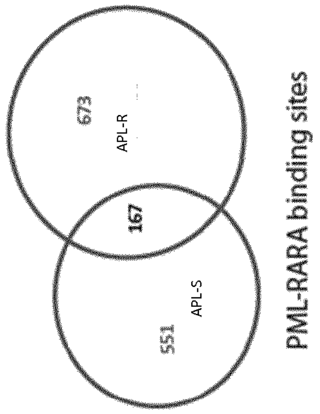


Fig. 1 (2/3)

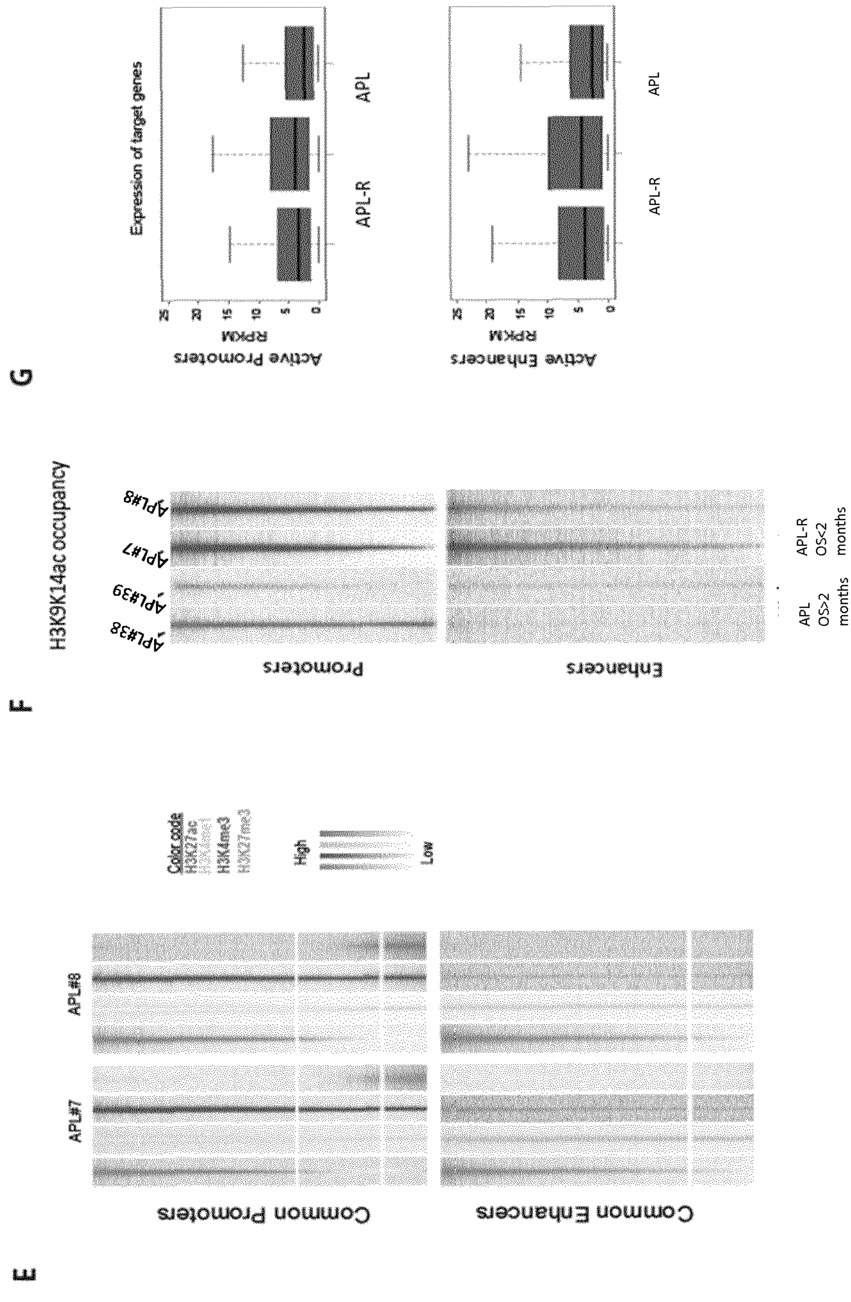
E
Common Promoters
Common Enhancers
APL#7
APL#8
Color code
H3K27ac
H3K4me1
H3K4me3
H3K27me3
High
Low
F
H3K9K14ac occupancy
Promoters
Enhancers
APL#38
APL#39
APL#7
APL#8
APL
OS>2
months
APL-R
OS<2
months
G
Expression of target genes
Active Promoters
RPKM
Active Enhancers
RPKM
25
20
15
10
5
0
APL-R
APL

Fig. 1 (3/3)

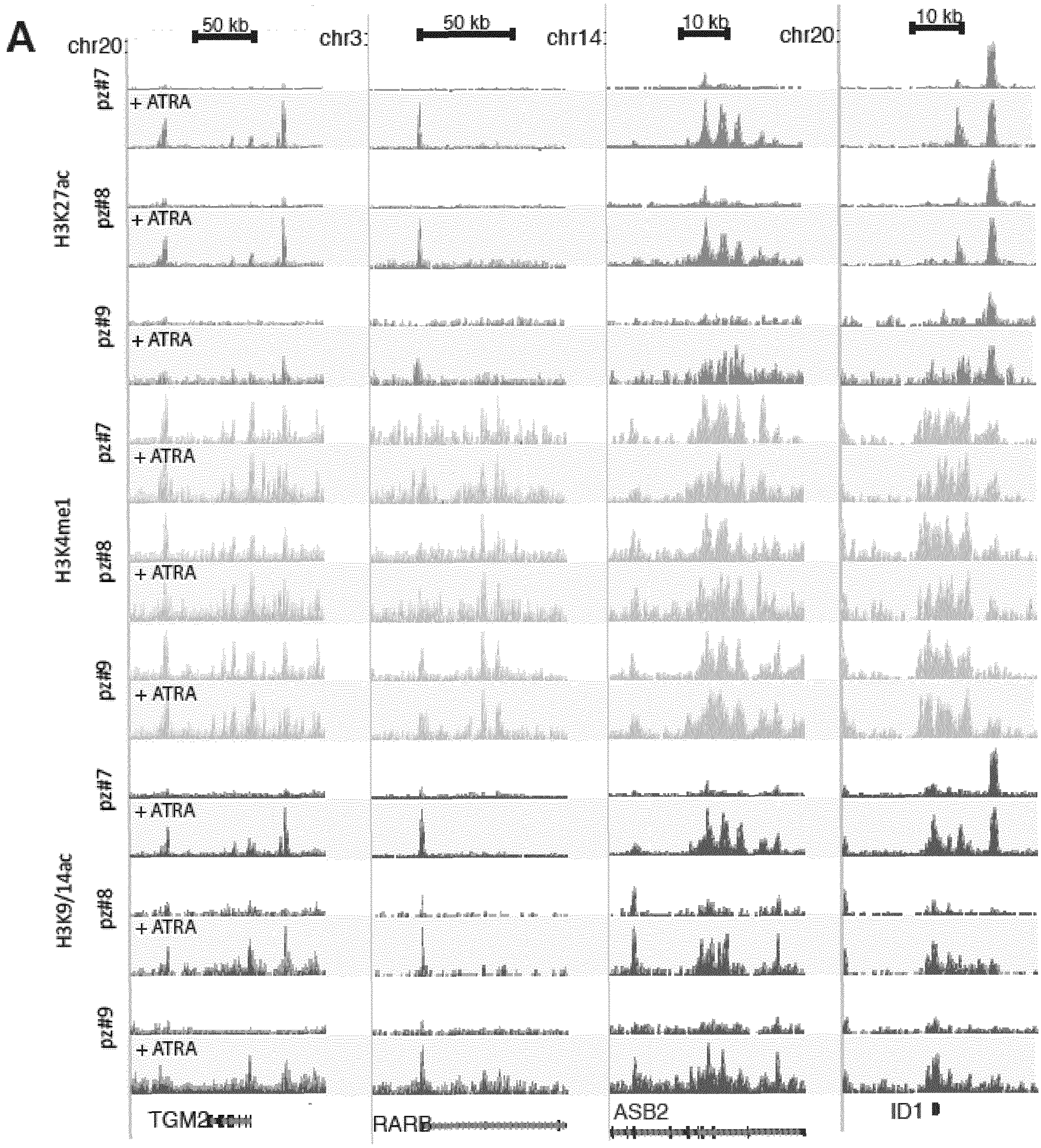
A
chr20
50 kb
chr3
50 kb
chr14
10 kb
chr20
10 kb
H3K27ac
pz#7
+ ATRA
pz#8
+ ATRA
pz#9
+ ATRA
H3K4me1
pz#7
+ ATRA
pz#8
+ ATRA
pz#9
+ ATRA
H3K9/14ac
pz#7
+ ATRA
pz#8
+ ATRA
pz#9
+ ATRA
TGM2
RARB
ASB2
ID1

Fig. 2 (1/4)

B

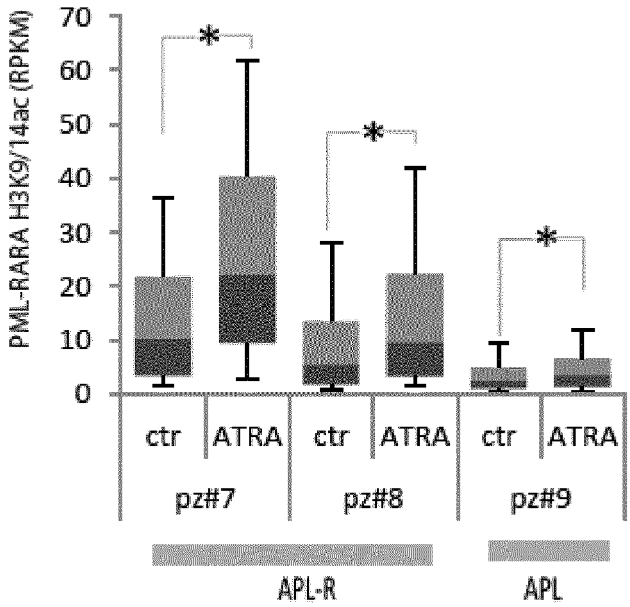
PML-RARA H3K9/14ac (RPKM)
70
60
50
40
30
20
10
0
*
*
*
ctr
ATRA
ctr
ATRA
ctr
ATRA
pz#7
pz#8
pz#9
APL-R
APL

C

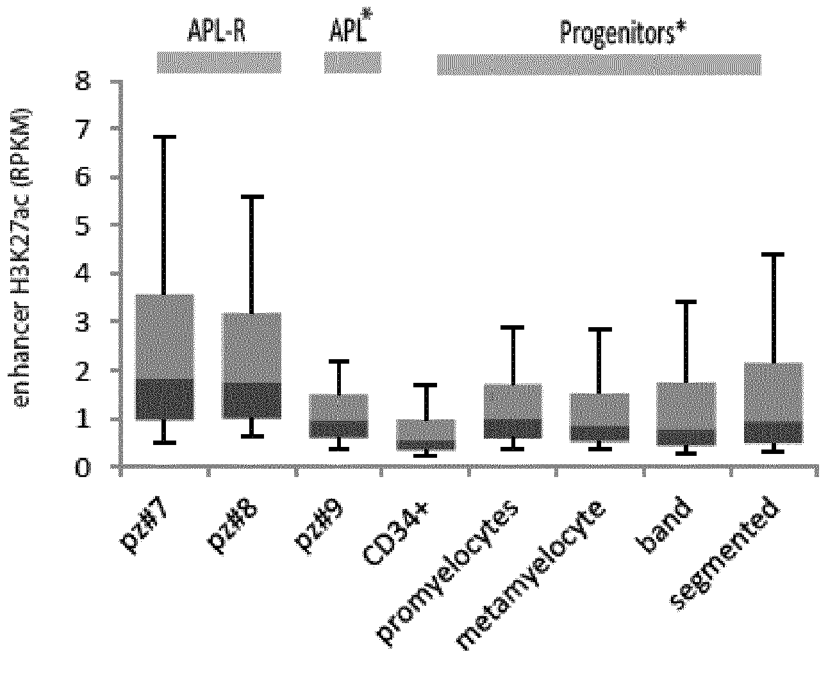
APL-R
APL*
Progenitors*
enhancer H3K27ac (RPKM)
8
7
6
5
4
3
2
1
0
pz#7
pz#8
pz#9
CD34+
promyelocytes
metamyelocyte
band
segmented

Fig. 2 (2/4)

D

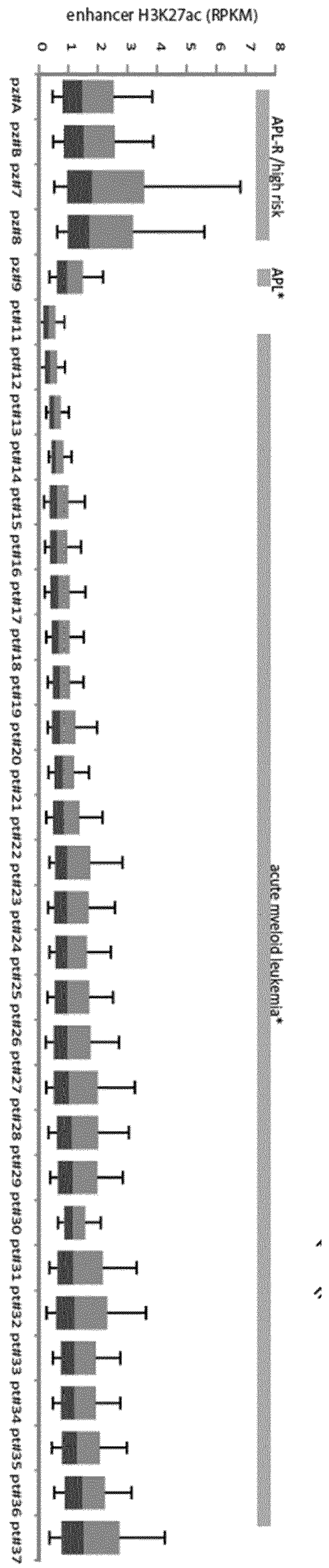
enhancer H3K27ac (RPKM)
0
1
2
3
4
5
6
7
8
APL-R /high risk
APL*
acute myeloid leukemia*
pz#A pz#B pz#7 pz#8 pz#9 pt#11 pt#12 pt#13 pt#14 pt#15 pt#16 pt#17 pt#18 pt#19 pt#20 pt#21 pt#22 pt#23 pt#24 pt#25 pt#26 pt#27 pt#28 pt#29 pt#30 pt#31 pt#32 pt#33 pt#34 pt#35 pt#36 pt#37

Fig. 2 (3/4)

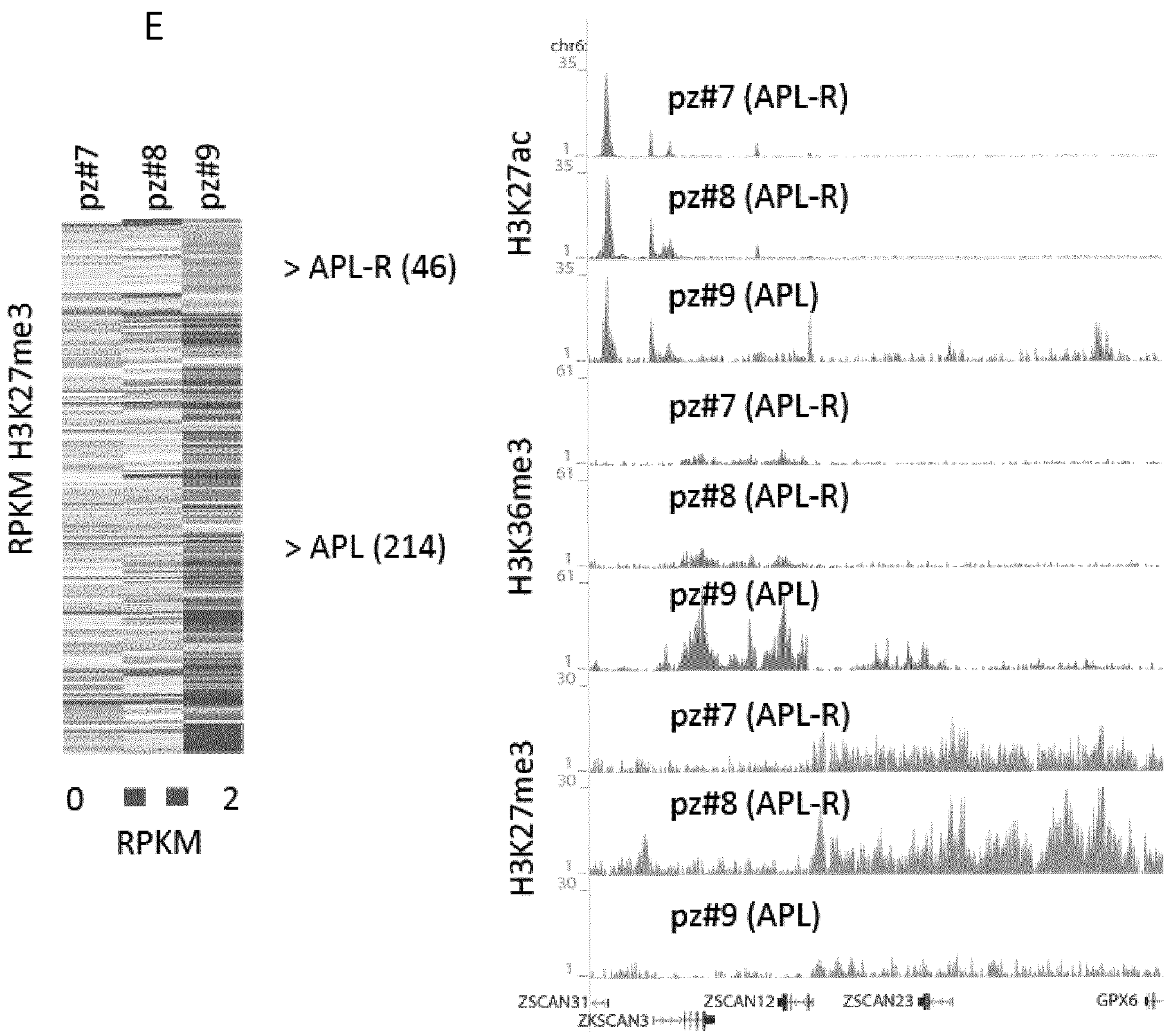
E
pz#7
pz#8
pz#9
> APL-R (46)
> APL (214)
RPKM H3K27me3
0
2
RPKM
chr6:
H3K27ac
pz#7 (APL-R)
pz#8 (APL-R)
pz#9 (APL)
H3K36me3
pz#7 (APL-R)
pz#8 (APL-R)
pz#9 (APL)
H3K27me3
pz#7 (APL-R)
pz#8 (APL-R)
pz#9 (APL)
ZSCAN31
ZKSCAN3
ZSCAN12
ZSCAN23
GPX6

Fig. 2 (4/4)

A

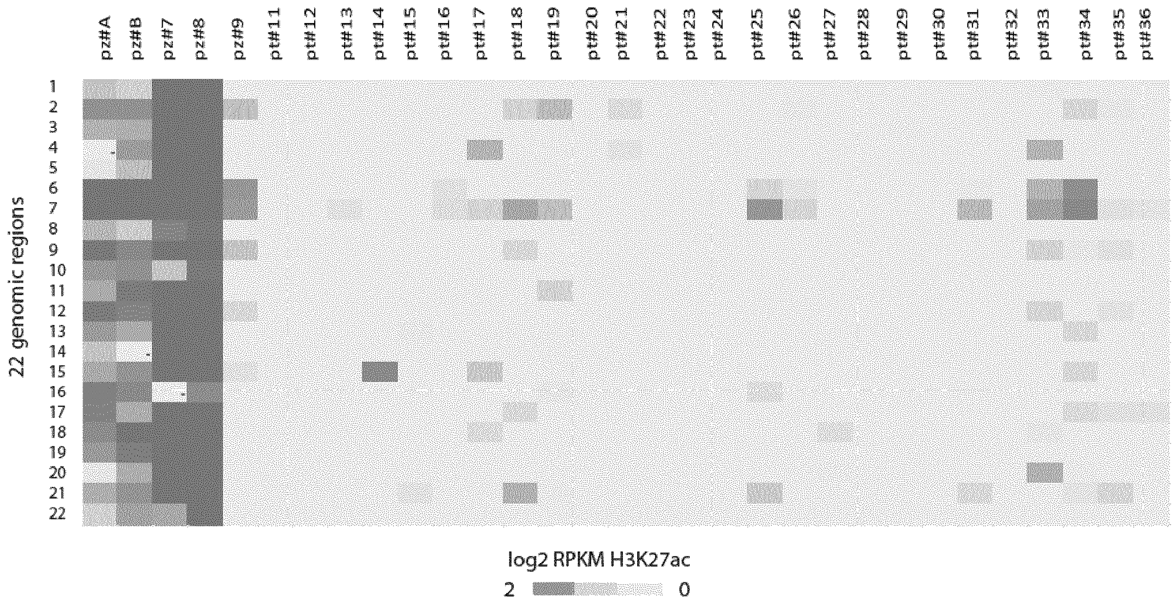
pz#A
pz#B
pz#7
pz#8
pz#9
pt#11
pt#12
pt#13
pt#14
pt#15
pt#16
pt#17
pt#18
pt#19
pt#20
pt#21
pt#22
pt#23
pt#24
pt#25
pt#26
pt#27
pt#28
pt#29
pt#30
pt#31
pt#32
pt#33
pt#34
pt#35
pt#36
22 genomic regions
1
2
3
4
5
6
7
8
9
10
11
12
13
14
15
16
17
18
19
20
21
22
log2 RPKM H3K27ac
2
0

B

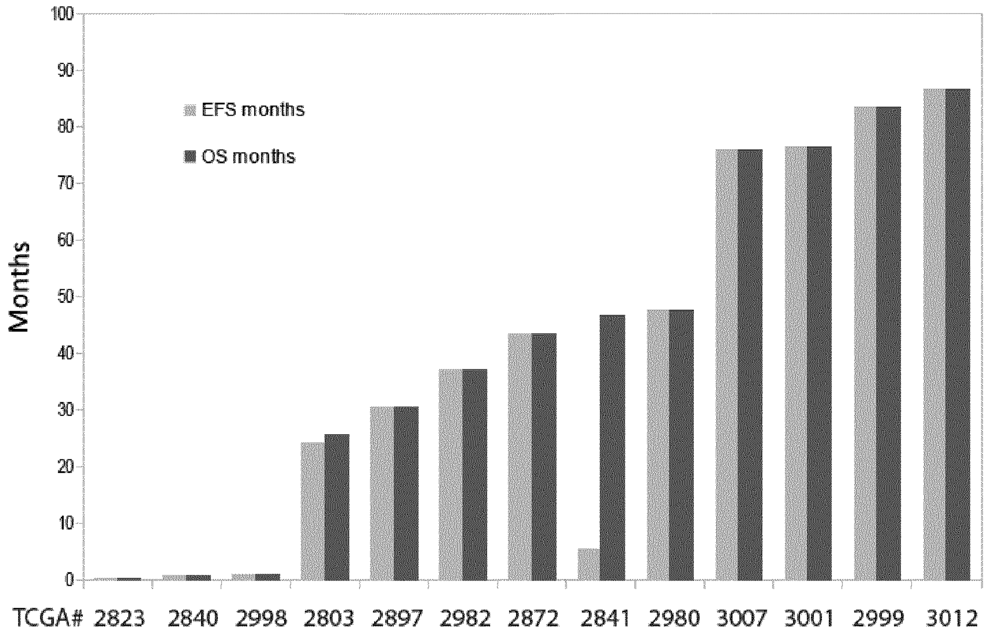
EFS months
OS months
Months
100
90
80
70
60
50
40
30
20
10
0
TCGA#
2823
2840
2998
2803
2897
2982
2872
2841
2980
3007
3001
2999
3012

Fig. 3 (1/2)

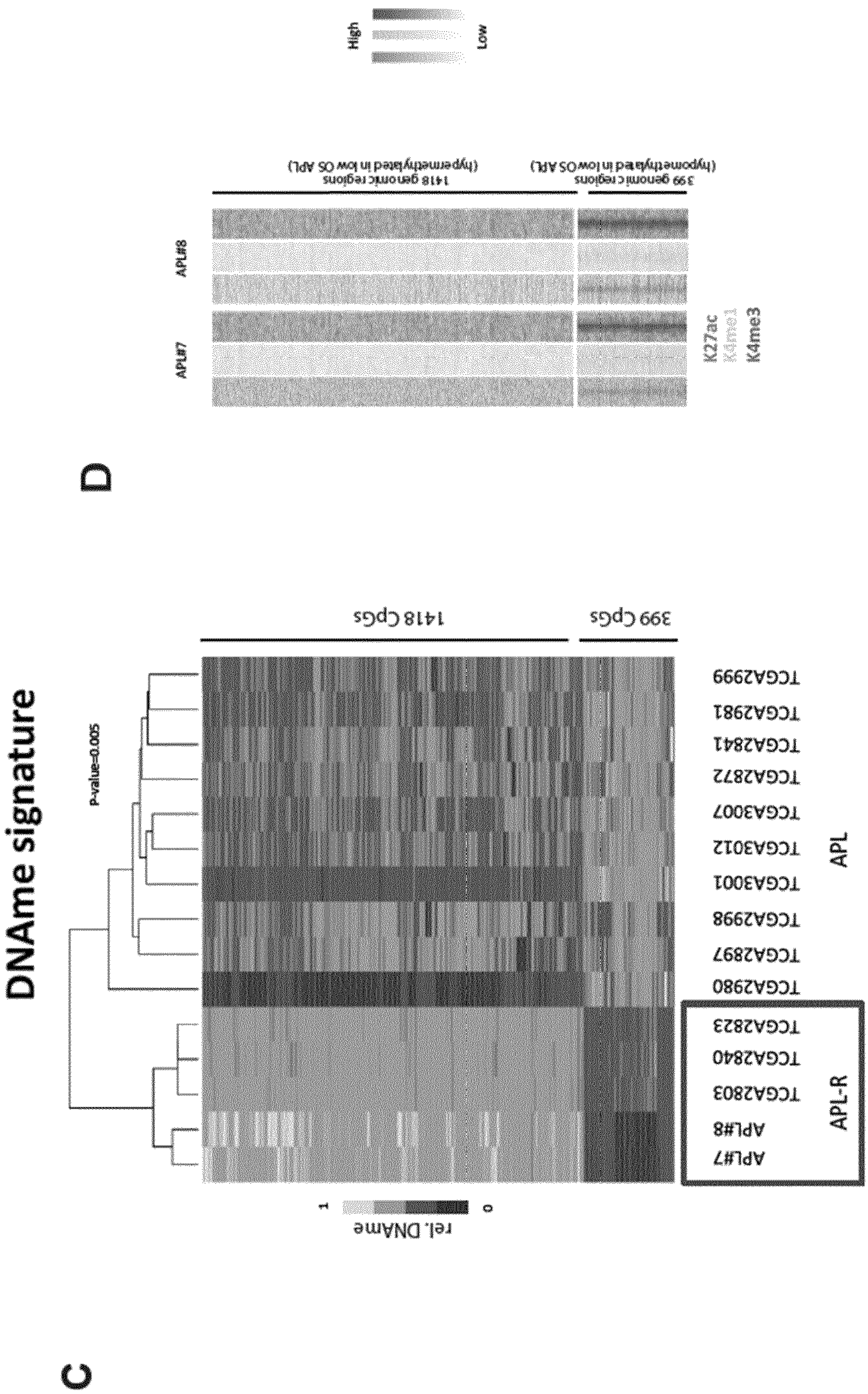

High
Low
D
APL#7
APL#8
K27ac
K4me1
K4me3
1418 genomic regions (hypermethylated in low OS APL)
399 genomic regions (hypomethylated in low OS APL)
C
DNAme signature
P-value=0.005
1418 CpGs
399 CpGs
TCGA2999
TCGA2981
TCGA2841
TCGA2872
TCGA3007
TCGA3012
TCGA3001
TCGA2998
TCGA2897
TCGA2980
TCGA2823
TCGA2840
TCGA2803
APL#8
APL#7
APL
APL-R
rel. DNAme
1
0


Fig. 3 (2/2)

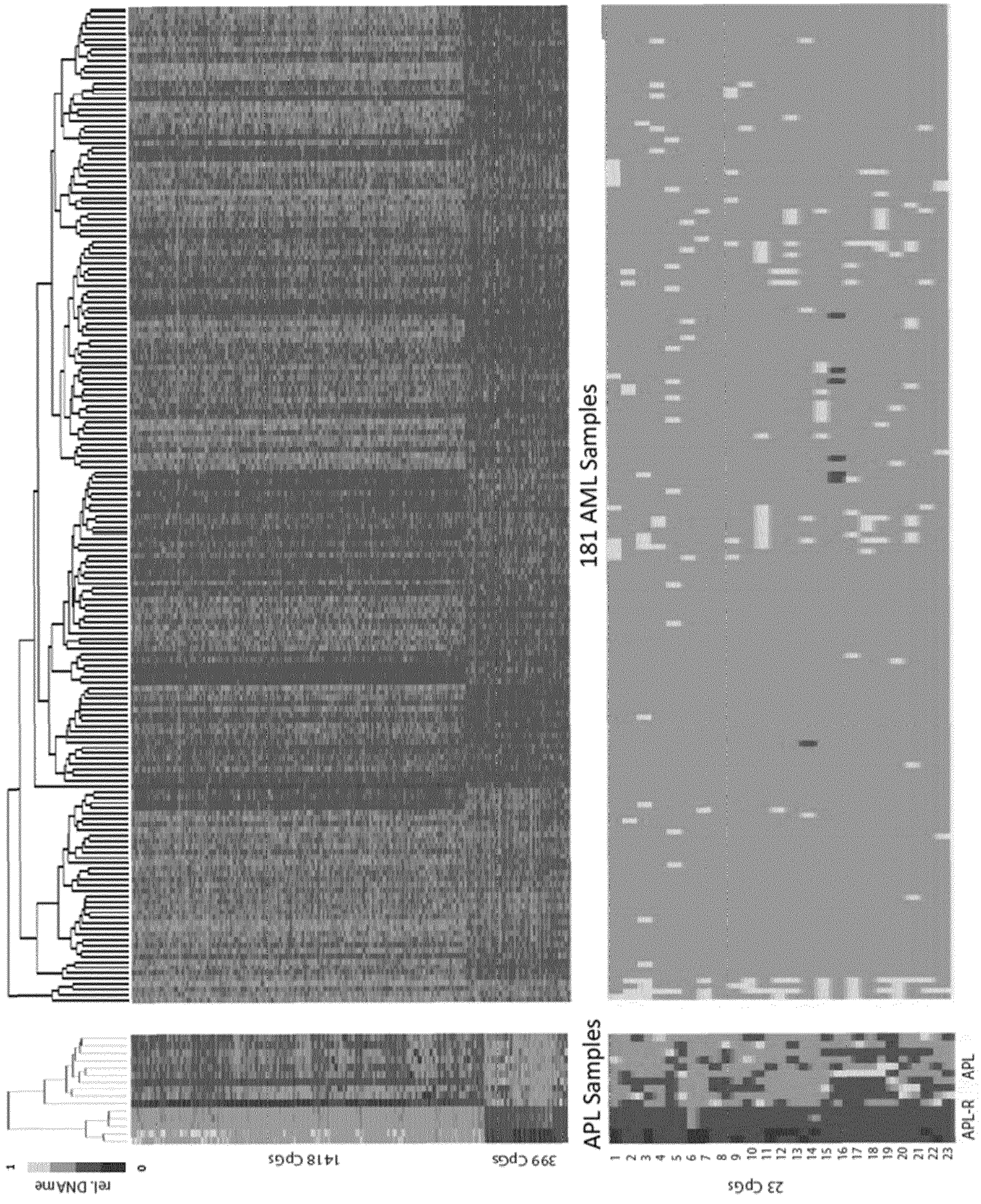
181 AML Samples
APL Samples
APL
APL-R
1418 CpGs
399 CpGs
23 CpGs
rel. DNAme
0
1
1 2 3 4 5 6 7 8 9 10 11 12 13 14 15 16 17 18 19 20 21 22 23

Fig. 4

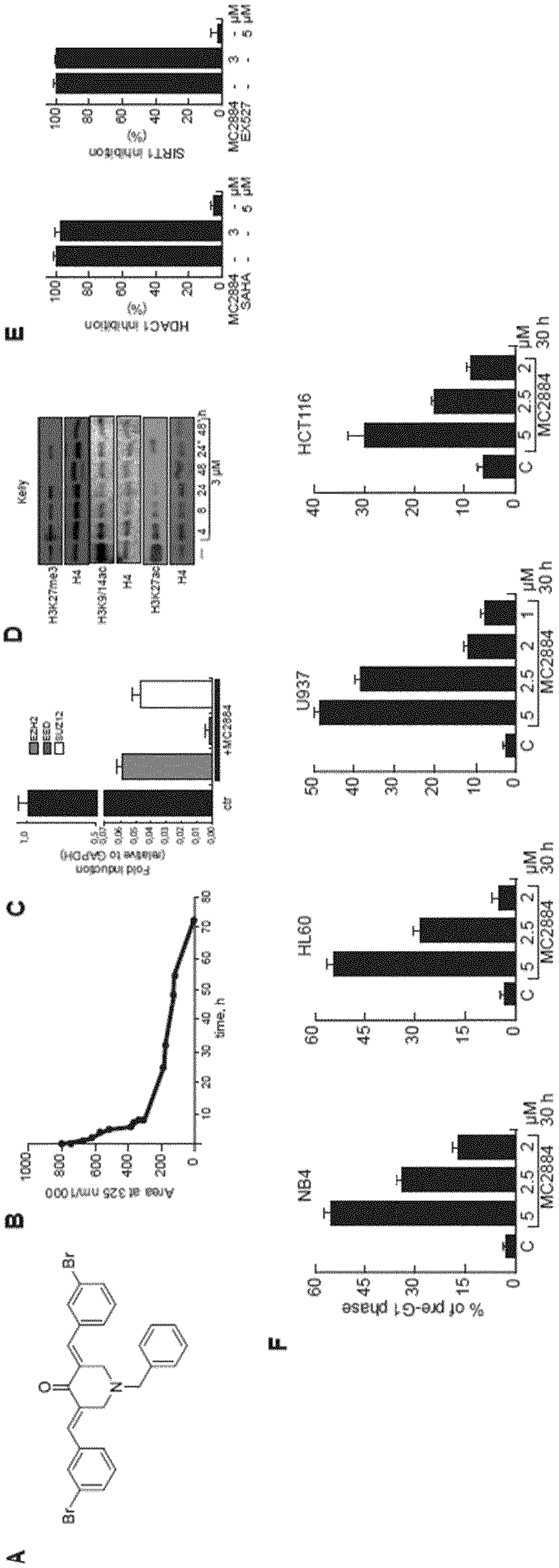
A
B
C
D
E
F
Br
Br
O
N
Area at 325 nm/1000
time, h
Fold induction (relative to GAPDH)
EZH2
EED
SUZ12
ctr
+MC2884
Kelly
H3K27me3
H4
H3K9/14ac
H4
H3K27ac
H4
3 μM
HDAC1 inhibition (%)
MC2884
SAHA
SIRT1 inhibition (%)
MC2884
EX527
% of pre-G1 phase
NB4
HL60
U937
HCT116
MC2884
μM
30 h

Fig. 5 (1/2)

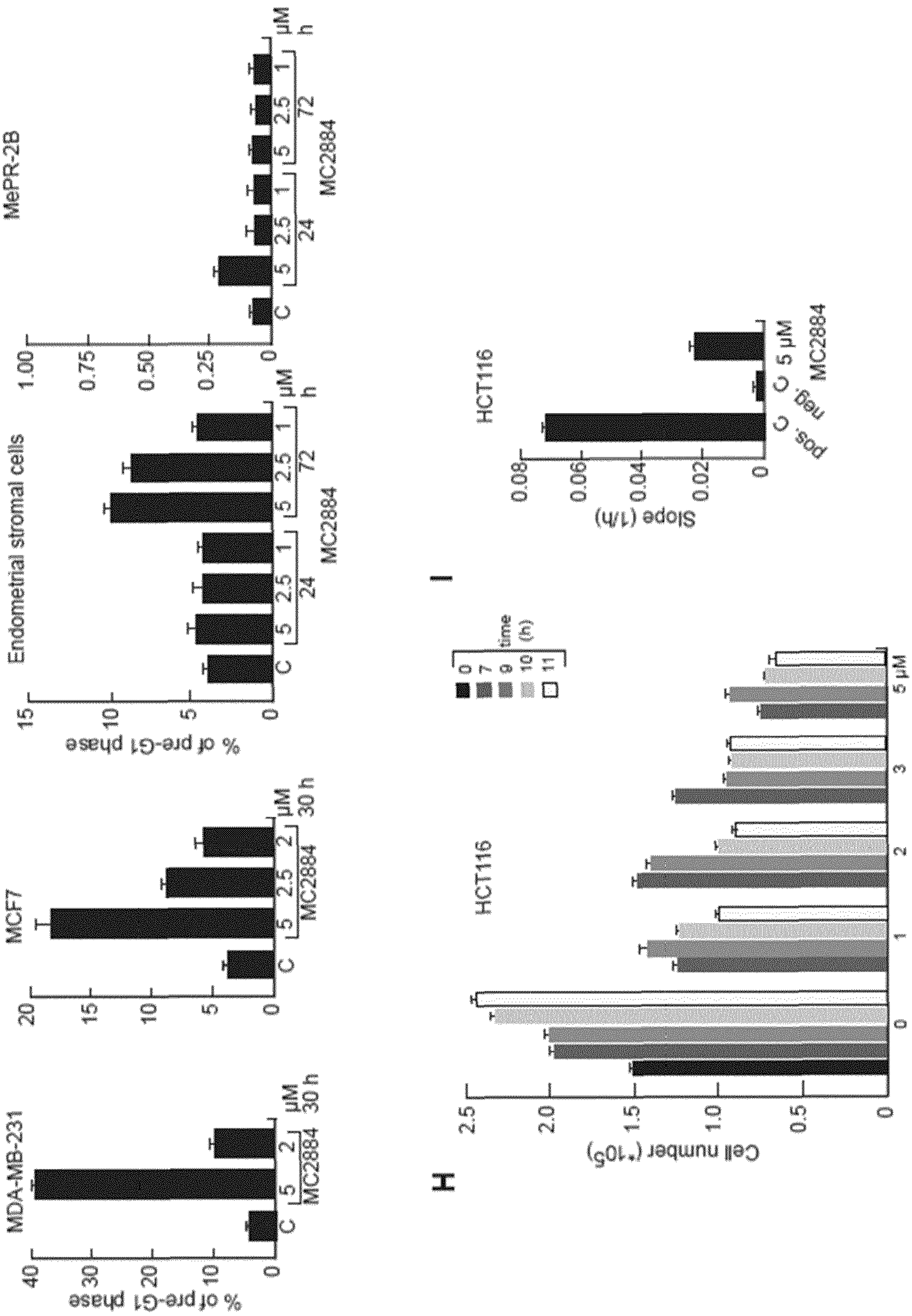
G
MDA-MB-231
% of pre-G1 phase
C
5
2
MC2884
μM
30 h
MCF7
C
5
2.5
2
MC2884
μM
30 h
Endometrial stromal cells
% of pre-G1 phase
C
5
2.5
1
24
72
MC2884
μM
h
MePR-2B
C
5
2.5
1
24
72
MC2884
μM
h
H
HCT116
Cell number (*10⁵)
time (h)
0
7
9
10
11
0
1
2
3
5 μM
MC2884
I
HCT116
Slope (1/h)
pos. C
neg. C
5 μM
MC2884

Fig. 5 (2/2)

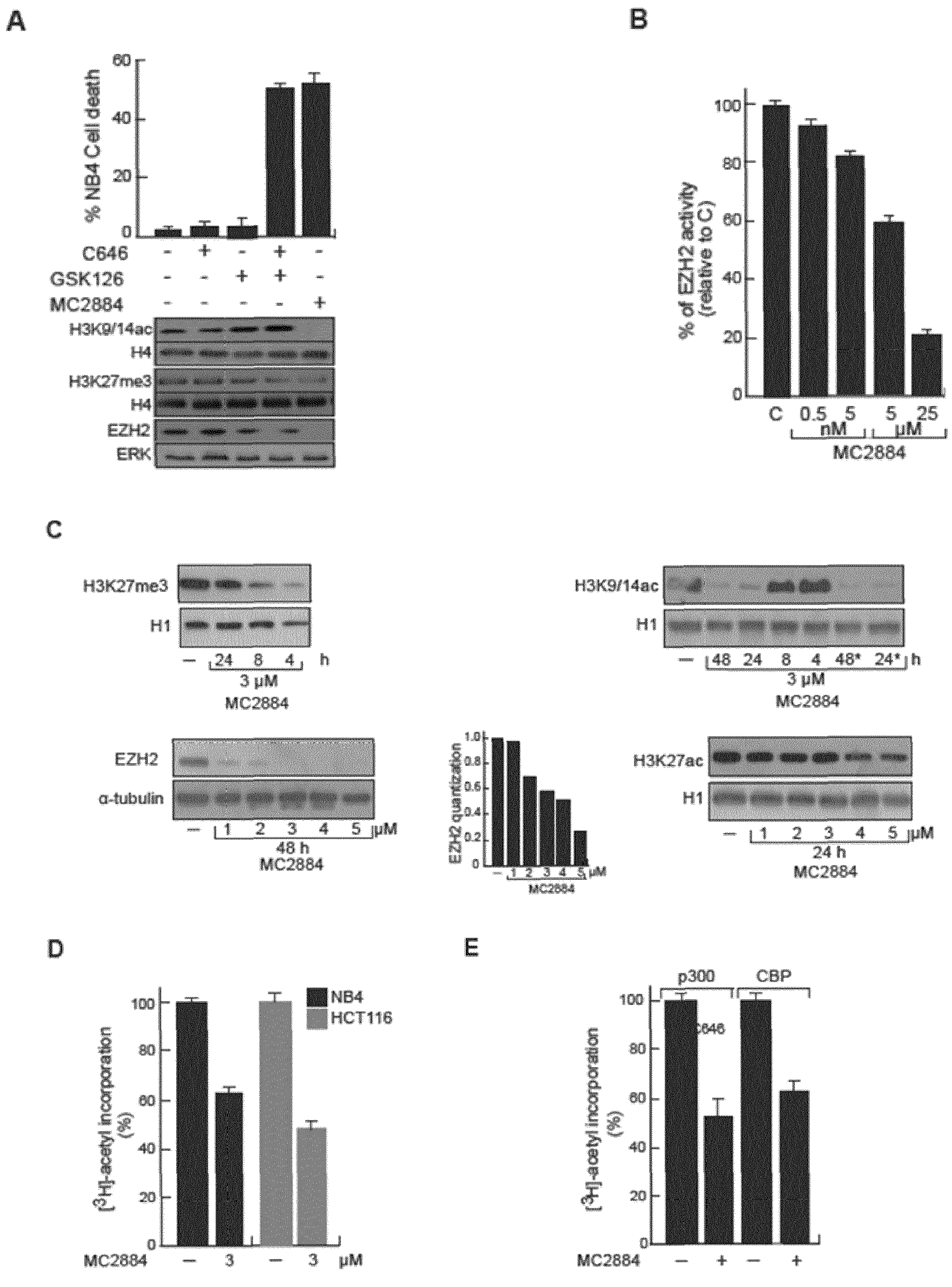
A
% NB4 Cell death
60
40
20
0
C646
GSK126
MC2884
H3K9/14ac
H4
H3K27me3
H4
EZH2
ERK
B
% of EZH2 activity (relative to C)
100
80
60
40
20
0
C
0.5
5
5
25
nM
μM
MC2884
C
H3K27me3
H1
24
8
4
h
3 μM
MC2884
H3K9/14ac
H1
48
24
8
4
48*
24*
h
3 μM
MC2884
EZH2
α-tubulin
1
2
3
4
5
μM
48 h
MC2884
EZH2 quantization
H3K27ac
H1
1
2
3
4
5
μM
24 h
MC2884
D
[3H]-acetyl incorporation (%)
NB4
HCT116
MC2884
3
3
μM
E
p300
CBP
[3H]-acetyl incorporation (%)
MC2884

Fig. 6

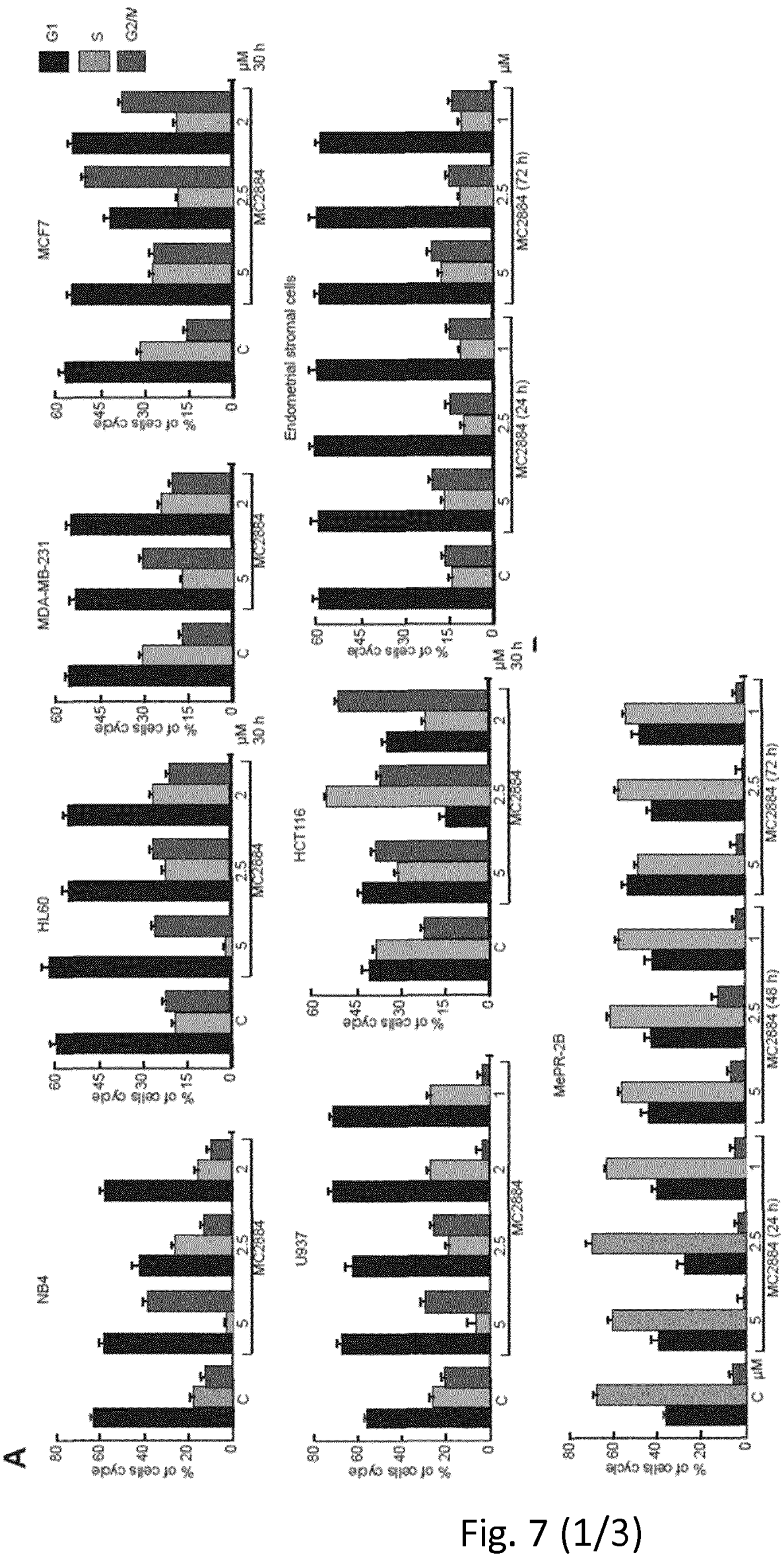
A
NB4
HL60
MDA-MB-231
MCF7
U937
HCT116
Endometrial stromal cells
MePR-2B
G1
S
G2/M
% of cells cycle
MC2884
MC2884 (24 h)
MC2884 (48 h)
MC2884 (72 h)
µM
30 h

Fig. 7 (1/3)

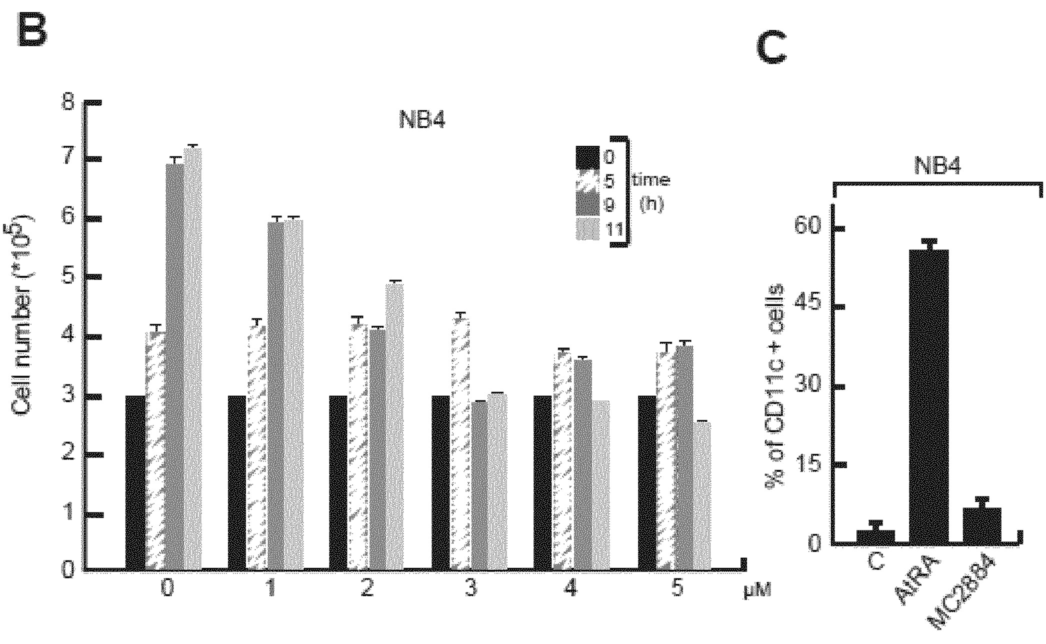
B
NB4
0
5
9
11
time
(h)
Cell number (*10^5)
8
7
6
5
4
3
2
1
0
0
1
2
3
4
5
µM
C
NB4
% of CD11c + cells
60
45
30
15
0
C
ATRA
MC2884

Fig. 7 (2/3)

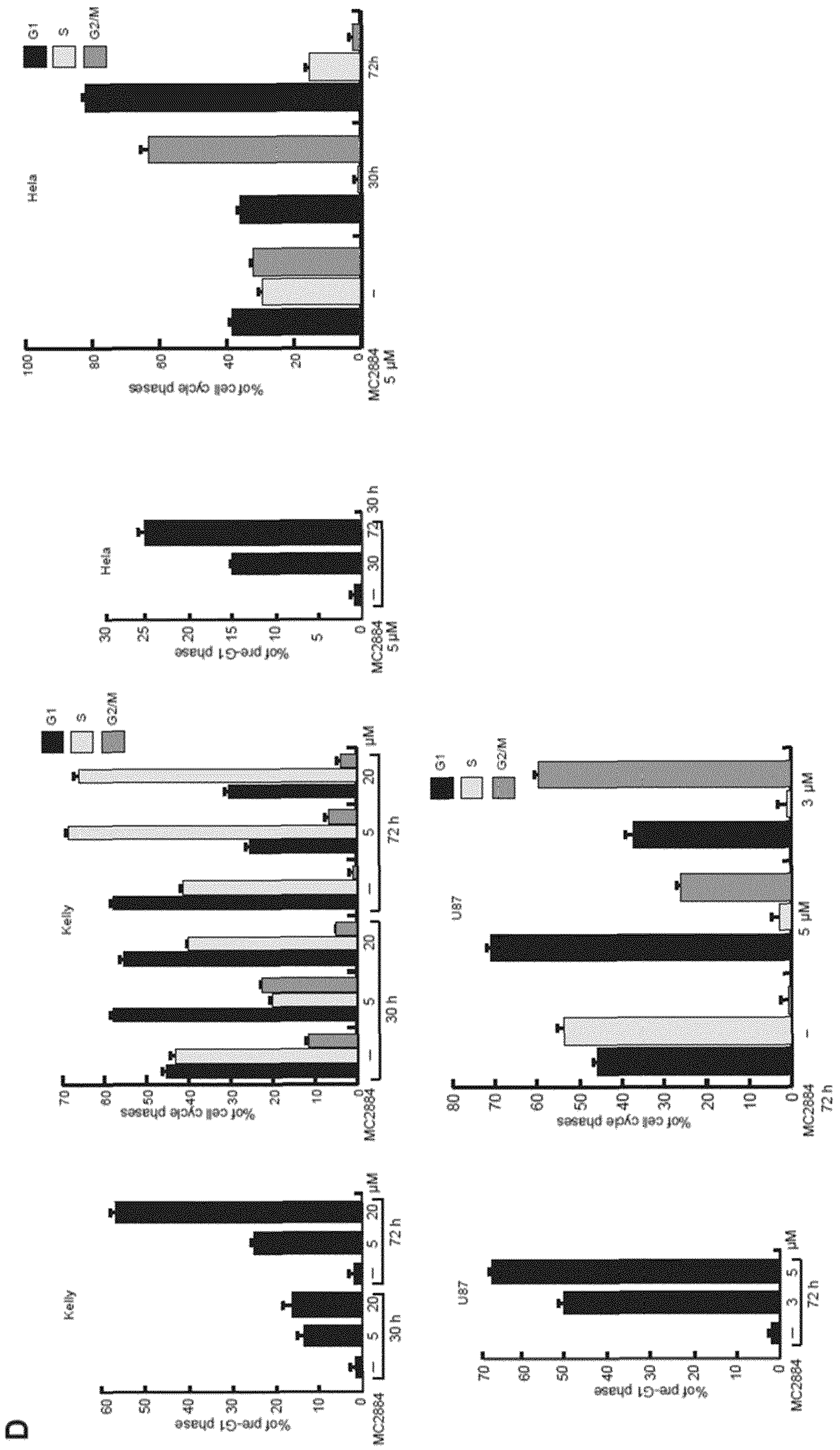
D
Kelly
Hela
U87
G1
S
G2/M
%of pre-G1 phase
%of cell cycle phases
MC2884
5 µM
30 h
72 h
µM

Fig. 7 (3/3)

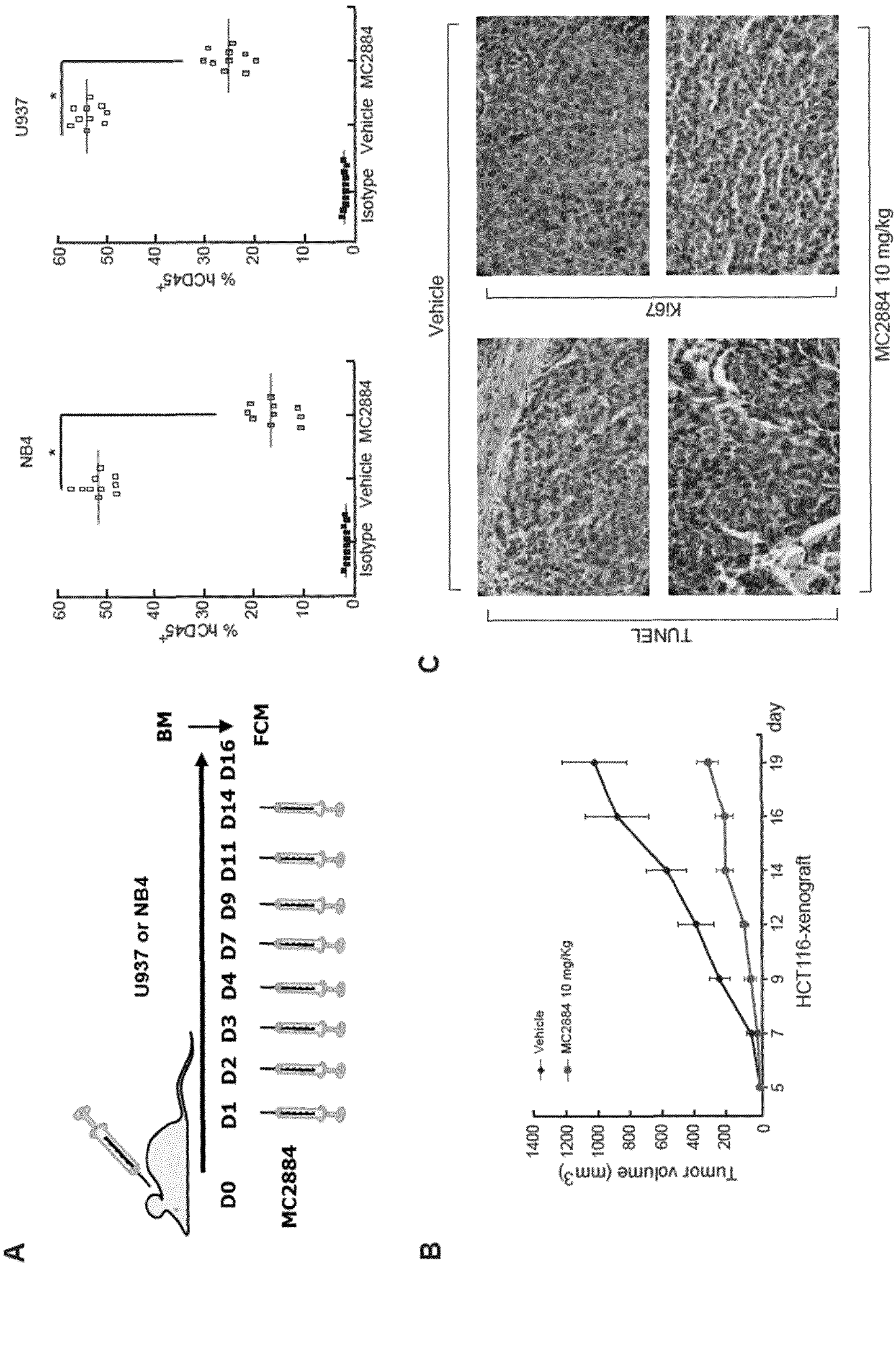
A
U937 or NB4
D0 D1 D2 D3 D4 D7 D9 D11 D14 D16
BM
FCM
MC2884
NB4
U937
% hCD45+
60 50 40 30 20 10 0
*
Isotype Vehicle MC2884
B
Tumor volume (mm3)
1400 1200 1000 800 600 400 200 0
Vehicle
MC2884 10 mg/Kg
5 7 9 12 14 16 19
day
HCT116-xenograft
C
Vehicle
TUNEL
Ki67
MC2884 10 mg/kg

Fig. 8 (1/2)

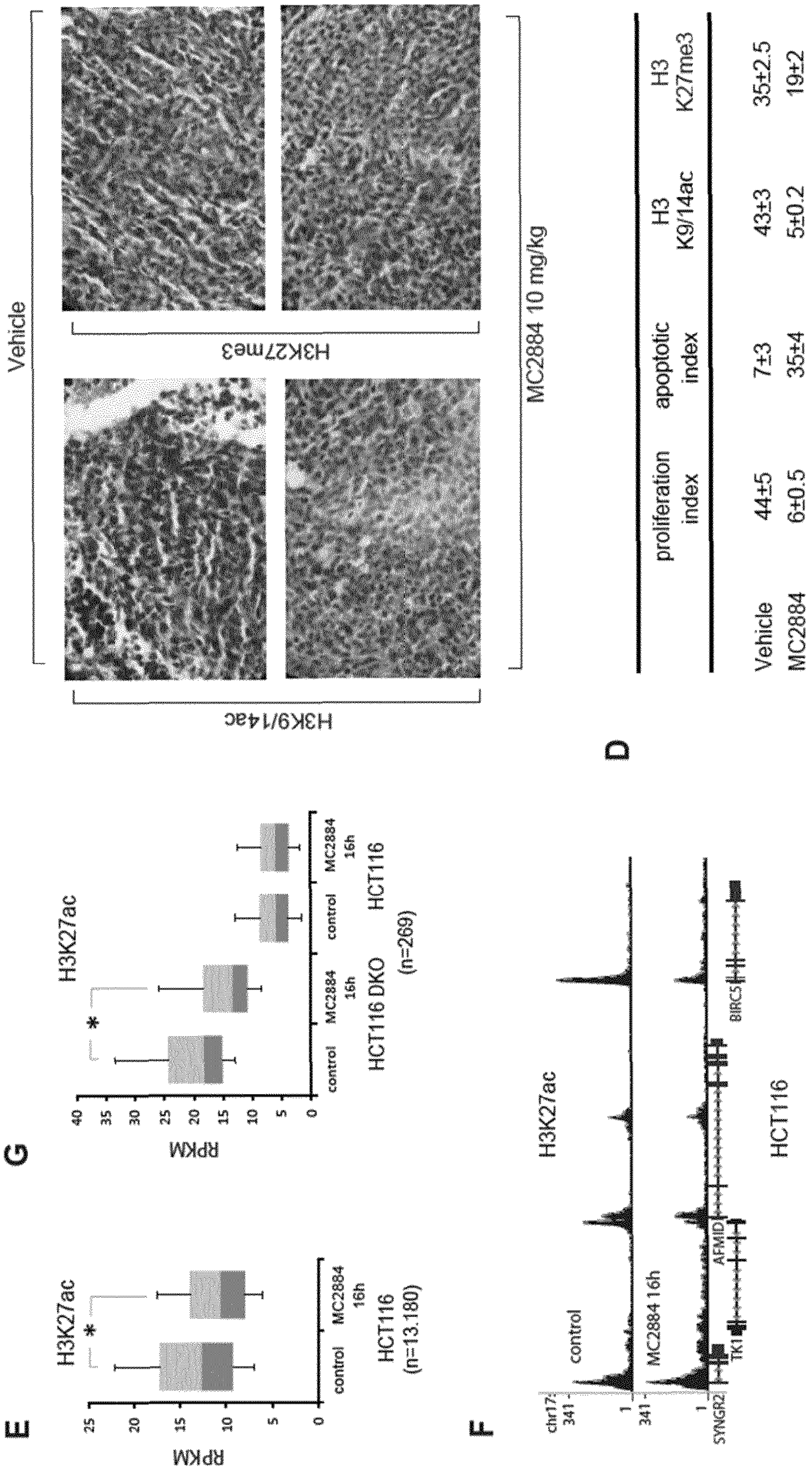
D
Vehicle
MC2884 10 mg/kg
H3K9/14ac
H3K27me3
proliferation index
apoptotic index
H3 K9/14ac
H3 K27me3
Vehicle 44±5 7±3 43±3 35±2.5
MC2884 6±0.5 35±4 5±0.2 19±2
E
H3K27ac
RPKM
control
MC2884 16h
HCT116
(n=13.180)
G
H3K27ac
RPKM
control
MC2884 16h
HCT116 DKO
(n=269)
HCT116
F
chr17
341
1
control
MC2884 16h
H3K27ac
SYNGR2
TK1
AFMID
BIRC5
HCT116

Fig. 8 (2/2)

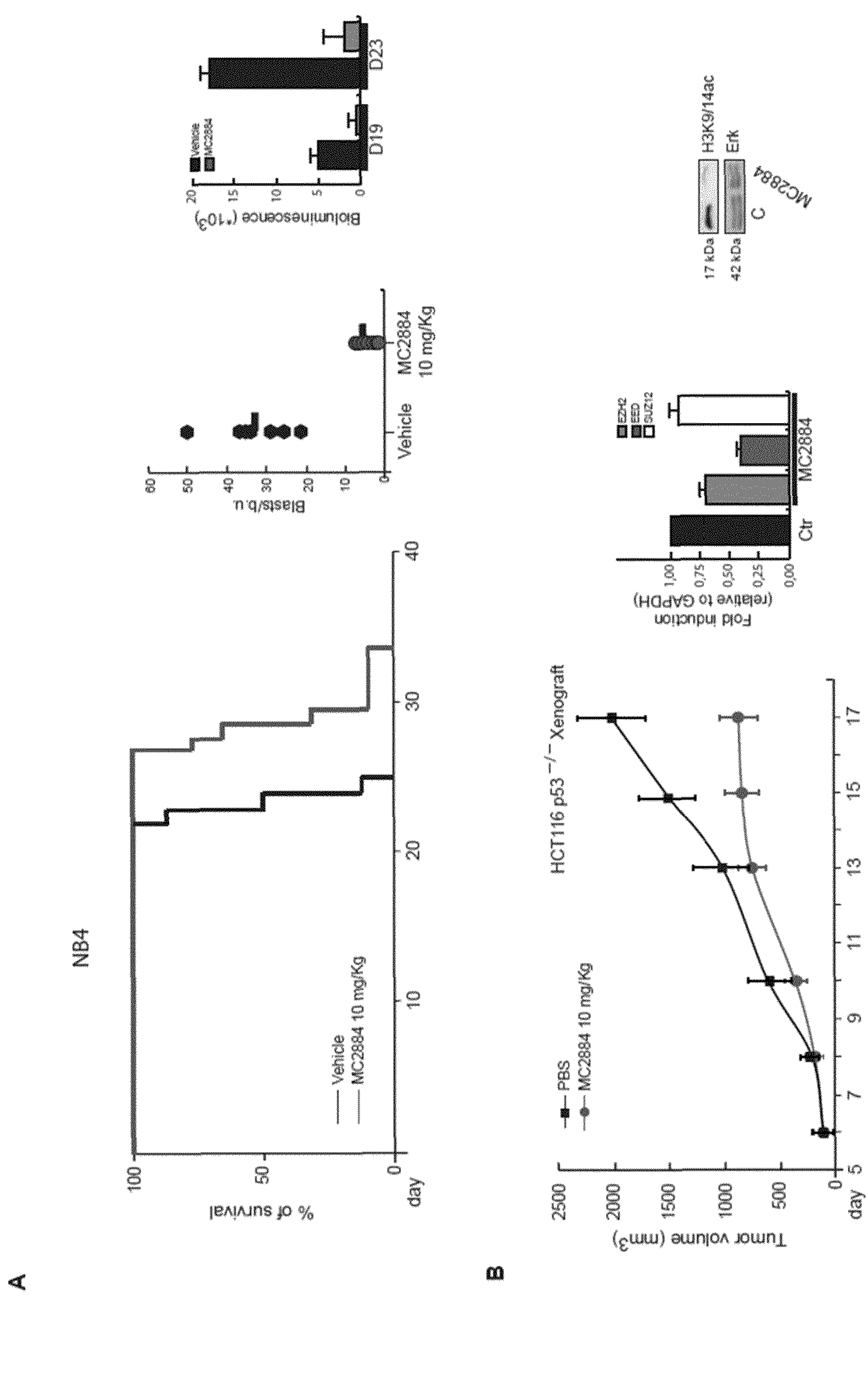
A
NB4
% of survival
day
Vehicle
MC2884 10 mg/Kg
Blasts/b.u.
MC2884 10 mg/Kg
Bioluminescence (*10³)
D19
D23
B
HCT116 p53 −/− Xenograft
Tumor volume (mm³)
PBS
MC2884 10 mg/Kg
Fold induction (relative to GAPDH)
EZH2
EED
SUZ12
Ctr
MC2884
17 kDa
42 kDa
H3K9/14ac
Erk
C
MC2884

Fig. 9 (1/2)

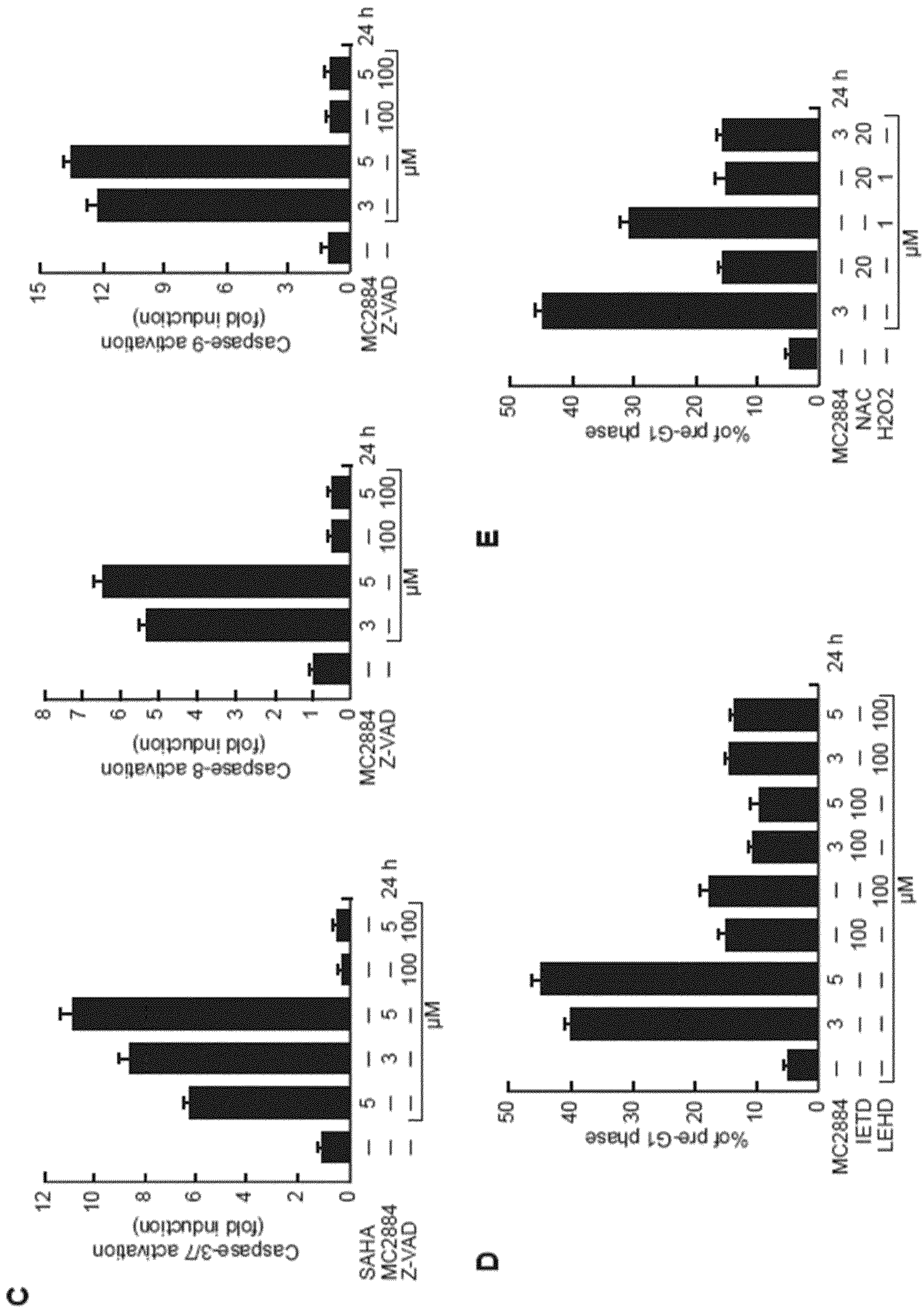
C
Caspase-3/7 activation (fold induction)
SAHA
MC2884
Z-VAD
µM
24 h
Caspase-8 activation (fold induction)
MC2884
Z-VAD
µM
24 h
Caspase-9 activation (fold induction)
MC2884
Z-VAD
µM
24 h
D
%of pre-G1 phase
MC2884
IETD
LEHD
µM
24 h
E
%of pre-G1 phase
MC2884
NAC
H2O2
µM
24 h

Fig. 9 (2/2)

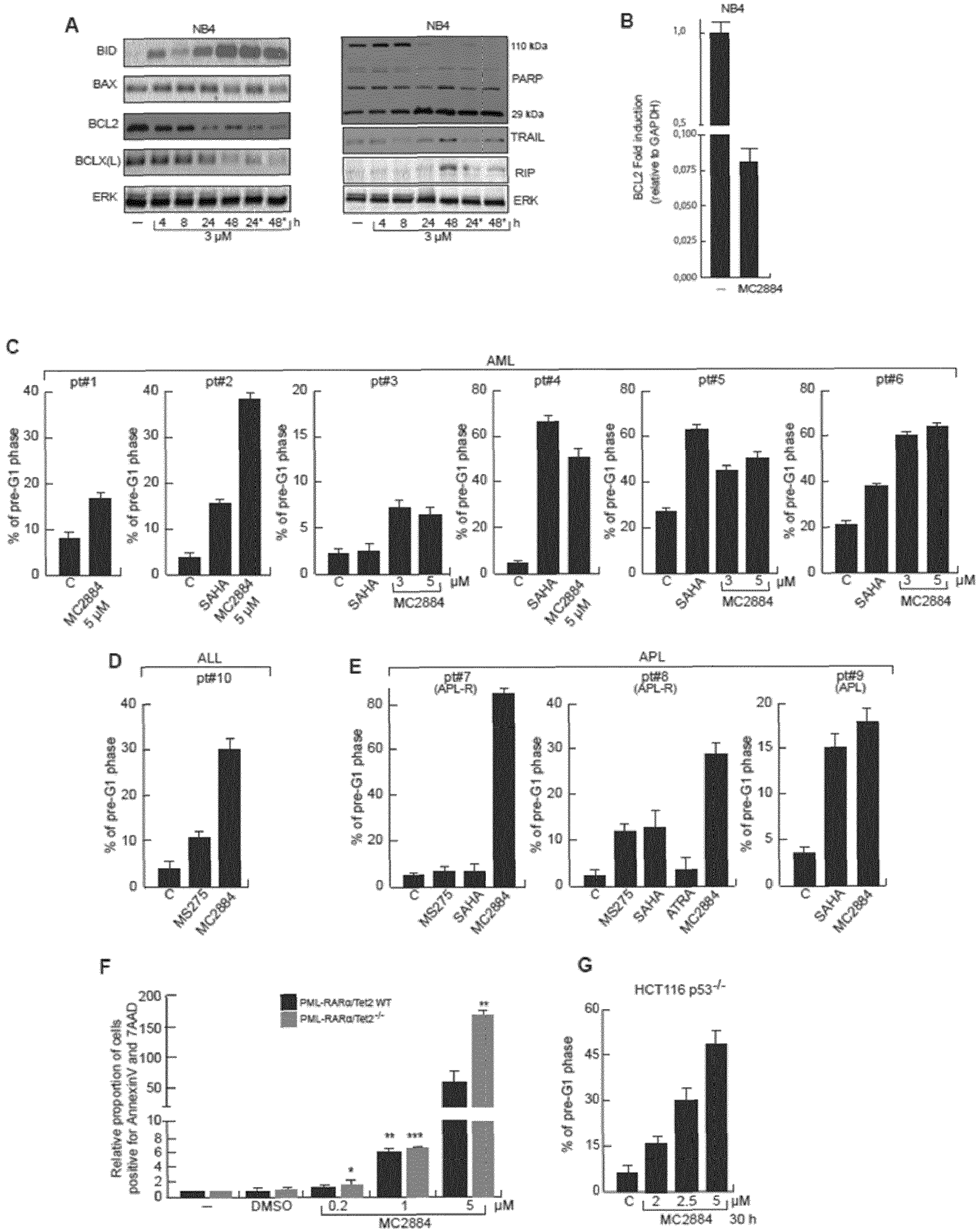
A
NB4
BID
BAX
BCL2
BCLX(L)
ERK
— 4 8 24 48 24* 48* h
3 μM
NB4
110 kDa
PARP
29 kDa
TRAIL
RIP
ERK
— 4 8 24 48 24* 48* h
3 μM
B
NB4
BCL2 Fold induction (relative to GAPDH)
— MC2884
C
AML
pt#1 pt#2 pt#3 pt#4 pt#5 pt#6
% of pre-G1 phase
C MC2884 5 μM
C SAHA MC2884 5 μM
C SAHA 3 5 μM MC2884
D
ALL
pt#10
C MS275 MC2884
E
APL
pt#7 (APL-R)
pt#8 (APL-R)
pt#9 (APL)
C MS275 SAHA MC2884
C MS275 SAHA ATRA MC2884
C SAHA MC2884
F
PML-RARα/Tet2 WT
PML-RARα/Tet2-/-
Relative proportion of cells positive for AnnexinV and 7AAD
— DMSO 0.2 1 5 μM
MC2884
G
HCT116 p53-/-
% of pre-G1 phase
C 2 2.5 5 μM
MC2884 30 h

Fig. 10

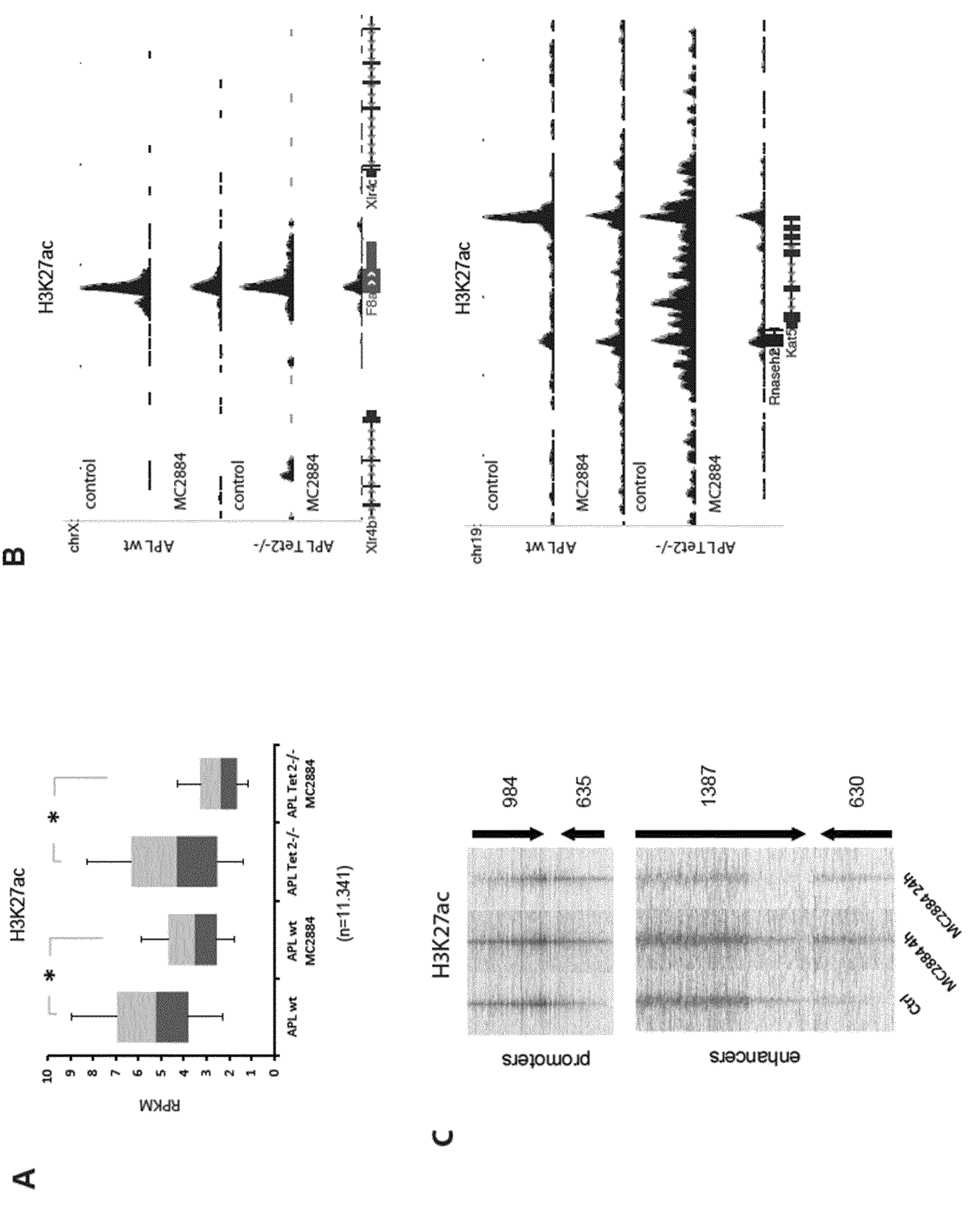
A
H3K27ac
RPKM
10
9
8
7
6
5
4
3
2
1
0
*
*
APL wt
APL wt MC2884
APL Tet 2-/-
APL Tet 2-/- MC2884
(n=11.341)
B
H3K27ac
chrX
APL wt
APL Tet2-/-
control
MC2884
control
MC2884
Xlr4b
F8a
Xlr4c
H3K27ac
chr19
APL wt
APL Tet2-/-
control
MC2884
control
MC2884
Rnaseh2
Kat5
C
H3K27ac
promoters
enhancers
984
635
1387
630
Ctrl
MC2884 4h
MC2884 24h

Fig. 11 (1/2)

E

D

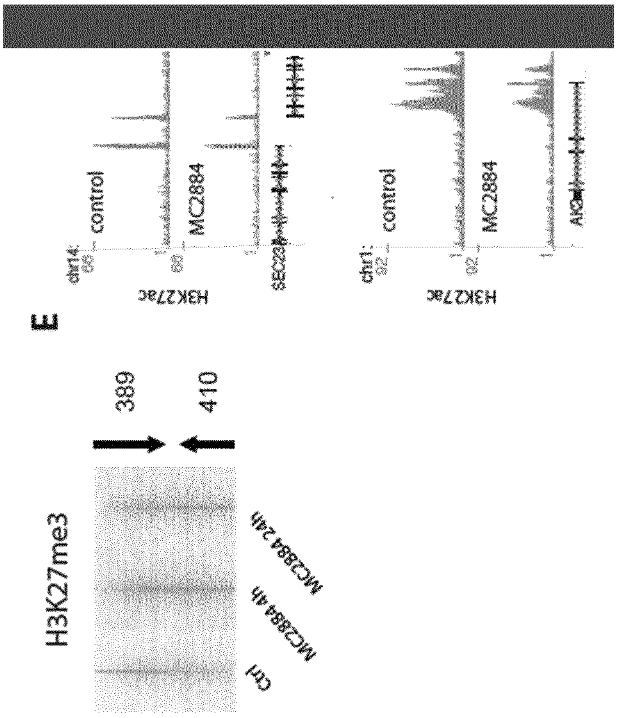


F

| Term | p-value | FDR Q-value |
|---|---|---|
| Genes with promoters occupied by PML-RARA | 4.44E-21 | 4.98E-18 |
| acute myeloid leukemia | 1.66E-12 | 2.85E-10 |
| JAK/STAT signaling pathway | 0.000016158 | 0.001228 |
| Toll receptor signaling pathway | 0.000016939 | 0.00085825 |
| Ras pathway | 0.00039575 | 0.010026 |

Fig. 11 (2/2)

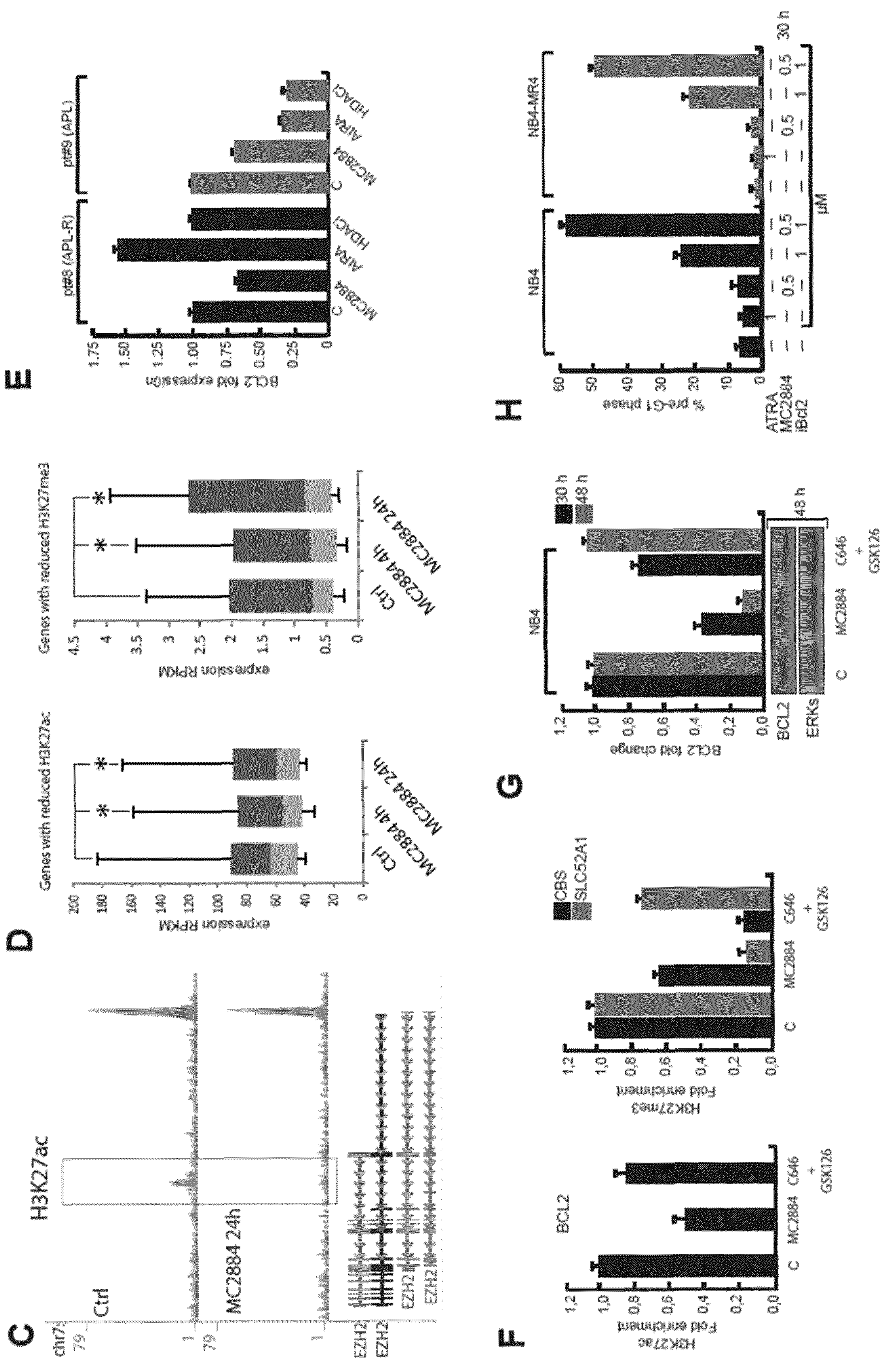
C
H3K27ac
Ctrl
MC2884 24h
EZH2
D
Genes with reduced H3K27ac
Genes with reduced H3K27me3
expression RPKM
Ctrl
MC2884 4h
MC2884 24h
E
BCL2 fold expression
pt#8 (APL-R)
pt#9 (APL)
C
MC2884
ATRA
HDACi
F
BCL2
H3K27ac Fold enrichment
H3K27me3 Fold enrichment
CBS
SLC52A1
C
MC2884
C646 + GSK126
G
NB4
BCL2 fold change
30 h
48 h
BCL2
ERKs
H
NB4
NB4-MR4
% pre-G1 phase
ATRA
MC2884
iBcl2
µM
30 h

Fig. 12

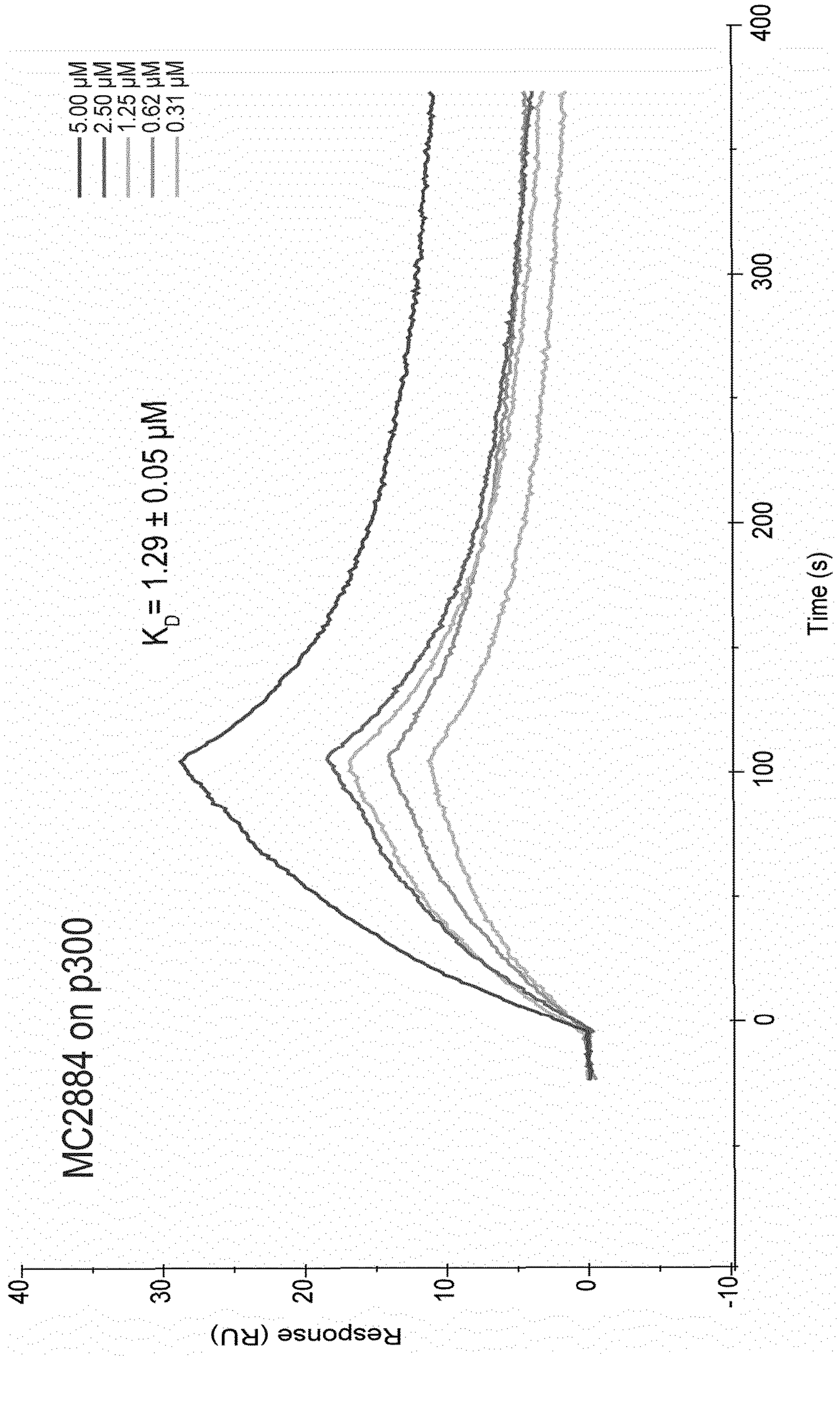
MC2884 on p300
K_D = 1.29 ± 0.05 µM
5.00 µM
2.50 µM
1.25 µM
0.62 µM
0.31 µM
Response (RU)
Time (s)
40
30
20
10
0
-10
0
100
200
300
400

Fig. 13

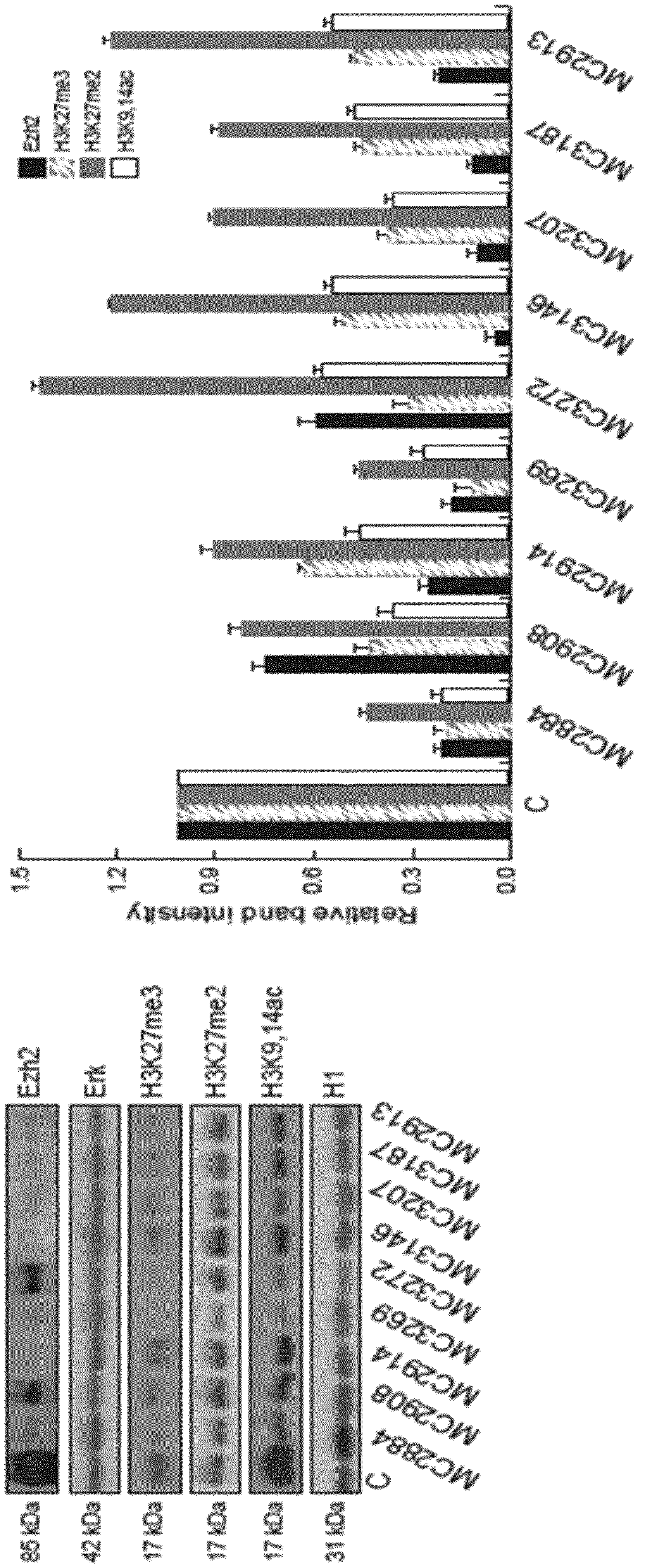
Relative band intensity
1.5
1.2
0.9
0.6
0.3
0.0
Ezh2
H3K27me3
H3K27me2
H3K9,14ac
C
MC2884
MC2908
MC2914
MC3269
MC3272
MC3146
MC3207
MC3187
MC2913
85 kDa
42 kDa
17 kDa
17 kDa
17 kDa
31 kDa
Ezh2
Erk
H3K27me3
H3K27me2
H3K9,14ac
H1

Fig. 14

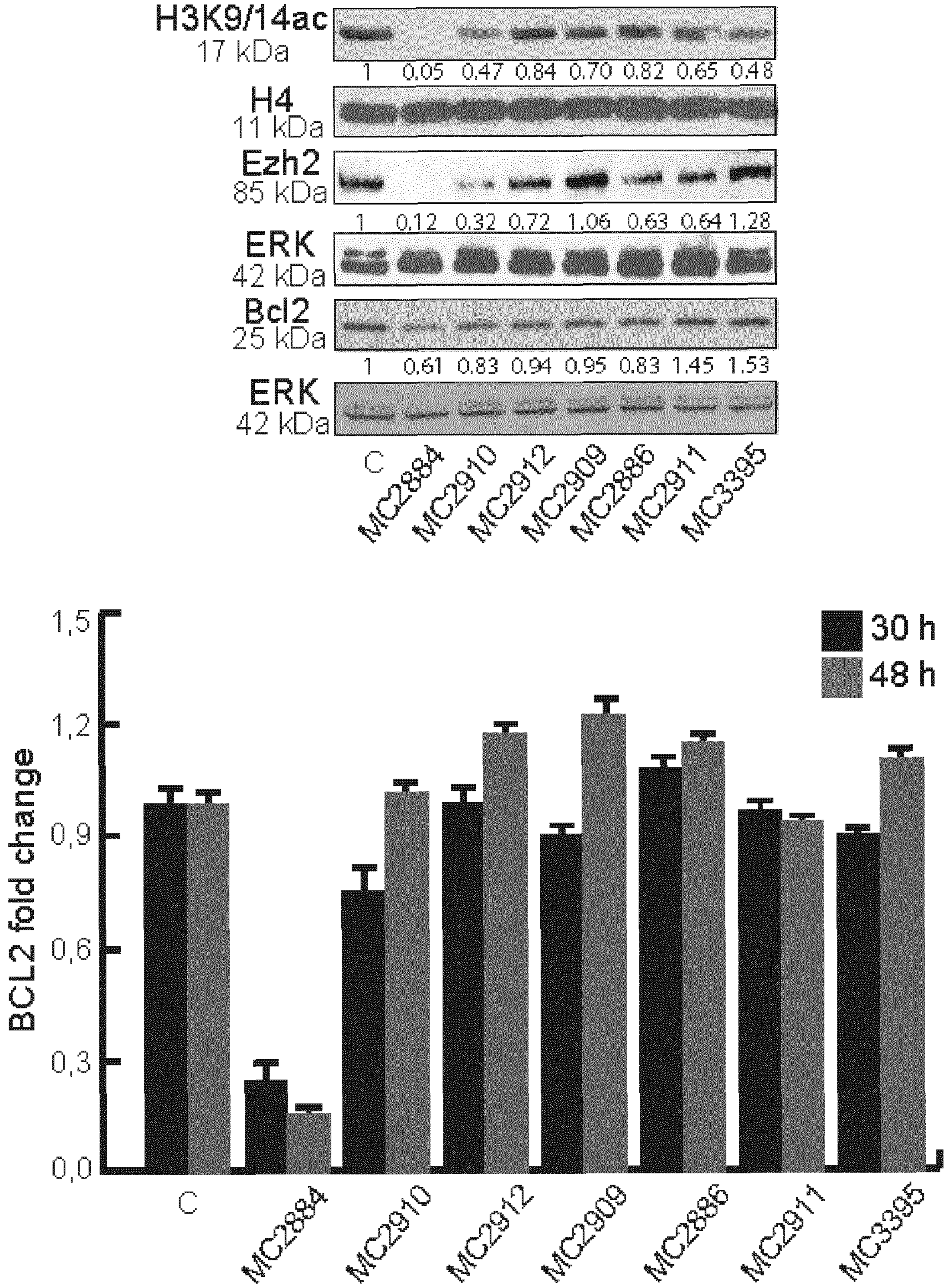
H3K9/14ac
17 kDa
1 0.05 0.47 0.84 0.70 0.82 0.65 0.48
H4
11 kDa
Ezh2
85 kDa
1 0.12 0.32 0.72 1.06 0.63 0.64 1.28
ERK
42 kDa
Bcl2
25 kDa
1 0.61 0.83 0.94 0.95 0.83 1.45 1.53
ERK
42 kDa
C
MC2884
MC2910
MC2912
MC2909
MC2886
MC2911
MC3395
30 h
48 h
BCL2 fold change
1,5
1,2
0,9
0,6
0,3
0,0
C
MC2884
MC2910
MC2912
MC2909
MC2886
MC2911
MC3395

Fig. 15

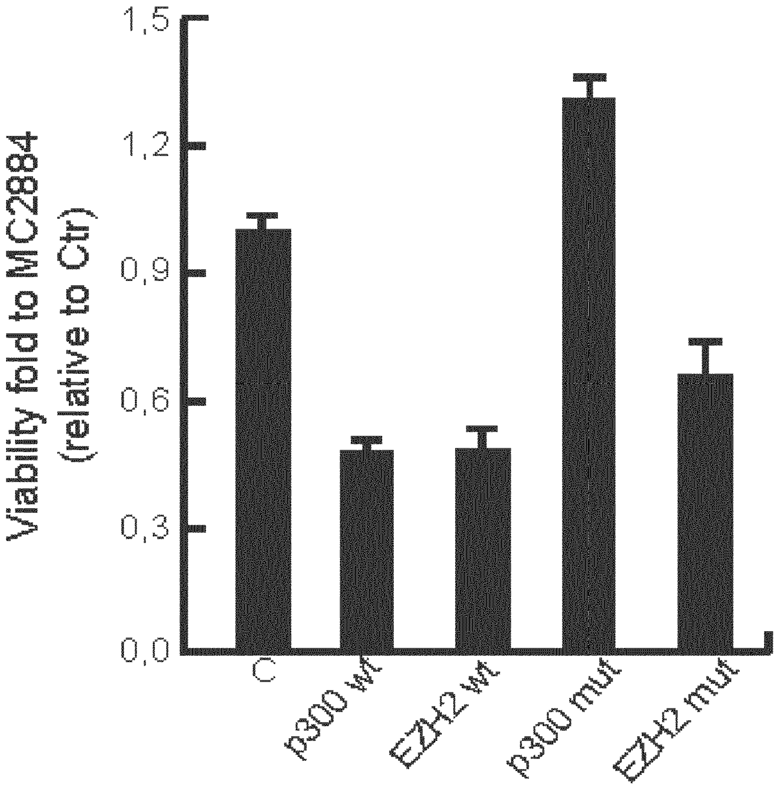
Viability fold to MC2884
(relative to Ctr)
1,5
1,2
0,9
0,6
0,3
0,0
C
p300 wt
EZH2 wt
p300 mut
EZH2 mut

Fig. 16

| Average | animal(s) ,1,2,3 | | | | | | | | |
|---|---|---|---|---|---|---|---|---|---|
| Time (hr) | Pre-Dose | 0,08 | 0,25 | 0,50 | 1,00 | 2,00 | 4,00 | 8,00 | 24,00 |
| Conc (µg/mL) | | 0,401 | 0,639 | 4,589 | 7,796 | 5,146 | 0,453 | 0,109 | 0,030 |
| Std Dev | | 0,0443 | 0,4703 | 4,2551 | 1,4517 | 4,4802 | 0,4222 | 0,1082 | 0,0004 |
| %CV | | 11% | 74% | 93% | 19% | 87% | 93% | 99% | 1% |
| # Excluded Points | | | | | | | | | |
| # Tailpoints | 3 | | | | | | | | |

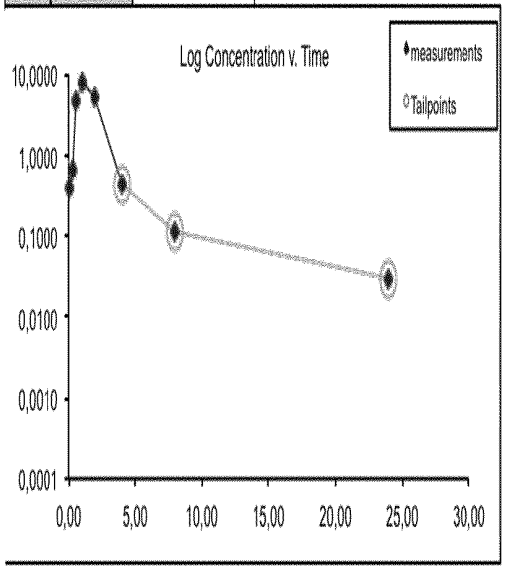


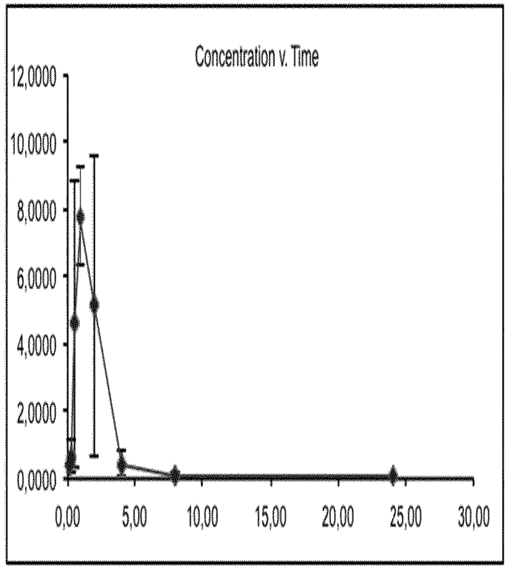


| | |
|---|---|
| Av. Weight (g) | 25 |
| Dose (mg) | 0,05 |
| Cmax (µg/mL) | 7,796 |
| C0 (µg/mL) | |
| Clast (µg/mL) | 0,030 |
| tmax (hr) | 1,00 |
| tlast (hr) | 24,00 |
| AUClast (µg-hr/mL) | 18,162 |
| AUCinf (µg-hr/mL) | 18,413 |
| t1/2 (hr) | NA |
| λz (1/hr) | 0,12 |
| MRTinf (hr) | 2,73 |
| Total CL (mL/hr) | NA |
| Total CL (mL/min/kg) | NA |
| VSS (mL) | NA |
| VZ (mL) | NA |

Fig. 17

| Average | animal(s) ,1,2,3 | | | | | | | | |
|---|---|---|---|---|---|---|---|---|---|
| Time (hr) | Pre-Dose | 0,10 | 0,25 | 0,52 | 1,00 | 2,03 | 4,02 | 8,03 | 24,02 |
| Conc (µg/mL) | | 29,456 | 4,389 | 1,787 | 0,766 | 0,322 | 0,225 | 0,093 | 0,010 |
| Std Dev | | 18,9115 | 0,9747 | 0,7734 | 0,0594 | 0,0172 | 0,0332 | 0,0116 | 0,0041 |
| %CV | | 64% | 22% | 43% | 8% | 5% | 15% | 12% | 42% |
| # Excluded Points | | | | | | | | | |
| # Tailpoints | 3 | | | | | | | | |

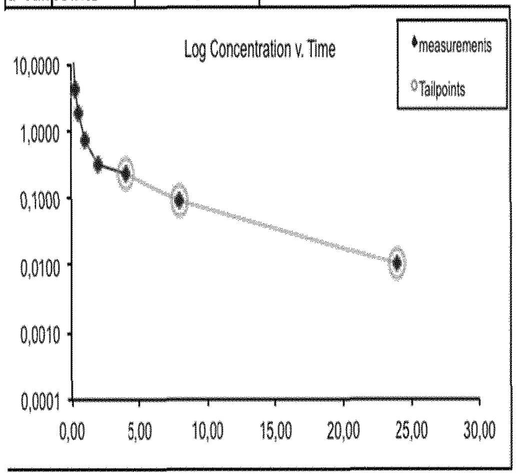


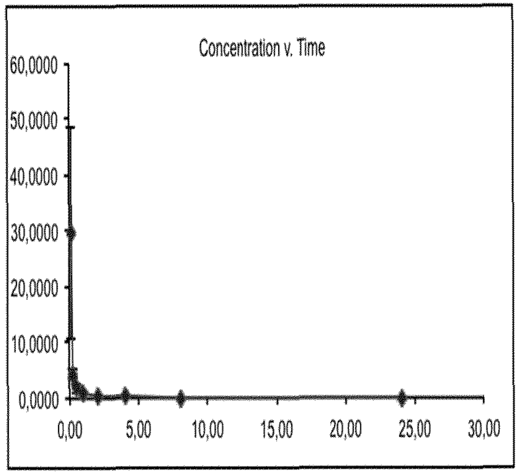


| | |
|---|---|
| Av. Weight (g) | 25 |
| Dose (mg) | 0,05 |
| Cmax (µg/mL) | |
| C0 (µg/mL) | 104,802 |
| Clast (µg/mL) | 0,010 |
| tmax (hr) | NA |
| tlast (hr) | 24,02 |
| AUClast (µg-hr/mL) | 13,256 |
| AUCinf (µg-hr/mL) | 13,322 |
| t1/2 (hr) | 4,58 |
| λz (1/hr) | 0,15 |
| MRTinf (hr) | 1,25 |
| Total CL (mL/hr) | 3,80 |
| Total CL (mL/min/kg) | 2,54 |
| VSS (mL) | 4,74 |
| VZ (mL) | 25,12 |

Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 102016000051855 A

### Non-patent literature cited in the description


- **MARIA E. FIGUEROA et al.** *CANCER CELL,* 01 January 2010, vol. 17 (1), 13-27
- **CHOR S. CHIM et al.** *British Journal of Haematology,* 01 August 2003, vol. 122 (4), 571-578
- **M F ARTEAGA et al.** *British Journal of Cancer,* 03 February 2015, vol. 112 (3), 413-418
- **T. SCHOOFS et al.** *Blood,* 03 January 2013, vol. 121 (1), 178-187
- **NASR R ; GUILLEMIN MC ; FERHI O ; SOILIHI H ; PERES L ; BERTHIER C ; ROUSSELOT P ; ROBLEDO-SARMIENTO M ; LALLEMAND-BREITENBACH V ; GOURMEL B.** *Nat Med.,* December 2008, vol. 14 (12), 1333-42
- Genomic and epigenomic landscapes of adult de novo acute myeloid leukemia. *N Engl J Med,* 2013, vol. 368 (22), 2059-74
- **WEINSTEIN JN et al.** *Nat Genet,* 2013, vol. 45 (10), 1113-20
- **SUN XJ et al.** *Front Oncol,* 2015, vol. 5, 108
- **AVVAKUMOV N ; COTE J.** *Oncogene,* 2007, vol. 26 (37), 5395-407
- **GREER EL ; SHI Y.** *Nat Rev Genet,* 2012, vol. 13 (5), 343-57
- **FALKENBERG KJ ; JOHNSTONE RW.** *Nat Rev Drug Discov,* 2014, vol. 13 (9), 673-91
- **PARRY L ; CLARKE AR.** *Genes Cancer,* 2011, vol. 2 (6), 618-30
- **MULLER S ; FILIPPAKOPOULOS P ; KNAPP S.** *Expert Rev Mol Med,* 2011, vol. 13, e29
- **FILIPPAKOPOULOS P et al.** *Nature,* 2010, vol. 468 (7327), 1067-73
- **KOLLA V et al.** *Cancer Res,* 2014, vol. 74 (3), 652-8
- **JARIWALA N et al.** *Int J Oncol,* 2015, vol. 46 (2), 465-73
- **MUSSELMAN CA et al.** *Mol Interv,* 2009, vol. 9 (6), 314-23
- **CONTE M et al.** *Clin Cancer Res,* 2012, vol. 18 (20), 5526-34
- **YOU JS et al.** *Cancer cell,* 2012, vol. 22 (1), 9-20
- **SHEN H ; LAIRD PW.** *Cell,* 2013, vol. 153 (1), 38-55
- **MANDOLI A et al.** *Cell reports,* 2016, vol. 17 (8), 2087-100
- **PAPAEMMANUIL E et al.** *N Engl J Med,* 2016, vol. 374 (23), 2209-21
- **QUEIROS AC et al.** *Cancer cell,* 2016, vol. 30 (5), 806-21
- **NEBBIOSO A et al.** *Expert Opin Ther Targets,* 2015, vol. 19 (9), 1187-202
- **NEBBIOSO A et al.** *Mol Oncol,* 2012, vol. 6 (6), 657-82
- **NEBBIOSO A et al.** *Clin Cancer Res,* 2016
- **BAYLIN SB et al.** *Nat Rev Cancer,* 2011, vol. 11 (10), 726-34
- **LAUBACH JP et al.** *Clin Cancer Res,* 2015, vol. 21 (21), 4767-73
- **WU H et al.** *PLoS One,* 2013, vol. 8 (12), e83737
- **WAGENER N et al.** *BMC Cancer,* 2010, vol. 10, 524
- **ANTONYSAMY S et al.** *PLoS One,* 2013, vol. 8 (12), e84147
- **DILLON SC et al.** *Genome Biol,* 2005, vol. 6 (8), 227
- **FRANCI G et al.** *Epigenetics,* 2016, vol. 0
- **MICELI M et al.** *Stem Cells Dev,* 2013, vol. 22 (17), 2368-83
- **MARTENS et al.** *FEBS Lett,* 2010, vol. 584 (12), 2662-9
- **CONTE M et al.** *Oncotarget,* 2015, vol. 6 (2), 886-901
- **SAEED S et al.** *Blood,* 2012, vol. 120 (15), 3058-68
- **DE BELLIS F et al.** *Cancer Res,* 2014, vol. 74 (8), 2328-39
- **NEBBIOSO A et al.** *Nat Med,* 2005, vol. 11 (1), 77-84
- **VALENTE S et al.** *Biochimie,* 2012, vol. 94 (11), 2308-13
- **NEBBIOSO A et al.** *EMBO Rep,* 2009, vol. 10 (7), 776-82
- **VALENTE S et al.** *J Med Chem,* 2016
- **ROTILI D et al.** *J Med Chem,* 2014, vol. 57 (1), 42-55

- **ADAMS D et al.** *Nat Biotechnol,* 2012, vol. 30 (3), 224-6
- **VILLA R et al.** *Cancer cell,* 2007, vol. 11 (6), 513-25
- **BOCCELLINO M et al.** *J Cell Biochem,* 2012, vol. 113 (4), 1292-301
- **MICELI M, A et al.** *Proteomics,* 2015
- **MAURANO MT et al.** *Cell reports,* 2015, vol. 12 (7), 1184-95
- **DE JONGE R et al.** *Blood,* 2009, vol. 113 (10), 2284-9
- **THALER R et al.** *J Biol Chem,* 2011, vol. 286 (7), 5578-88
- **ROSENAUER A et al.** *Blood,* 1996, vol. 88 (7), 2671-82
- **BENEDETTI R et al.** *Antioxid Redox Signal,* 2015, vol. 23 (1), 99-126
- **DELL'AVERSANA C et al.** *Leukemia,* 2017
- **DELHOMMEAU F et al.** *N Engl J Med,* 2009, vol. 360 (22), 2289-301
- **QUIVORON C et al.** *Cancer cell,* 2011, vol. 20 (1), 25-38
- **MALINGE S et al.** *Blood,* 2008, vol. 112 (10), 4220-6
- **ROOSWINKEL RW et al.** *Cell Death Dis,* 2012, vol. 3, e366
- **RAJESH SM ; BALA BD.** *Tetrahedron Letters,* 2012, vol. 53 (40), 5367-71
- **HAN Z. TS et al.** *Synthesis,* 2009, vol. 8
- **WEI X ; DU ZY et al.** *Eur J Med Chem,* 2012, vol. 53, 235-45
- **WAN YW et al.** *Synthetic Communications,* 2010, vol. 40 (15), 9
- **CHAIB H et al.** *Leukemia,* 2012, vol. 26 (4), 662-74
- **WEISBERG E et al.** *Mol Cancer Ther,* 2015, vol. 14 (10), 2249-59
- **RHEE I et al.** *Nature,* 2002, vol. 416 (6880), 552-6
- **MARTENS JH et al.** *Biochim Biophys Acta,* 2011, vol. 1812 (8), 818-23
- **WU TD et al.** *Bioinformatics,* 2010, vol. 26 (7), 873-81
- **KANITZ A et al.** *Genome Biol,* 2015, vol. 16, 150
- **VOGELSTEIN B et al.** *Science,* 2013, vol. 339 (6127), 1546-58
- **LEY TJ et al.** *N Engl J Med.,* 30 May 2013, vol. 368 (22), 2059-74
- **DI CERBO V et al.** *Brief Funct Genomics,* May 2013, vol. 12 (3), 231-43
- **MANZO F et al.** *Expert Opin Ther Pat.,* June 2009, vol. 19 (6), 761-74
- **KIM KH et al.** *Nat Med.,* 04 February 2016, vol. 22 (2), 128-34
- **TANAKA M et al.** *Pharm Pat Anal.,* 2015, vol. 4 (4), 261-84